(19) Europäisches Patentamt
European Patent Office
Office européen des brevets

(11) EP 3 490 998 B1

(12) # EUROPEAN PATENT SPECIFICATION

(45) Date of publication and mention of the grant of the patent:
**26.05.2021 Bulletin 2021/21**

(21) Application number: **17834941.1**

(22) Date of filing: **07.07.2017**

(51) Int Cl.:
***C07F 7/00*** *(2006.01)* ***B01J 31/22*** *(2006.01)*
***C07D 209/82*** *(2006.01)* ***C08F 4/76*** *(2006.01)*
***C08F 10/14*** *(2006.01)*

(86) International application number:
**PCT/US2017/041201**

(87) International publication number:
**WO 2018/022279 (01.02.2018 Gazette 2018/05)**

(54) **PHENOLATE TRANSITION METAL COMPLEXES, PRODUCTION AND USE THEREOF**

PHENOLATÜBERGANGSMETALLKOMPLEXE, HERSTELLUNG UND VERWENDUNG DAVON

LIGANDS PHÉNOLATE, COMPLEXES DE MÉTAUX DE TRANSITION, LEUR PRODUCTION ET LEUR UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.07.2016 US 201662368247 P**
**13.09.2016 EP 16188648**

(43) Date of publication of application:
**05.06.2019 Bulletin 2019/23**

(73) Proprietor: **ExxonMobil Chemical Patents Inc.**
**Baytown, TX 77520 (US)**

(72) Inventors:
- **ATIENZA, Crisita, Carmen, H.**
  **Houston, TX 77008 (US)**
- **CANO, David, A.**
  **Houston, TX 77066 (US)**
- **FALER, Catherine**
  **Houston, TX 77007 (US)**
- **RAMIREZ, Kevin**
  **IN 47906 (US)**

(74) Representative: **ExxonMobil Chemical Europe Inc.**
**IP Law Europe**
**Hermeslaan 2**
**1831 Machelen (BE)**

(56) References cited:
**WO-A1-00/39064** **WO-A1-01/21684**
**WO-A1-2012/111779** **WO-A1-2014/143202**
**WO-A1-2015/088819** **WO-A1-2016/089935**
**WO-A1-2016/094866** **WO-A2-00/16763**
**US-A1- 2004 132 789** **US-A1- 2012 029 159**

- **JAIN, MANISH ET AL.: "Antithrombotic activity of a newly synthesized coumarin derivative 3-(5-hydroxy-2,2-dimethylchroman-6-yl)-N-{ 2-[3 -(5-hydroxy-2,2-dimethylchroman-6-yl)p ropionylamino]ethyl}propionamide", CHEMICAL BIOLOGY & DRUG DESIGN, vol. 81, no. 4, 28 March 2013 (2013-03-28) , pages 499-508, XP002767260, ISSN: 1747-0277**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 490 998 B1

## Description

### FIELD OF THE INVENTION

The invention relates to phenolate ligands, phenolate transition metal complexes and processes for use of such complexes as catalysts for alkene polymerization processes, with or without chain transfer agents.

### BACKGROUND OF THE INVENTION

Olefin polymerization catalysts are of great use in industry. Hence there is interest in finding new catalyst systems that increase the commercial usefulness of the catalyst and allow the production of polymers having improved properties.

Catalysts for olefin polymerization have been based on bisphenolate complexes as catalyst precursors, which are typically activated with an alumoxane or with an activator containing a non-coordinating anion.

Diamine bis(phenolate) Group IV complexes have been used as transition metal components in the copolymerization of ethylene and hexene, see for example, Macromolecules 2005, 38, 2552-2558, and in the homopolymerization of 1-hexene, see for example J. Am. Chem. Soc. 2000, 122, 10706, and propylene, see for example, Macromolecules 2010, 43, 1689.

WO 2002/036638 and WO 2012/098521 disclose diamine bis(phenolate) compounds for use as alpha olefin polymerization catalysts.

WO 2012/027448 and WO 2003/091262 disclose bridged bis(phenyl phenol) compounds for olefin polymerization catalysts.

US 8,071,701 discloses bridged polydentate catalyst complexes that produce low molecular weight (approx. 21,000 Mw or less) homopolypropylene where the examples have one carbon in the bridge.

WO 2016/089935 A1 discloses molecular transition metal complex containing a penta-coordinate metal with a single atom bridge structure which complexes can be used to form low molecular weight ethylene-based polymers.

Other references of interest include: WO 2016/003878; WO 2016/003879; US 6,841,502; US 6,596,827; WO 2014/143202; WO 2014/022746; WO 2014/022010; WO 2014/022012; WO 2014/22008; WO 2014/022011; WO 2015/088819; US 8,791,217; US 7,812,104; US 6,232,421; US 6,333,389; US 6,333,423; US 8,907,032; WO 2007/130306; Israel Journal of Chemistry, Volume 42, 2002 pg. 373-381; Macromolecules, 2007, 40, 7061-7064; Chem. Comm. 2000, 379-380; Organometallics, 2001, 20, 3017-3028; Organometallics, 2005, 24, 200-202; Organometallics, 2009, 28, 1391-1405; Journal of Polymer Science, Part A: Polymer Chemistry, 2013, 51, 593-600; WO 2014/070502; US 2016/0280722; US 2017/0096509; Organometallics, 2002, 662-670; WO 2013/096573; Macromolecules 2013, 46, 2569-2575; J. Am. Chem. Soc., 2010, 132, 5566-5567; Macromolecules 2013, 46, 6758-6764; WO 2016/094861; WO 2016/089935; WO 2006/020624; US 2006/0025548; US 2006/0052554; WO 2005/108406; US 2006/0024517; US 8,957,171; US 8,957,172; US 8,952,114; US 9,045,568; US 9,365,661; US 9,382,349; US 8,937,137; US 9,150,676; US 9,200,100; US 9,200,099; US 9,290,589; US 9,534,070; US 2015/0291713; US 2015/0337062; US 2015/0344601; US 9,000,108; US 9,422,383; US 8,609,794 and US 9,193,813.

There still is need for adding to the range of catalysts complexes that may be prepared and broaden their performance in alkene polymerization. Further, there is a need in the art for new catalysts with high activity that can produce crystalline polymers with good molecular weights.

### SUMMARY OF INVENTION

The present invention is concerned with a transition metal complex according to claim 1, a catalyst system according to claim 7, and a polymerization process according to claim 10 This disclosure relates to ligands represented by the formula (A):

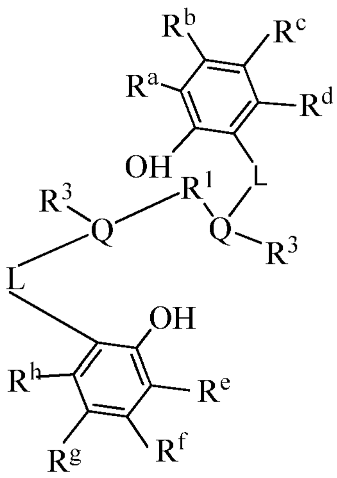

(A)

where each Q is a neutral group comprising at least one atom from Group 15 or Group 16 (such as O, N, S, or P), and $R^3$ is not present when Q is a Group 16 atom;
each L is independently

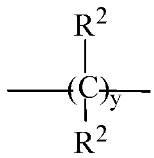

and is not part of an aromatic ring;
y is greater than or equal to 2;
$R^1$ is a divalent $C_1$-$C_{40}$ hydrocarbyl radical or divalent substituted hydrocarbyl radical comprising a portion that comprises a linker backbone comprising from 1 to 18 carbon atoms linking or bridging between the two Q groups;
each $R^a$, $R^b$, $R^c$, $R^d$, $R^e$, $R^f$, $R^g$, and $R^h$ is, independently, a hydrogen, a $C_1$-$C_{60}$ hydrocarbyl radical, a $C_1$-$C_{60}$ substituted hydrocarbyl radical, a heteroatom or a heteroatom-containing group, or two or more of $R^a$ to $R^h$ may independently join together to form a $C_4$ to $C_{62}$ cyclic, polycyclic or heterocyclic structure, or a combination thereof;
each $R^2$ is independently a hydrogen, a $C_1$-$C_{40}$ hydrocarbyl radical, a $C_1$-$C_{40}$ substituted hydrocarbyl radical, a heteroatom or a heteroatom-containing group, or two or more adjacent $R^2$ groups may independently join together to form a $C_4$ to $C_{62}$ cyclic or polycyclic ring structure, or a combination thereof, provided that such cyclic or polycyclic ring structure is not aromatic; and
each $R^3$ is independently a hydrogen, a $C_1$-$C_{40}$ hydrocarbyl radical, a $C_1$-$C_{40}$ substituted hydrocarbyl radical, a heteroatom or a heteroatom-containing group.

This invention relates to transition metal complexes represented by the formula (I):

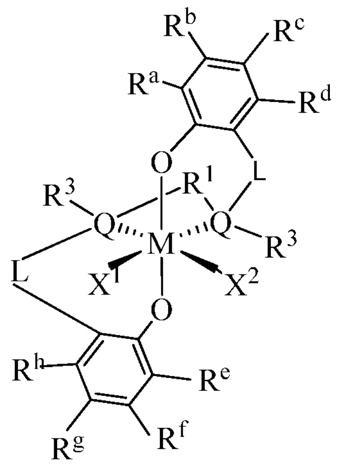

(I)

wherein M is Hf or Zr;
each Q is neutral donor group comprising at least one atom from O, N, S, or P, and $R^3$ is not present when Q is O or S;
each L is independently

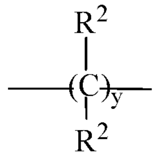

and is not part of an aromatic ring;
y is 2, 3, 4, 5, or 6;
$X^1$ and $X^2$ are, independently, a univalent $C_1$ to $C_{20}$ hydrocarbyl radical, a $C_1$ to $C_{20}$ substituted hydrocarbyl radical, a heteroatom or a heteroatom-containing group, or $X^1$ and $X^2$ join together to form a $C_4$ to $C_{62}$ cyclic, polycyclic or heterocyclic structure;
$R^1$ is a divalent $C_1$-$C_{40}$ hydrocarbyl radical or divalent substituted hydrocarbyl radical comprising a portion that comprises a linker backbone comprising from 1 to 18 carbon atoms linking between the two Q groups;
each $R^a$, $R^b$, $R^c$, $R^d$, $R^e$, $R^f$, $R^g$, and $R^h$ is, independently, a hydrogen, a $C_1$-$C_{60}$ hydrocarbyl radical, a $C_1$-$C_{60}$ substituted hydrocarbyl radical, a heteroatom or a heteroatom-containing group, or two or more of $R^a$ to $R^h$ may independently join together to form a $C_4$ to $C_{62}$ cyclic, polycyclic or heterocyclic structure, or a combination thereof;
each $R^2$ is, independently, a hydrogen, a $C_1$-$C_{40}$ hydrocarbyl radical, a $C_1$-$C_{40}$ substituted hydrocarbyl radical, a heteroatom or a heteroatom-containing group, or two or more adjacent $R^2$ groups may independently join together to form a $C_4$ to $C_{62}$ cyclic or polycyclic ring structure, or a combination thereof, provided that such cyclic or polycyclic ring structure is not aromatic; and
each $R^3$ is, independently, a hydrogen, a $C_1$-$C_{40}$ hydrocarbyl radical, a $C_1$-$C_{40}$ substituted hydrocarbyl radical, a heteroatom or a heteroatom-containing group.

This invention relates to transition metal complexes represented by the formula (II)

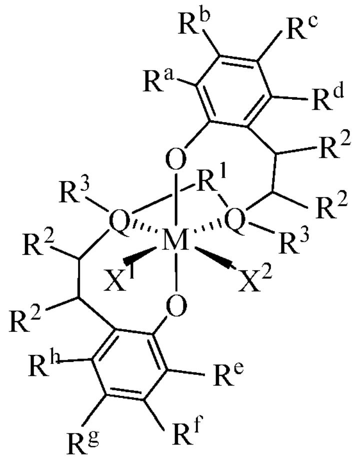

(II)

wherein M is Hf or Zr;

each Q is neutral donor group comprising at least one atom from O, N, S, or P, and $R^3$ is not present when Q is O or S;

$X^1$ and $X^2$ are, independently, a univalent $C_1$ to $C_{20}$ hydrocarbyl radical, a $C_1$ to $C_{20}$ substituted hydrocarbyl radical, a heteroatom or a heteroatom-containing group, or $X^1$ and $X^2$ join together to form a $C_4$ to $C_{62}$ cyclic, polycyclic, or heterocyclic structure;

$R^1$ is a divalent $C_1$-$C_{40}$ hydrocarbyl radical or divalent substituted hydrocarbyl radical comprising a portion that comprises a linker backbone comprising from 1 to 18 carbon atoms linking or bridging between the two Q groups;

each $R^a$, $R^b$, $R^c$, $R^d$, $R^e$, $R^f$, $R^g$, and $R^h$ is, independently, a hydrogen, a $C_1$-$C_{60}$ hydrocarbyl radical, a $C_1$-$C_{60}$ substituted hydrocarbyl radical, a heteroatom or a heteroatom-containing group, or two or more of $R^a$ to $R^h$ may independently join together to form a $C_4$ to $C_{62}$ cyclic, polycyclic or heterocyclic structure, or a combination thereof;

each $R^2$ is, independently, a hydrogen, a $C_1$-$C_{40}$ hydrocarbyl radical, a $C_1$-$C_{40}$ substituted hydrocarbyl radical, a heteroatom or a heteroatom-containing group, or two or more adjacent $R^2$ groups may independently join together to form a $C_4$ to $C_{62}$ cyclic or polycyclic ring structure, or a combination thereof, provided that such cyclic, polycyclic or heterocyclic structure is not aromatic; and

each $R^3$ is, independently, a hydrogen, a $C_1$-$C_{40}$ hydrocarbyl radical, a $C_1$-$C_{40}$ substituted hydrocarbyl radical, a heteroatom or a heteroatom-containing group.

This invention also relates to a catalyst system comprising an activator and one or more catalysts compounds described herein.

This invention also relates to a process to make polyolefin using the catalyst systems described herein.

This invention further relates to methods to polymerize olefins using the above complex in the presence of a chain transfer agent.

## BRIEF DESCRIPTION OF THE DRAWINGS

Figure 1 is a representation of 1-Zr.

Figure 2 is a plot of the chain transfer efficiency of catalyst 1-Zr from Table 14, which is based on the number of polymer chains transferred to each mole of metal of the chain transfer agent.

Figure 3 is a plot of the chain transfer efficiency of catalyst 1-Hf from Table 14 which is based on the number of polymer chains transferred to each mole of metal of the chain transfer agent.

Figure 4 is the $^1$HNMR spectrum for compound A-6 in $CD_2Cl_2$.

Figure 5 is the $^1$HNMR spectrum for compound B-4 in $CD_2Cl_2$.

Figure 6 is the $^1$HNMR spectrum for compound 1-Zr in $CD_2Cl_2$.

Figure 7 is the $^1$HNMR spectrum for compound 1-Hf in $CD_2Cl_2$.

Figure 8 is the $^1$HNMR spectrum for compound 2-Zr in $CD_2Cl_2$.

Figure 9 is the $^1$HNMR spectrum for compound 2-Hf in $CD_2Cl_2$.

Figure 10 is the GPC data for Table 1, Entry 25 (catalyst = 1-Zr; polymer = HDPE).

Figure 11 is the GPC data for Table 1, Entry 27 (catalyst = 1-Hf; polymer = HDPE).

Figure 12 is the GPC data for Table 6, Entry 6 (catalyst = 1-Zr; polymer = EP).

Figure 13 is the GPC data for Table 6, Entry 12 (catalyst = 1-Hf; polymer = EP).

## Detailed Description of the Invention

The specification describes transition metal complexes. The term complex is used to describe molecules in which an ancillary ligand is coordinated to a central transition metal atom. The ligand is bulky and stably bonded to the transition metal so as to maintain its influence during use of the catalyst, such as polymerization. The ligand may be coordinated to the transition metal by covalent bond and/or electron donation coordination or intermediate bonds. The transition metal complexes are generally subjected to activation to perform their polymerization or oligomerization function using an activator, which is believed to create a cation as a result of the removal of an anionic group, often referred to as a leaving group, from the transition metal.

In the structures depicted throughout this specification and the claims, a solid line indicates a bond, an arrow indicates that the bond may be active, and each dashed line represents a bond having varying degrees of covalency and a varying degree of coordination.

As used herein, the numbering scheme for the Periodic Table groups is the new notation as set out in Chemical and Engineering News, 63(5), 27 (1985).

As used herein, Me is methyl, Et is ethyl, Bu is butyl, t-Bu and $^t$Bu are tertiary butyl, Pr is propyl, iPr and $^i$Pr are isopropyl, Cy is cyclohexyl, THF (also referred to as thf) is tetrahydrofuran, Bn is benzyl, $[H_2CO]_x$ is paraformaldehyde, and Ph is phenyl.

The terms "hydrocarbyl radical," "hydrocarbyl," and "hydrocarbyl group" are used interchangeably throughout this document unless otherwise specified. For purposes of this disclosure, a hydrocarbyl radical is defined to be $C_1$ to $C_{70}$ radicals, or $C_1$ to $C_{20}$ radicals, or $C_1$ to $C_{10}$ radicals, or $C_6$ to $C_{70}$ radicals, or $C_6$ to $C_{20}$ radicals, or $C_7$ to $C_{20}$ radicals that may be linear, branched, or cyclic (including polycyclic) and aromatic or non-aromatic.

For purposes herein, a carbazole radical or substituted carbazole radical is represented by the formula:

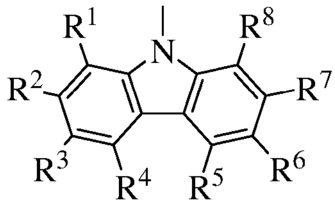

wherein each $R^1$ through $R^8$ is, independently, a hydrogen, a $C_1$-$C_{40}$ hydrocarbyl radical, a functional group comprising elements from Group 13 to 17 of the Periodic Table of the Elements, or two or more of $R^1$ to $R^8$ may independently join together to form a $C_4$ to $C_{62}$ cyclic or polycyclic ring structure, or a combination thereof.

A substituted or unsubstituted fluorenyl radical is represented by the formula:

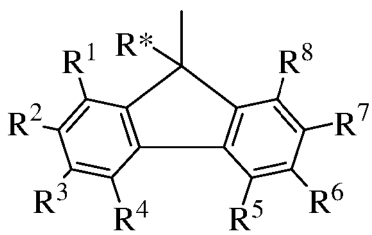

wherein each $R^1$ through $R^8$ is, independently, a hydrogen, a $C_1$-$C_{40}$ hydrocarbyl radical, a functional group comprising elements from Group 13 to 17 of the periodic table of the elements, or two or more of $R^1$ to $R^8$ may independently join together to form a $C_4$ to $C_{62}$ cyclic or polycyclic ring structure, or a combination thereof; R* is a hydrogen, a $C_1$-$C_{40}$ hydrocarbyl radical, a substituted $C_1$-$C_{40}$ hydrocarbyl radical (preferably R* is methyl, phenyl, or substituted phenyl).

The term "catalyst system" is defined to mean a complex/activator pair. When "catalyst system" is used to describe such a pair before activation, it means the unactivated catalyst complex (precatalyst) together with an activator, optionally, a chain transfer agent, and, optionally, a co-activator. When it is used to describe such a pair after activation, it means the activated complex and the activator or other charge-balancing moiety. The transition metal compound may be neutral as in a precatalyst, or a charged species with a counter ion as in an activated catalyst system.

Complex, as used herein, is also often referred to as catalyst precursor, precatalyst, catalyst, catalyst compound, transition metal compound, or transition metal complex. These words are used interchangeably.

A "neutral donor group" is a neutrally charged group which donates one or more pairs of electrons to a metal.

An "olefin," alternatively referred to as "alkene," is a linear, branched, or cyclic compound of carbon and hydrogen having at least one double bond. For purposes of this specification and the claims appended thereto, when a polymer or copolymer is referred to as comprising an olefin, the olefin present in such polymer or copolymer is the polymerized form of the olefin. For example, when a copolymer is said to have an "ethylene" content of 35 wt% to 55 wt%, it is understood that the mer unit in the copolymer is derived from ethylene in the polymerization reaction and said derived units are present at 35 wt% to 55 wt%, based upon the weight of the copolymer. A "polymer" has two or more of the same or different mer units. A "homopolymer" is a polymer having mer units that are the same. A "copolymer" is a polymer having two or more mer units that are different from each other. A "terpolymer" is a polymer having three mer units that are different from each other. "Different" as used to refer to mer units indicates that the mer units differ from each other by at least one atom or are different isomerically. Accordingly, the definition of copolymer, as used herein, includes terpolymers and the like. An "ethylene polymer" or "ethylene copolymer" is a polymer or copolymer comprising at least 50 mole% ethylene derived units, a "propylene polymer" or "propylene copolymer" is a polymer or copolymer comprising at least 50 mole% propylene derived units, and so on.

For the purposes of this invention, ethylene shall be considered an $\alpha$-olefin.

For purposes of this invention and claims thereto, the term "substituted" means that a hydrogen group has been replaced with a heteroatom, or a heteroatom-containing group. For example, a "substituted hydrocarbyl" is a radical made of carbon and hydrogen where at least one hydrogen is replaced by a heteroatom or heteroatom-containing group. However, for purposes of this invention and claims thereto in relation to the catalyst compounds described herein, the term "substituted" means that a hydrogen group has been replaced with a hydrocarbyl group, a heteroatom, or a heteroatom-containing group. For example, methyl cyclopentadiene (Cp) is a Cp group substituted with a methyl group.

Unless otherwise noted, all molecular weights units (e.g., Mw, Mn, Mz) are g/mol.

Unless otherwise noted all melting points ($T_m$) are DSC second melt.

The term "aryl", "aryl radical", and/or "aryl group" refers to aromatic cyclic structures, which may be substituted with hydrocarbyl radicals and/or functional groups as defined herein.

As used herein the term "aromatic" also refers to pseudoaromatic heterocycles which are heterocyclic substituents that have similar properties and structures (nearly planar) to aromatic heterocyclic ligands, but are not by definition aromatic; likewise, the term aromatic also refers to substituted aromatics.

The term "continuous" means a system that operates without interruption or cessation. For example, a continuous process to produce a polymer would be one where the reactants are continually introduced into one or more reactors and polymer product is continually withdrawn.

A solution polymerization means a polymerization process in which the polymer is dissolved in a liquid polymerization medium, such as an inert solvent or monomer(s) or their blends. A solution polymerization is typically homogeneous. A homogeneous polymerization is one where the polymer product is dissolved in the polymerization medium. Such systems are preferably not turbid as described in J. Vladimir Oliveira, C. Dariva and J. C. Pinto, Ind. Eng, Chem. Res. 29, 2000, 4627.

A bulk polymerization means a polymerization process in which the monomers and/or comonomers being polymerized are used as a solvent or diluent using little or no inert solvent as a solvent or diluent. A small portion of inert solvent might be used as a carrier for catalyst and scavenger. A bulk polymerization system contains less than 25 wt% of inert solvent or diluent, preferably less than 10 wt%, preferably less than 1 wt%, preferably 0 wt%.

For purposes herein, RT is room temperature, which is defined as 25°C unless otherwise specified. All percentages are weight percent (wt%) unless otherwise specified.

Catalyst Ligands and Compounds

In a first aspect, this disclosure relates to ligands represented by the formula (A):

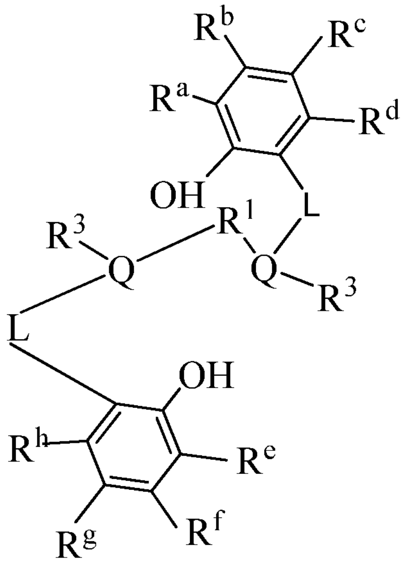

where each Q is neutral donor group comprising at least one atom from Group 15 or Group 16 (such as O, N, S, or P), and $R^3$ is not present when Q is a Group 16 atom;
each L is independently

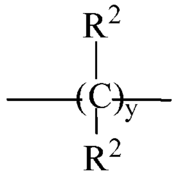

and is not part of an aromatic ring;
y is greater than or equal to 2, preferably 2, 3, 4, 5, or 6;
$R^1$ is a divalent $C_1$-$C_{40}$ (alternately $C_1$ to $C_{20}$) hydrocarbyl radical or divalent substituted hydrocarbyl radical comprising a portion that comprises a linker backbone comprising from 1 to 18 carbon atoms linking or bridging between the two Q groups, preferably as described below for formulas I and II;
each $R^a$, $R^b$, $R^c$, $R^d$, $R^e$, $R^f$, $R^g$, and $R^h$ is, independently, a hydrogen, a $C_1$-$C_{60}$ (alternately $C_1$ to $C_{40}$) hydrocarbyl radical, a $C_1$-$C_{60}$ (alternately $C_1$ to $C_{40}$) substituted hydrocarbyl radical, a heteroatom or a heteroatom-containing group, or two or more of $R^a$ to $R^h$ may independently join together to form a $C_4$ to $C_{62}$ cyclic, polycyclic or heterocyclic structure, or a combination thereof, preferably as described below for formulas I and II;
each $R^2$ is, independently, a hydrogen, a $C_1$-$C_{40}$ hydrocarbyl radical, a $C_1$-$C_{40}$ substituted hydrocarbyl radical, a heteroatom or a heteroatom-containing group, or two or more adjacent $R^2$ groups may independently join together to form a $C_4$ to $C_{62}$ cyclic or polycyclic ring structure, or a combination thereof, provided that such cyclic or polycyclic ring structure is not aromatic, preferably as described below for formulas I and II; and
each $R^3$ is, independently, a hydrogen, a $C_1$-$C_{40}$ hydrocarbyl radical, a $C_1$-$C_{40}$ substituted hydrocarbyl radical, a heteroatom or a heteroatom-containing group, preferably as described below for formulas I and II.

In an aspect of the invention there is provided a transition metal complex (optionally for use in alkene polymerization) represented by the formula (I) or (II):

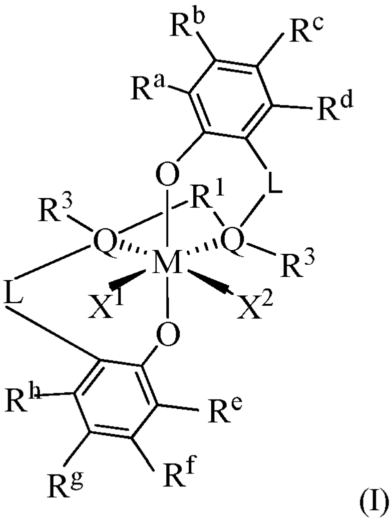

(I) or

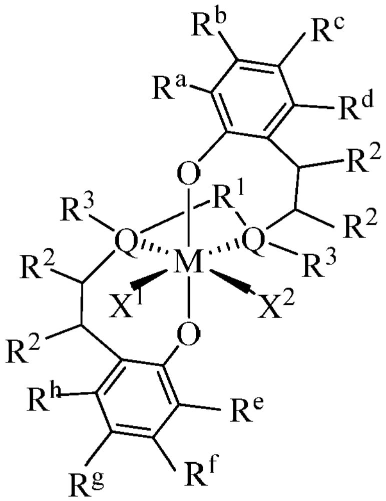

(II)

wherein M is Hf or Zr;

each L is independently

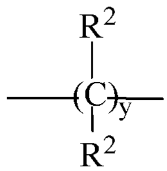

and is not part of an aromatic ring;

y is 2, 3, 4, 5, or 6;

$X^1$ and $X^2$ are, independently, a univalent $C_1$ to $C_{20}$ hydrocarbyl radical, a $C_1$ to $C_{20}$ substituted hydrocarbyl radical, a heteroatom or a heteroatom-containing group, or $X^1$ and $X^2$ join together to form a $C_4$ to $C_{62}$ cyclic, polycyclic or heterocyclic ring structure (preferably benzyl, methyl, ethyl, chloro, bromo, and the like);

each Q is independently a neutral donor group comprising at least one atom from O, N, S, or P (preferably O or N), and $R^3$ is not present when Q is O or S;

$R^1$ is a divalent $C_1$-$C_{40}$ (alternately $C_1$ to $C_{20}$) hydrocarbyl radical or divalent substituted hydrocarbyl radical comprising a portion that comprises a linker backbone comprising from 1 to 18 carbon atoms linking or bridging between the two Q groups, preferably $R^1$ is a $-(CR^5{}_2)_n$-group, where n is 2, 3, 4, 5, or 6, (preferably 2 or 3) each $R^5$ is H or a $C_1$ to $C_{40}$ hydrocarbyl radical, a $C_1$ to $C_{40}$ substituted hydrocarbyl radical, a heteroatom or a heteroatom-containing group, or multiple $R^5$ groups may join together to form a $C_4$ to $C_{62}$ cyclic or polycyclic ring structure (preferably a benzene ring, substituted benzene ring, cyclohexyl, substituted cyclohexyl, cyclooctyl, or substituted cyclooctyl), preferably each $R^5$ is, independently, a $C_1$-$C_{20}$ hydrocarbyl radical, preferably a $C_1$-$C_{20}$ alkyl radical, preferably each $R^5$ is, independently, selected from the group consisting of hydrogen, methyl, ethyl, ethenyl and isomers of propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, and eicosyl;

each $R^a$, $R^b$, $R^c$, $R^d$, $R^e$, $R^f$, $R^g$, and $R^h$ is, independently, a hydrogen, a $C_1$-$C_{60}$ (alternately $C_1$ to $C_{40}$) hydrocarbyl radical, a $C_1$-$C_{60}$ substituted hydrocarbyl radical, a heteroatom or a heteroatom-containing group, or two or more adjacent $R^a$ to $R^h$ groups may independently join together to form a $C_4$ to $C_{62}$ cyclic or polycyclic ring structure, or a combination thereof, preferably each $R^a$, $R^b$, $R^c$, $R^d$, $R^e$, $R^f$, $R^g$, and $R^h$ is, independently, a $C_1$-$C_{20}$ hydrocarbyl radical, preferably a $C_1$-$C_{20}$ alkyl or aromatic radical, preferably each $R^a$, $R^b$, $R^c$, $R^d$, $R^e$, $R^f$, $R^g$, and $R^h$ is, independently, selected from the group consisting of hydrogen, methyl, ethyl, ethenyl and isomers of propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, adamantyl, substituted adamantyl, cyclohexyl, substituted cyclohexyl phenyl, substituted phenyl, fluorenyl, substituted fluorenyl, carbazolyl, substituted carbazolyl, naphthyl, substituted naphthyl, phenanthryl, substituted phenanthryl, anthracenyl, substituted anthracenyl, indanyl, substituted indanyl, indenyl, substituted indenyl;

each $R^2$ is, independently, a hydrogen, a $C_1$-$C_{40}$ hydrocarbyl radical, a $C_1$-$C_{40}$ substituted hydrocarbyl radical, a heteroatom or a heteroatom-containing group, or two or more adjacent $R^2$ groups may independently join together to form a $C_4$ to $C_{62}$ cyclic or polycyclic ring structure, or a combination thereof, provided that such cyclic or polycyclic

ring structure is not aromatic, preferably each $R^2$ is, independently, a $C_1$-$C_{20}$ hydrocarbyl radical, preferably a $C_1$-$C_{20}$ alkyl radical, preferably each $R^2$ is, independently, selected from the group consisting of hydrogen, methyl, ethyl, ethenyl and isomers of propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, and eicosyl; and
each $R^3$ is, independently, a hydrogen, a $C_1$-$C_{40}$ hydrocarbyl radical, a $C_1$-$C_{40}$ substituted hydrocarbyl radical, a heteroatom or a heteroatom-containing group, preferably a $C_1$-$C_{20}$ hydrocarbyl radical, preferably a $C_1$-$C_{20}$ alkyl radical, preferably each $R^3$ is, independently, selected from the group consisting of hydrogen, methyl, ethyl, ethenyl and isomers of propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, and eicosyl, phenyl, and substituted phenyl.

In another aspect, this invention relates to catalyst compounds represented by the formula (I) or (II) where $R^a$ and/or $R^e$ (preferably $R^a$ and $R^e$) are independently a carbazole radical or substituted carbazole radical is represented by the formula:

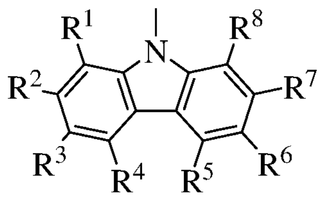

wherein each $R^1$, $R^2$, $R^3$, $R^4$, $R^5$, $R^6$, $R^7$, and $R^8$ is, independently, a hydrogen, a $C_1$-$C_{40}$ hydrocarbyl radical, a functional group comprising elements from Group 13 to 17 of the periodic table of the elements, or two or more of $R^1$ to $R^8$ may independently join together to form a $C_4$ to $C_{62}$ cyclic or polycyclic ring structure, or a combination thereof, preferably each $R^1$, $R^2$, $R^3$, $R^4$, $R^5$, $R^6$, $R^7$, and $R^8$ is hydrogen.

For purposes herein, any hydrocarbyl radical (and any alkyl radical) may be independently selected from methyl, ethyl, ethenyl and isomers of propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, heneicosyl, docosyl, tricosyl, tetracosyl, pentacosyl, hexacosyl, heptacosyl, octacosyl, nonacosyl, triacontyl, propenyl, butenyl, pentenyl, hexenyl, heptenyl, octenyl, nonenyl, decenyl, undecenyl, dodecenyl, tridecenyl, tetradecenyl, pentadecenyl, hexadecenyl, heptadecenyl, octadecenyl, nonadecenyl, eicosenyl, heneicosenyl, docosenyl, tricosenyl, tetracosenyl, pentacosenyl, hexacosenyl, heptacosenyl, octacosenyl, nonacosenyl, triacontenyl, propynyl, butynyl, pentynyl, hexynyl, heptynyl, octynyl, nonynyl, decynyl, undecynyl, dodecynyl, tridecynyl, tetradecynyl, pentadecynyl, hexadecynyl, heptadecynyl, octadecynyl, nonadecynyl, eicosynyl, heneicosynyl, docosynyl, tricosynyl, tetracosynyl, pentacosynyl, hexacosynyl, heptacosynyl, octacosynyl, nonacosynyl, and triacontynyl.

In any embodiment of the transition metal complexes described herein M may be Hf, Ti or Zr.

In any embodiment of the transition metal complexes described herein, each of $X^1$ and $X^2$ is independently selected from the group consisting of hydrocarbyl radicals having from 1 to 20 carbon atoms (such as methyl, ethyl, ethenyl and isomers of propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl), hydrides, amides, alkoxides having from 1 to 20 carbon atoms, sulfides, phosphides, halides, sulfoxides, sulfonates, phosphonates, nitrates, carboxylates, carbonates, and combinations thereof, preferably each of $X^1$ and $X^2$ is independently selected from the group consisting of halides (F, Cl, Br, I), alkyl radicals having from 1 to 7 carbon atoms (methyl, ethyl, propyl, butyl, pentyl, hexyl, heptyl, and isomers thereof), benzyl radicals, or a combination thereof.

In any embodiment of the transition metal complexes described herein, $R^1$ is a divalent $C_1$-$C_{40}$ hydrocarbyl radical or divalent substituted hydrocarbyl radical comprising a portion that comprises a linker backbone comprising from 1 to 18 carbon atoms linking or bridging between Q and Q. In an embodiment, $R^1$ is selected from the group consisting of ethylene ($-CH_2CH_2-$), 1,2-cyclohexylene and 1,2-phenylene. In an embodiment, $R^1$ is $-CH_2CH_2CH_2-$ derived from propylene. In an embodiment, $R^1$ is selected form the group consisting of $C_1$ to $C_{20}$ alkyl groups, such as divalent methyl, ethyl, ethenyl and isomers of propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, and eicosyl.

In a useful embodiment, each $R^a$, $R^b$, $R^c$, $R^d$, $R^e$, $R^f$, $R^g$, and $R^h$ is, independently, a hydrogen, a $C_1$-$C_{20}$ hydrocarbyl radical, a substituted $C_1$ to $C_{20}$ hydrocarbyl radical, or two or more of $R^1$ to $R^{10}$ may independently join together to form a $C_4$ to $C_{62}$ cyclic or polycyclic ring structure, or a combination thereof.

In any embodiment of the transition metal complexes described herein, each $R^a$, $R^b$, $R^c$, $R^d$, $R^e$, $R^f$, $R^g$, and $R^h$ is independently hydrogen, a halogen, a $C_1$ to $C_{30}$ hydrocarbyl radical, a $C_1$ to $C_{20}$ hydrocarbyl radical, or a $C_1$ to $C_{10}$ hydrocarbyl radical (such as methyl, ethyl, ethenyl and isomers of propyl, butyl, pentyl, hexyl, heptyl, octyl, nonyl,

decyl, undecyl, dodecyl, tridecyl, tetradecyl, pentadecyl, hexadecyl, heptadecyl, octadecyl, nonadecyl, eicosyl).

In any embodiment of the transition metal complexes described herein, each $R^a$, $R^b$, $R^c$, $R^d$, $R^e$, $R^f$, $R^g$, and $R^h$ is, independently, a substituted $C_1$ to $C_{30}$ hydrocarbyl radical, a substituted $C_1$ to $C_{20}$ hydrocarbyl radical, or a substituted $C_1$ to $C_{10}$ hydrocarbyl radical (such as 4-fluorophenyl, 4-chlorophenyl, 4-bromophenyl, 4-methoxyphenyl, 4-trifluoromethylphenyl, 4-dimethylaminophenyl, 4-trimethylsilylphenyl, 4-triethylsilylphenyl, trifluoromethyl, fluoromethyl, trichloromethyl, chloromethyl, mesityl, methylthio, phenylthio, (trimethylsilyl)methyl, and (triphenylsilyl)methyl).

In an embodiment of the invention, one or more of $R^a$, $R^b$, $R^c$, $R^d$, $R^e$, $R^f$, $R^g$, and $R^h$ is a methyl radical, a fluoride, chloride, bromide, iodide, methoxy, ethoxy, isopropoxy, trifluoromethyl, dimethylamino, diphenylamino, adamantyl, phenyl, pentafluorphenyl, naphthyl, anthracenyl, dimethylphosphanyl, diisopropylphosphanyl, diphenylphosphanyl, methylthio, and phenylthio or a combination thereof.

In any embodiment of the transition metal complexes described herein, Q is preferably a neutral donor group comprising at least one atom from Group 15 or Group 16, preferably Q is $NR'_2$, OR', SR', $PR'_2$, where R' is as defined for $R^a$ (preferably R' is methyl, ethyl, propyl, isopropyl, phenyl, cyclohexyl or linked together to form a five-membered ring such as pyrrolidinyl or a six-membered ring such as piperidinyl), preferably the -(-Q-$R^1$-Q-)-fragment can form a substituted or unsubstituted heterocycle which may or may not be aromatic and may have multiple fused rings. In any embodiment of the transition metal complexes described herein, Q is preferably $NR'_2$, where R' is methyl, ethyl, propyl, isopropyl, phenyl, cyclohexyl or linked together to form a five-membered ring such as pyrrolidinyl or a six-membered ring such as piperidinyl).

In a useful embodiment of the transition metal complexes described herein, $R^a$ and or $R^e$ are the same, preferably $R^a$ and or $R^e$ are C-R''', where each R''' is H or a $C_1$ to $C_{12}$ hydrocarbyl or substituted hydrocarbyl (such as methyl, ethyl, ethenyl and isomers of propyl, butyl, pentyl, hexyl, heptyl, octyl, nonyl, decyl, undecyl, dodecyl, trifluoromethylphenyl, tolyl, phenyl, methoxyphenyl, tertbutylphenyl, fluorophenyl, diphenyl, dimethylaminophenyl, chlorophenyl, bromophenyl, iodophenyl, (trimethylsilyl)phenyl, (triethylsilyl)phenyl, (trimethylsilyl)methyl, (triethylsilyl)methyl). In a useful embodiment of the transition metal complexes described herein, $R^a$ and or $R^e$ are different.

In a useful embodiment of the transition metal complexes described herein, $R^a$ and or $R^e$ are the same, preferably $R^a$ and or $R^e$ are carbazolyl, substituted carbazolyl, indolyl, substituted indolyl, indolinyl, substituted indolinyl, imidazolyl, substituted imidazolyl, indenyl, substituted indenyl, indanyl, substituted indanyl, fluorenyl, or substituted fluorenyl. In a useful embodiment of the transition metal complexes described herein, $R^a$ and or $R^e$ are different. In a useful embodiment of the transition metal complexes described herein, $R^a$ and or $R^e$ are the same.

In an embodiment, M is Zr or Hf; $X^1$ and $X^2$ are benzyl radicals; and $R^1$ is ethylene ($-CH_2CH_2-$).

In an embodiment, M is Zr or Hf; $X^1$ and $X^2$ are benzyl radicals; $R^c$ and $R^g$ are methyl radicals; $R^b$, $R^d$, $R^h$, and $R^f$ are hydrogen; and $R^1$ is ethylene ($-CH_2CH_2-$), each Q is an O-containing group, $R^a$ and $R^e$ are carbazolyl or fluorenyl.

In a particularly preferred embodiment of the invention, the catalyst complex is represented by formula:

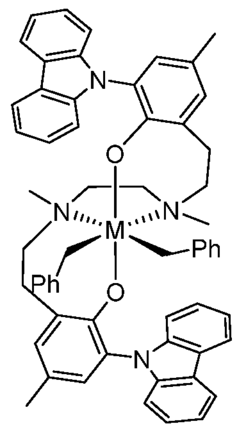
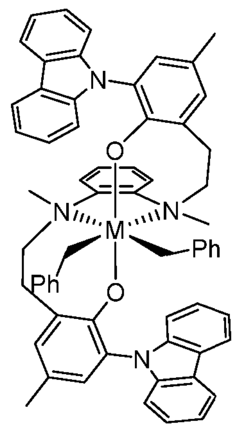
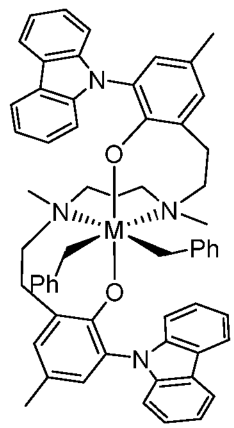

where M is preferably Zr or Hf.

<u>Methods to Prepare the Catalyst Compounds</u>

In embodiments, the transition metal compounds may be prepared by three general synthetic routes. In the first method, the phenol is allylated via a nucleophilic substitution followed by a Claisen rearrangement. The resulting allyl-substituted phenol is then protected and oxidized with ozone to the corresponding aldehyde. Reductive amination of the carbonyl with the diamine followed by deprotection results in the final ligand. Alternatively, the aldehyde can be transformed to the corresponding ethyl bromide compound, which is then reacted via nucleophilic substitution with the

precursor of the bridging group, e.g., diamine or diol. In the second method, the phenol is ortho-formylated then transformed to the vinyl phenol via a Wittig reaction. The vinyl group is oxidized to the alcohol by hydroboration-oxidation then selectively transformed to the aldehyde, which is subsequently reacted as in Method 1 to the final ligand. In the third method, 2-(2-hydroxyphenyl)acetic acid is reduced to corresponding ethyl bromide following a reduction to the alcohol. The ethyl bromide compound is then protected and reacted as in Method 1. The ligand is then typically reacted with the metal tetra-alkyl compound, e.g., tetrabenzyl, to yield the metal dibenzyl complex of the ligand.

Method 1:

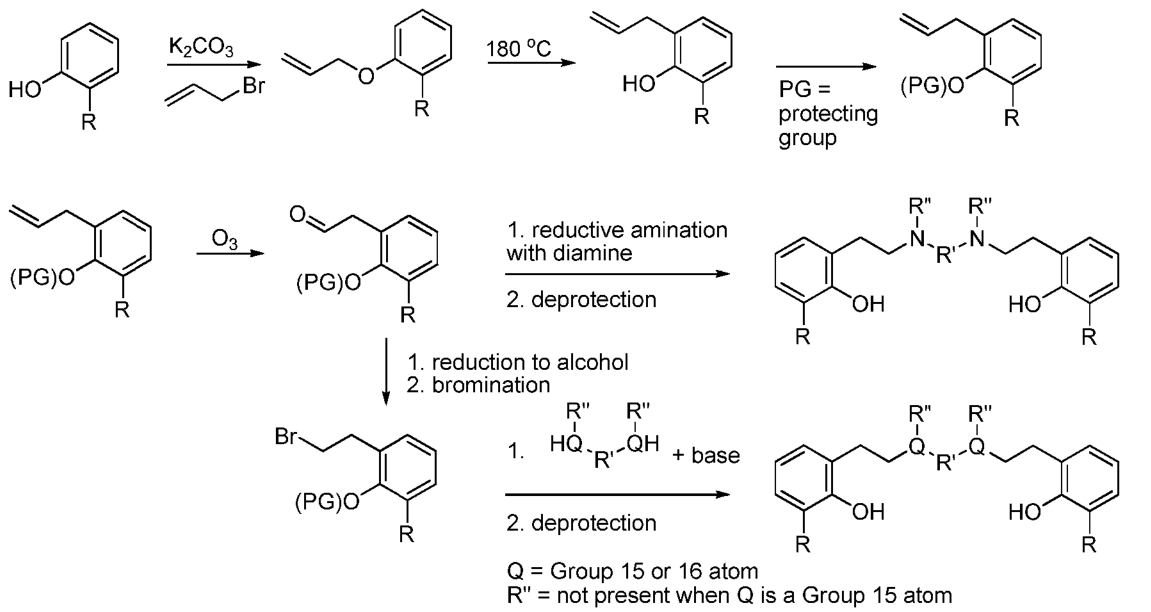


Method 2:

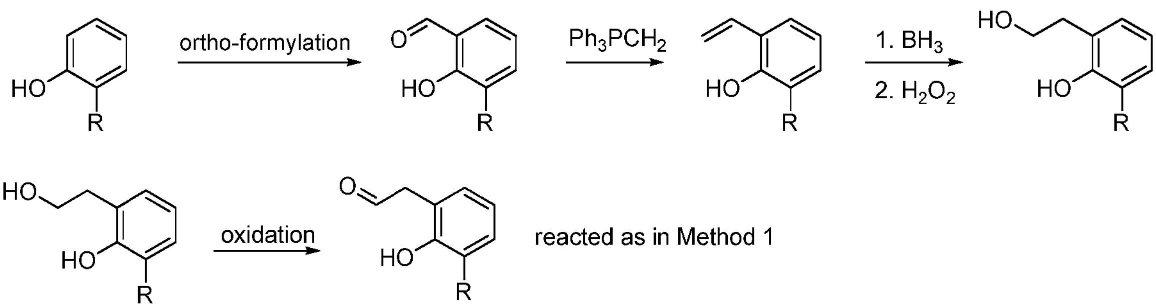


Method 3:

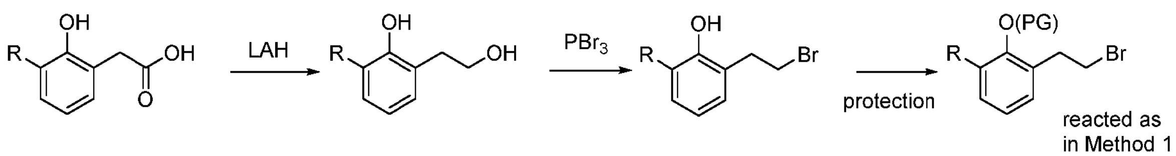


where the protection may be any useful material, for example tert-butyldimethylsilyl chloride/imidazole.

Ligand metalation:

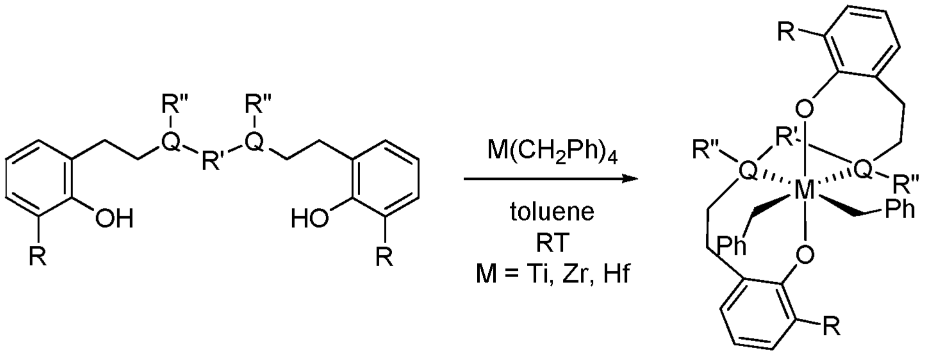


Activators

The terms "cocatalyst" and "activator" are used herein interchangeably and are defined to be any compound which can activate any one of the catalyst compounds described above by converting the neutral catalyst compound to a catalytically active catalyst compound cation.

After the complexes described above have been synthesized, catalyst systems may be formed by combining them with activators in any manner known from the literature including by supporting them for use in slurry or gas phase polymerization. The catalyst systems may also be added to or generated in solution polymerization or bulk polymerization (in the monomer). The catalyst system typically comprises a complex as described above and an activator such as alumoxane or a non-coordinating anion.

Non-limiting activators, for example, include alumoxanes, aluminum alkyls, ionizing activators, which may be neutral or ionic, and conventional-type cocatalyst. Preferred activators typically include alumoxane compounds, modified alumoxane compounds, and ionizing anion precursor compounds that abstract a reactive, σ-bound, metal ligand making the metal complex cationic and providing a charge-balancing non-coordinating or weakly coordinating anion.

Alumoxane Activators

In one embodiment, alumoxane activators are utilized as an activator in the catalyst system. Alumoxanes are generally oligomeric compounds containing -Al($R^1$)-O- sub-units, where $R^1$ is an alkyl group. Examples of alumoxanes include methylalumoxane (MAO), modified methylalumoxane (MMAO), ethylalumoxane and isobutylalumoxane. Alkylalumoxanes and modified alkylalumoxanes are suitable as catalyst activators, particularly when the abstractable ligand is an alkyl, halide, alkoxide, or amide. Mixtures of different alumoxanes and modified alumoxanes may also be used. It may be preferable to use a visually clear methylalumoxane. A cloudy or gelled alumoxane can be filtered to produce a clear solution or clear alumoxane can be decanted from the cloudy solution. A useful alumoxane is a modified methyl alumoxane (MMAO) cocatalyst type 3A (commercially available from Akzo Chemicals, Inc. under the trade name Modified Methylalumoxane type 3A, covered under patent number US 5,041,584).

When the activator is an alumoxane (modified or unmodified), some embodiments select the maximum amount of activator typically at up to a 5000-fold molar excess Al/M over the catalyst compound (per metal catalytic site). The minimum activator-to-catalyst-compound is a 1:1 molar ratio. Alternate preferred ranges include from 1:1 to 500:1, alternately from 1:1 to 200:1, alternately from 1:1 to 100:1, or alternately from 1:1 to 50:1.

In an alternate embodiment, little or no alumoxane is used in the polymerization processes described herein. Preferably, alumoxane is present at zero mole %, alternately the alumoxane is present at a molar ratio of aluminum to catalyst compound transition metal less than 500:1, preferably less than 300:1, preferably less than 100:1, preferably less than 1:1.

Non-Coordinating Anion Activators

A non-coordinating anion (NCA) is defined to mean an anion either that does not coordinate to the catalyst metal cation or that does coordinate to the metal cation, but only weakly. The term NCA is also defined to include multicomponent NCA-containing activators, such as N,N-dimethylanilinium tetrakis(pentafluorophenyl)borate, that contain an acidic cationic group and the non-coordinating anion. The term NCA is also defined to include neutral Lewis

acids, such as tris(pentafluorophenyl)boron, that can react with a catalyst to form an activated species by abstraction of an anionic group. An NCA coordinates weakly enough that a neutral Lewis base, such as an olefinically or acetylenically unsaturated monomer can displace it from the catalyst center. Any metal or metalloid that can form a compatible, weakly coordinating complex may be used or contained in the non-coordinating anion. Suitable metals include, but are not limited to, aluminum, gold, and platinum. Suitable metalloids include, but are not limited to, boron, aluminum, phosphorus, and silicon. A stoichiometric activator can be either neutral or ionic. The terms ionic activator, and stoichiometric ionic activator can be used interchangeably. Likewise, the terms neutral stoichiometric activator, and Lewis acid activator can be used interchangeably. The term non-coordinating anion includes neutral stoichiometric activators, ionic stoichiometric activators, ionic activators, and Lewis acid activators.

"Compatible" non-coordinating anions are those which are not degraded to neutrality when the initially formed complex decomposes. Further, the anion will not transfer an anionic substituent or fragment to the cation so as to cause it to form a neutral transition metal compound and a neutral by-product from the anion. Non-coordinating anions useful in accordance with this invention are those that are compatible, stabilize the transition metal cation in the sense of balancing its ionic charge at +1, and yet retain sufficient lability to permit displacement during polymerization.

It is within the scope of this invention to use an ionizing or stoichiometric activator, neutral or ionic, such as tri (n-butyl) ammonium tetrakis (pentafluorophenyl) borate, a tris perfluorophenyl boron metalloid precursor or a tris perfluoronaphthyl boron metalloid precursor, polyhalogenated heteroborane anions (WO 98/43983), boric acid (US 5,942,459), or combination thereof. It is also within the scope of this invention to use neutral or ionic activators alone or in combination with alumoxane or modified alumoxane activators.

The catalyst systems of this invention can include at least one non-coordinating anion (NCA) activator.

In a preferred embodiment boron containing NCA activators represented by the formula below can be used:

$$Z_d^+ (A^{d-})$$

where: Z is (L-H) or a reducible Lewis acid; L is a neutral Lewis base; H is hydrogen;
(L-H) is a Bronsted acid; $A^{d-}$ is a boron containing non-coordinating anion having the charge d-; d is 1, 2, or 3.

The cation component, $Z_d^+$ may include Bronsted acids such as protons or protonated Lewis bases or reducible Lewis acids capable of protonating or abstracting a moiety, such as an alkyl or aryl, from the bulky ligand metallocene containing transition metal catalyst precursor, resulting in a cationic transition metal species.

The activating cation $Z_d^+$ may also be a moiety such as silver, tropylium, carboniums, ferroceniums and mixtures, preferably carboniums and ferroceniums. Most preferably $Z_d^+$ is triphenyl carbonium. Preferred reducible Lewis acids can be any triaryl carbonium (where the aryl can be substituted or unsubstituted, such as those represented by the formula: ($Ar_3C^+$), where Ar is aryl or aryl substituted with a heteroatom, a $C_1$ to $C_{40}$ hydrocarbyl, or a substituted $C_1$ to $C_{40}$ hydrocarbyl), preferably the reducible Lewis acids in formula (14) above as "Z" include those represented by the formula: ($Ph_3C$), where Ph is a substituted or unsubstituted phenyl, preferably substituted with $C_1$ to $C_{40}$ hydrocarbyls or substituted a $C_1$ to $C_{40}$ hydrocarbyls, preferably $C_1$ to $C_{20}$ alkyls or aromatics or substituted $C_1$ to $C_{20}$ alkyls or aromatics, preferably Z is a triphenylcarbonium.

When $Z_d^+$ is the activating cation $(L\text{-}H)_d^+$, it is preferably a Bronsted acid, capable of donating a proton to the transition metal catalytic precursor resulting in a transition metal cation, including ammoniums, oxoniums, phosphoniums, silyliums, and mixtures thereof, preferably ammoniums of methylamine, aniline, dimethylamine, diethylamine, N-methylaniline, diphenylamine, trimethylamine, triethylamine, N,N-dimethylaniline, methyldiphenylamine, pyridine, p-bromo N,N-dimethylaniline, p-nitro-N,N-dimethylaniline, phosphoniums from triethylphosphine, triphenylphosphine, and diphenylphosphine, oxoniums from ethers such as dimethyl ether diethyl ether, tetrahydrofuran and dioxane, sulfoniums from thioethers, such as diethyl thioethers, tetrahydrothiophene, and mixtures thereof.

The anion component $A^{d-}$ includes those having the formula $[M^{k+} Q_n]^{d-}$ wherein k is 1, 2, or 3; n is 1, 2, 3, 4, 5, or 6 (preferably 1, 2, 3, or 4); n - k = d; M is an element selected from Group 13 of the Periodic Table of the Elements, preferably boron or aluminum, and Q is independently a hydride, bridged or unbridged dialkylamido, halide, alkoxide, aryloxide, hydrocarbyl, substituted hydrocarbyl, halocarbyl, substituted halocarbyl, and halosubstituted-hydrocarbyl radicals, said Q having up to 20 carbon atoms with the proviso that in not more than 1 occurrence is Q a halide. Preferably, each Q is a fluorinated hydrocarbyl group having 1 to 20 carbon atoms, more preferably each Q is a fluorinated aryl group, and most preferably each Q is a pentafluoryl aryl group. Examples of suitable $A^{d-}$ also include diboron compounds as disclosed in US 5,447,895.

Illustrative, but not limiting examples of boron compounds which may be used as an activating cocatalyst are the compounds described as (and particularly those specifically listed as) activators in US 8,658,556.

Most preferably, the ionic stoichiometric activator $Z_d^+ (A^{d-})$ is one or more of N,N-dimethylanilinium tetra(perfluorophenyl)borate, N,N-dimethylanilinium tetrakis(perfluoronaphthyl)borate, N,N-dimethylanilinium tetrakis(perfluorobiphenyl)borate, N,N-dimethylanilinium tetrakis(3,5-bis(trifluoromethyl)phenyl)borate, triphenylcarbenium tetrakis(perfluoronaphthyl)borate, triphenylcarbenium tetrakis(perfluorobiphenyl)borate, triphenylcarbenium tetrakis(3,5-bis(trifluoromethyl)phenyl)borate, or triphenylcarbenium tetra(perfluorophenyl)borate.

Bulky activators are also useful herein as NCAs. "Bulky activator" as used herein refers to anionic activators represented by the formula:

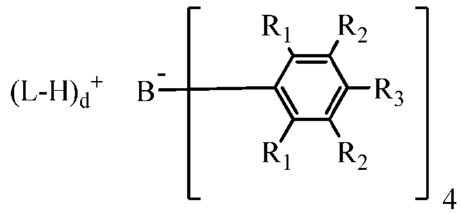

or

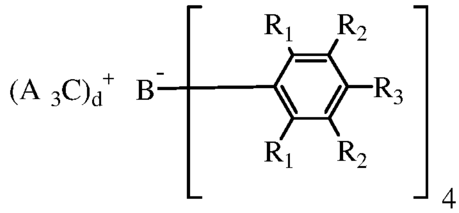

where:

- each $R_1$ is, independently, a halide, preferably a fluoride;
- Ar is substituted or unsubstituted aryl group (preferably a substituted or unsubstituted phenyl), preferably substituted with $C_1$ to $C_{40}$ hydrocarbyls, preferably $C_1$ to $C_{20}$ alkyls or aromatics;
- each $R_2$ is, independently, a halide, a $C_6$ to $C_{20}$ substituted aromatic hydrocarbyl group or a siloxy group of the formula -O-Si-$R_a$, where $R_a$ is a $C_1$ to $C_{20}$ hydrocarbyl or hydrocarbylsilyl group (preferably $R_2$ is a fluoride or a perfluorinated phenyl group);
- each $R_3$ is a halide, $C_6$ to $C_{20}$ substituted aromatic hydrocarbyl group or a siloxy group of the formula -O-Si-$R_a$, where $R_a$ is a $C_1$ to $C_{20}$ hydrocarbyl or hydrocarbylsilyl group (preferably $R_3$ is a fluoride or a $C_6$ perfluorinated aromatic hydrocarbyl group); wherein $R_2$ and $R_3$ can form one or more saturated or unsaturated, substituted or unsubstituted rings (preferably $R_2$ and $R_3$ form a perfluorinated phenyl ring);
- L is a neutral Lewis base; $(L\text{-}H)^+$ is a Bronsted acid; d is 1, 2, or 3;
- wherein the anion has a molecular weight of greater than 1020 g/mol; and
- wherein at least three of the substituents on the B atom each have a molecular volume of greater than 250 cubic Å, alternately greater than 300 cubic Å, or alternately greater than 500 cubic Å.

Preferably $(Ar_3C)_d^+$ is $(Ph_3C)_d^+$, where Ph is a substituted or unsubstituted phenyl, preferably substituted with $C_1$ to $C_{40}$ hydrocarbyls or substituted $C_1$ to $C_{40}$ hydrocarbyls, preferably $C_1$ to $C_{20}$ alkyls or aromatics or substituted $C_1$ to $C_{20}$ alkyls or aromatics.

"Molecular volume" is used herein as an approximation of spatial steric bulk of an activator molecule in solution. Comparison of substituents with differing molecular volumes allows the substituent with the smaller molecular volume to be considered "less bulky" in comparison to the substituent with the larger molecular volume. Conversely, a substituent with a larger molecular volume may be considered "more bulky" than a substituent with a smaller molecular volume.

Molecular volume may be calculated as reported in "A Simple 'Back of the Envelope' Method for Estimating the Densities and Molecular Volumes of Liquids and Solids," Journal of Chemical Education, Vol. 71, No. 11, November 1994, pp. 962-964. Molecular volume (MV), in units of cubic Å, is calculated using the formula: $MV = 8.3V_S$, where $V_S$ is the scaled volume. $V_S$ is the sum of the relative volumes of the constituent atoms, and is calculated from the molecular formula of the substituent using the following table of relative volumes. For fused rings, the $V_S$ is decreased by 7.5% per fused ring.

| Element | Relative Volume |
|---|---|
| H | 1 |
| 1st short period, Li to F | 2 |
| 2nd short period, Na to Cl | 4 |
| 1st lons period, K to Br | 5 |
| 2nd lons period, Rb to I | 7.5 |
| 3rd lons period, Cs to Bi | 9 |

For a list of particularly useful Bulky activators please see US 8,658,556.

In another embodiment, one or more of the NCA activators is chosen from the activators described in US 6,211,105.

Preferred activators include N,N-dimethylanilinium tetrakis(perfluoronaphthyl)borate, N,N-dimethylanilinium tetrakis(perfluorobiphenyl)borate, N,N-dimethylanilinium tetrakis(perfluorophenyl)borate, N,N-dimethylanilinium tetrakis(3,5-bis(trifluoromethyl)phenyl)borate, triphenylcarbenium tetrakis(perfluoronaphthyl)borate, triphenylcarbenium tetrakis(perfluorobiphenyl)borate, triphenylcarbenium tetrakis(3,5-bis(trifluoromethyl)phenyl)borate, triphenylcarbenium tetrakis(perfluorophenyl)borate, $[Ph_3C^+][B(C_6F_5)_4^-]$, $[Me_3NH^+][B(C_6F_5)_4^-]$, 1-(4-(tris(pentafluorophenyl)borate)-2,3,5,6-tetrafluorophenyl)pyrrolidinium, and tetrakis(pentafluorophenyl)b orate, 4-(tris(pentafluorophenyl)borate)-2,3,5,6-tetrafluoropyridine.

In a preferred embodiment, the activator comprises a triaryl carbonium (such as triphenylcarbenium tetraphenylborate, triphenylcarbenium tetrakis(pentafluorophenyl)borate, triphenyl carbenium tetrakis-(2,3,4, 6-tetrafluorophenyl)borate, triphenylcarbenium tetrakis(perfluoronaphthyl)borate, triphenylcarbenium tetrakis(perfluorobiphenyl)borate, or triphenylcarbenium tetrakis(3,5-bis(trifluoromethyl)phenyl)borate).

In another embodiment, the activator comprises one or more of trialkylammonium tetrakis(pentafluorophenyl)borate, N,N-dialkylanilinium tetrakis(pentafluorophenyl)borate, N,N-dimethyl-(2,4,6-trimethylanilinium) tetrakis(pentafluorophenyl)borate, trialkylammonium tetrakis-(2,3,4,6-tetrafluorophenyl) borate, N,N-dialkylanilinium tetrakis-(2,3,4,6-tetrafluorophenyl)borate, trialkylammonium tetrakis(perfluoronaphthyl)borate, N,N-dialkylanilinium tetrakis(perfluoronaphthyl)borate, trialkylammonium tetrakis(perfluorobiphenyl)borate, N,N-dialkylanilinium tetrakis(perfluorobiphenyl)borate, trialkylammonium tetrakis(3,5-bis(trifluoromethyl)phenyl)borate, N,N-dialkylanilinium tetrakis(3,5-bis(trifluoromethyl)phenyl)borate, N,N-dialkyl-(2,4,6-trimethylanilinium) tetrakis(3,5-bis(trifluoromethyl)phenyl)borate, di-(i-propyl)ammonium tetrakis(pentafluorophenyl)borate, (where alkyl is methyl, ethyl, propyl, n-butyl, sec-butyl, or t-butyl).

The typical activator-to-catalyst ratio, e.g., all NCA activators-to-catalyst ratio is about a 1:1 molar ratio. Alternate preferred ranges include from 0.1:1 to 100:1, alternately from 0.5:1 to 200:1, alternately from 1:1 to 500:1, alternately from 1:1 to 1000:1. A particularly useful range is from 0.5:1 to 10:1, preferably 1:1 to 5:1.

It is also within the scope of this invention that the catalyst compounds can be combined with combinations of alumoxanes and NCA's (see, for example, US 5,153,157; US 5,453,410; EP 0 573 120; WO 94/07928; and WO 95/14044, which discuss, inter alia, the use of an alumoxane in combination with an ionizing activator).

Scavengers and Co-Activators

The catalyst system may further include scavengers and/or co-activators. In some embodiments, when using the complexes described herein, particularly when they are immobilized on a support, the catalyst system will additionally comprise one or more scavenging compounds. Here, the term scavenging compound means a compound that removes polar impurities from the reaction environment. A scavenger is typically added to facilitate polymerization by scavenging impurities. Some scavengers may also act as activators and may be referred to as co-activators. A co-activator, that is not a scavenger, may also be used in conjunction with an activator in order to form an active catalyst. In some embodiments, a co-activator can be pre-mixed with the transition metal compound to form an alkylated transition metal compound.

Typically, the scavenging compound will be an organometallic compound such as the Group-13 organometallic compounds of US 5,153,157; US 5,241,025; WO-A-91/09882; WO-A-94/03506; WO-A-93/14132; and that of WO 95/07941. Exemplary compounds include triethyl aluminum, triethyl borane, tri-*iso*-butyl aluminum, methyl alumoxane, *iso*-butyl alumoxane, and tri-n-octyl aluminum. Those scavenging compounds having bulky or $C_6$-$C_{20}$ linear hydrocarbyl substituents connected to the metal or metalloid center usually minimize adverse interaction with the active catalyst. Examples include triethylaluminum, but more preferably, bulky compounds such as tri-*iso*-butyl aluminum, tri-*iso*-prenyl aluminum, and long-chain linear alkyl-substituted aluminum compounds, such as tri-n-hexyl aluminum, tri-n-octyl aluminum, or tri-n-dodecyl aluminum. When alumoxane is used as the activator, any excess over that needed for activation will scavenge impurities and additional scavenging compounds may be unnecessary. Alumoxanes also may be added in scavenging quantities with other activators, e.g., methylalumoxane, $[Me_2HNPh]^+[B(pfp)_4]^-$ or $B(pfp)_3$ (perfluorophenyl = pfp = $C_6F_5$). In an embodiment, the scavengers are present at less than 14 wt%, or from 0.1 to 10 wt%, or from 0.5 to 7 wt%, by weight of the catalyst system.

Suitable aluminum alkyl or organoaluminum compounds which may be utilized as co-activators include, for example, trimethylaluminum, triethylaluminum, triisobutylaluminum, tri-n-hexylaluminum, tri-n-octylaluminum and the like. In an embodiment, the co-activators are present at less than 14 wt%, or from 0.1 to 10 wt%, or from 0.5 to 7 wt%, by weight of the catalyst system. Alternately, the complex-to-co-activator molar ratio is from 1:100 to 100:1; 1:75 to 75:1; 1:50 to 50:1; 1:25 to 25:1; 1:15 to 15:1; 1:10 to 10:1; 1:5 to 5:1; 1:2 to 2:1; 1:100 to 1:1; 1:75 to 1:1; 1:50 to 1:1; 1:25 to 1:1; 1:15 to 1:1; 1:10 to 1:1; 1:5 to 1:1; 1:2 to 1:1; 1:10 to 2:1.

## Chain Transfer Agents (CTAs)

A "chain transfer agent" is any agent capable of hydrocarbyl and/or polymeryl group exchange between a coordinative polymerization catalyst and the metal center of the chain transfer agent during a polymerization process. The chain transfer agent can be any desirable chemical compound such as those disclosed in WO 2007/130306. Preferably, the chain transfer agent is selected from Group 2, 12, or 13 alkyl or aryl compounds; preferably zinc, magnesium or aluminum alkyls or aryls; preferably where the alkyl is a $C_1$ to $C_{30}$ alkyl, alternately a $C_2$ to $C_{20}$ alkyl, alternately a $C_3$ to $C_{12}$ alkyl, typically selected independently from methyl, ethyl, propyl, butyl, isobutyl, tertbutyl, pentyl, hexyl, cyclohexyl, phenyl, octyl, nonyl, decyl, undecyl, and dodecyl; and where di-ethylzinc is particularly preferred.

In a particularly useful embodiment, this invention relates to a catalyst system comprising activator, catalyst complex as described herein, and chain transfer agent wherein the chain transfer agent is selected from Group 2, 12, or 13 alkyl or aryl compounds.

In a particularly useful embodiment, the chain transfer agent is selected from dialkyl zinc compounds, where the alkyl is selected independently from methyl, ethyl, propyl, butyl, isobutyl, tertbutyl, pentyl, hexyl, cyclohexyl, and phenyl.

In a particularly useful embodiment, the chain transfer agent is selected from trialkyl aluminum compounds, where the alkyl is selected independently from methyl, ethyl, propyl, butyl, isobutyl, tertbutyl, pentyl, hexyl, and cyclohexyl.

In a particularly useful embodiment, the chain transfer agent is selected from tri aryl aluminum compounds where the aryl is selected from phenyl and substituted phenyl.

The inventive process may be characterized by the transfer of at least 0.5 polymer chains (preferably 0.5 to 3) polymer chains, where n is the maximum number of polymer chains that can be transferred to the chain transfer agent metal, preferably n is 1 to 3 for trivalent metals (such as Al) and 1 to 2 for divalent metals (such as Zn), preferably n is 1.5 to 3 for trivalent metals (such as Al) and 1.5 - 2 for divalent metals (such as Zn). The number of chains transferred per metal is the slope of the plot of moles of polymer produced versus the moles of the chain transfer agent metal (as determined from at least four points, CTA metal:catalyst transition metal of 20:1, 80:1, 140:1 and 200:1, using least squares fit (Microsoft™ Excel 2010, version 14.0.7113.5000 (32bit)) to draw the line. For example, in Table 10, for entries 1-8, the slope is 0.0682 and for entries 17-24, the slope is 0.7593.

Useful chain transfer agents are typically present at from 10 or 20 or 50 or 100 equivalents to 600 or 700 or 800 or 1000 or 2000 or 4000 equivalents relative to the catalyst component. Alternately the chain transfer agent is preset at a catalyst complex-to-CTA molar ratio of from about 1:12,000 to 10:1; alternatively 1:6,000; alternatively, 1:3,000 to 10:1; alternatively 1:2,000 to 10:1; alternatively 1:1,000 to 10:1; alternatively, 1:500 to 1:1; alternatively 1:300 to 1:1; alternatively 1:200 to 1:1; alternatively 1:100 to 1:1; alternatively 1:50 to 1:1; alternatively 1:10 to 1:1.

Useful chain transfer agents include diethylzinc, tri-n-octyl aluminum, trimethylaluminum, triethylaluminum, tri-isobutylaluminum, tri-n-hexylaluminum, diethyl aluminum chloride, dibutyl zinc, di-n-propylzinc, di-n-hexylzinc, di-n-pentylzinc, di-n-decylzinc, di-n-dodecylzinc, di-n-tetradecylzinc, di-n-hexadecylzinc, di-n-octadecylzinc, diphenylzinc, diisobutylaluminum hydride, diethylaluminum hydride, di-n-octylaluminum hydride, dibutylmagnesium, diethylmagnesium, dihexylmagnesium, and triethylboron.

In a preferred embodiment, two or more complexes are combined with diethyl zinc and/or tri-n-octylaluminum in the same reactor with monomer(s). Alternately, one or more complexes is/are combined with another catalyst (such as a metallocene) and diethyl zinc and/or tri-n-octylaluminum in the same reactor with monomer(s).

In a preferred embodiment, one or more complexes is/are combined with a mixture of diethyl zinc and an aluminum reagent in the same reactor with monomer(s). Alternately, one or more complexes is/are combined with two chain transfer agents in the same reactor with monomer(s).

## Supports

In some embodiments, the complexes described herein may be supported (with or without an activator) by any method effective to support other coordination catalyst systems, effectively meaning that the catalyst so prepared can be used for oligomerizing or polymerizing olefin in a heterogeneous process. The catalyst precursor, activator, co-activator, if needed, suitable solvent, and support may be added in any order or simultaneously. Typically, the complex and activator may be combined in solvent to form a solution. Then the support is added, and the mixture is stirred for 1 minute to 10 hours. The total solution volume may be greater than the pore volume of the support, but some embodiments limit the total solution volume below that needed to form a gel or slurry (about 90% to 400%, preferably about 100 to 200% of the pore volume). After stirring, the residual solvent is removed under vacuum, typically at ambient temperature and over 10-16 hours. But greater or lesser times and temperatures are possible.

The complex may also be supported absent the activator; in that case, the activator (and co-activator if needed) is added to a polymerization process's liquid phase. Additionally, two or more different complexes may be placed on the same support. Likewise, two or more activators or an activator and co-activator may be placed on the same support.

Suitable solid particle supports are typically comprised of polymeric or refractory oxide materials, each being preferably porous. Preferably any support material that has an average particle size greater than 10 μm is suitable for use in this invention. Various embodiments select a porous support material, such as for example, talc, inorganic oxides, inorganic chlorides, for example, magnesium chloride and resinous support materials such as polystyrene polyolefin or polymeric compounds or any other organic support material and the like. Some embodiments select inorganic oxide materials as the support material including Group -2, -3, -4, -5, -13, or -14 metal or metalloid oxides. Some embodiments select the catalyst support materials to include silica, alumina, silica-alumina, and their mixtures. Other inorganic oxides may serve either alone or in combination with the silica, alumina, or silica-alumina. These are magnesia, titania, zirconia, and the like. Lewis acidic materials such as montmorillonite and similar clays may also serve as a support. In this case, the support can, optionally, double as the activator component, however, an additional activator may also be used.

The support material may be pretreated by any number of methods. For example, inorganic oxides may be calcined, chemically treated with dehydroxylating agents, such as aluminum alkyls and the like, or both.

As stated above, polymeric carriers will also be suitable in accordance with the invention, see for example the descriptions in WO 95/15815 and US 5,427,991. The methods disclosed may be used with the catalyst complexes, activators or catalyst systems of this invention to adsorb or absorb them on the polymeric supports, particularly if made up of porous particles, or may be chemically bound through functional groups bound to or in the polymer chains.

Useful supports typically have a surface area of from 10-700 $m^2/g$, a pore volume of 0.1-4.0 cc/g and an average particle size of 10-500 μm. Some embodiments select a surface area of 50-500 $m^2/g$, a pore volume of 0.5-3.5 cc/g, or an average particle size of 10-200 μm. Other embodiments select a surface area of 100-400 $m^2/g$, a pore volume of 0.8-3.0 cc/g, and an average particle size of 50-100 μm. Useful supports typically have a pore size of 10-1000 Angstroms, alternatively 50-500 Angstroms, or 75-350 Angstroms.

The catalyst complexes described herein are generally deposited on the support at a loading level of 10-100 micromoles of complex per gram of solid support; alternately 20-80 micromoles of complex per gram of solid support; or 40-60 micromoles of complex per gram of support. But greater or lesser values may be used provided that the total amount of solid complex does not exceed the support's pore volume.

Polymerization

Inventive catalyst complexes are useful in polymerizing unsaturated monomers conventionally known to undergo metallocene-catalyzed polymerization such as solution, slurry, gas-phase, and high-pressure polymerization. Typically one or more of the complexes described herein, one or more activators, and one or more monomers are contacted to produce polymer. In certain embodiments, the complexes may be supported and as such will be particularly useful in the known, fixed-bed, moving-bed, fluid-bed, slurry, solution, or bulk operating modes conducted in single, series, or parallel reactors.

One or more reactors in series or in parallel may be used in the present invention. The complexes, activator and when required, co-activator, may be delivered as a solution or slurry, either separately to the reactor, activated in-line just prior to the reactor, or preactivated and pumped as an activated solution or slurry to the reactor. Polymerizations are carried out in either single reactor operation, in which monomer, comonomers, catalyst/activator/co-activator, optional scavenger, and optional modifiers are added continuously to a single reactor or in series reactor operation, in which the above components are added to each of two or more reactors connected in series. The catalyst components can be added to the first reactor in the series. The catalyst component may also be added to both reactors, with one component being added to first reaction and another component to other reactors. In one preferred embodiment, the complex is activated in the reactor in the presence of olefin.

In a particularly preferred embodiment, the polymerization process is a continuous process.

Polymerization processes used herein typically comprise contacting one or more alkene monomers with the complexes (and, optionally, activator) described herein. For purpose of this invention alkenes are defined to include multi-alkenes (such as dialkenes) and alkenes having just one double bond. Polymerization may be homogeneous (solution or bulk polymerization) or heterogeneous (slurry - in - liquid diluent, or gas phase - in - gaseous diluent). In the case of heterogeneous slurry or gas phase polymerization, the complex and activator may be supported. Silica is useful as a support herein. Chain transfer agents may also be used herein.

The present polymerization processes may be conducted under conditions preferably including a temperature of about 30°C to about 200°C, preferably from 60°C to 195°C, preferably from 75°C to 190°C. The process may be conducted at a pressure of from 0.05 MPa to 1500 MPa. In a preferred embodiment, the pressure is between 1.7 MPa and 30 MPa, or in another embodiment, especially under supercritical conditions, the pressure is between 15 MPa and 1500 MPa.

Monomers

Monomers useful herein include olefins having from 2 to 20 carbon atoms, alternately 2 to 12 carbon atoms (preferably ethylene, propylene, butylene, pentene, hexene, heptene, octene, nonene, decene, and dodecene) and, optionally, also polyenes (such as dienes). Particularly preferred monomers include ethylene, and mixtures of $C_2$ to $C_{10}$ alpha olefins, such as ethylene-propylene, ethylene-hexene, ethylene-octene, propylene-hexene, and the like.

The complexes described herein are also particularly effective for the polymerization of ethylene, either alone or in combination with at least one other olefinically unsaturated monomer, such as a $C_3$ to $C_{20}$ $\alpha$-olefin, and particularly a $C_3$ to $C_{12}$ $\alpha$-olefin. Likewise, the present complexes are also particularly effective for the polymerization of propylene, either alone or in combination with at least one other olefinically unsaturated monomer, such as ethylene or a $C_4$ to $C_{20}$ $\alpha$-olefin, and particularly a $C_4$ to $C_{20}$ $\alpha$-olefin. Examples of preferred $\alpha$-olefins include ethylene, propylene, butene-1, pentene-1, hexene-1, heptene-1, octene-1, nonene-1, decene-1, dodecene-1, 4-methylpentene-1, 3-methylpentene-1, 3,5,5-trimethylhexene-1, and 5-ethylnonene-1.

In some embodiments, the monomer mixture may also comprise one or more dienes at up to 10 wt%, such as from 0.00001 to 1.0 wt%, for example, from 0.002 to 0.5 wt%, such as from 0.003 to 0.2 wt%, based upon the monomer mixture. Non-limiting examples of useful dienes include, cyclopentadiene, norbornadiene, dicyclopentadiene, 5-ethylidene-2-norbornene ("ENB"), 5-vinyl-2-norbornene, 1,4-hexadiene, 1,5-hexadiene, 1,5-heptadiene, 1,6-heptadiene, 6-methyl-1,6-heptadiene, 1,7-octadiene, 7-methyl-1,7-octadiene, 1,9-decadiene, 1 and 9-methyl-1,9-decadiene.

In a useful embodiment of the invention, the monomers comprise ethylene and one or more $C_3$ to $C_{12}$ alkenes, such as propylene.

Particularly preferred monomers combinations include: ethylene-propylene, ethylene-hexene, ethylene-octene, and the like.

Where olefins are used that give rise to short chain branching, such as propylene, the catalyst systems may, under appropriate conditions, generate stereoregular polymers or polymers having stereoregular sequences in the polymer chains.

Polymer Products

The homopolymer and copolymer products produced by the present process may have an Mw of about 1,000 to about 2,000,000 g/mol, alternately of about 30,000 to about 600,000 g/mol, or alternately of about 100,000 to about 500,000 g/mol, as determined by GPC. Preferred polymers produced here may be homopolymers or copolymers. In a preferred embodiment, the comonomer(s) are present at up to 50 mol%, preferably from 0.01 to 40 mol%, preferably 1 to 30 mol%, preferably from 5 to 20 mol%. In some embodiments herein, a multimodal polyolefin composition is produced, comprising a first polyolefin component and at least another polyolefin component, different from the first polyolefin component by molecular weight, preferably such that the GPC trace has more than one peak or inflection point.

Unless otherwise indicated, measurements of weight average molecular weight (Mw), number average molecular weight (Mn), and z average molecular weight (Mz) are determined by the GPC-SEC procedure as described below in the Experimental section.

In a preferred embodiment, the homopolymer and copolymer products produced by the present process may have an Mw of about 1,000 to about 2,000,000 g/mol, alternately of about 30,000 to about 600,000 g/mol, or alternately of about 100,000 to about 500,000 g/mol, as determined by GPC-SEC.

In an alternate embodiment, the homopolymer and copolymer products produced by the present process may have a multi-modal, such as bimodal, Mw/Mn.

The term "multimodal," when used to describe a polymer or polymer composition, means "multimodal molecular weight distribution," which is understood to mean that the Gel Permeation Chromatography (GPC-SEC) trace, plotted as Absorbance versus Retention Time (seconds), has more than one peak or inflection point. An "inflection point" is that point where the second derivative of the curve changes in sign (e.g., from negative to positive or vice versa). For example, a polyolefin composition that includes a first lower molecular weight polymer component (such as a polymer having an Mw of 100,000 g/mol) and a second higher molecular weight polymer component (such as a polymer having an Mw of 300,000 g/mol) is considered to be a "bimodal" polyolefin composition.

In an alternate embodiment, the polymer produced herein has an Mw/Mn of from 1 to 40, alternately from greater than 1 to 5.

End Uses

Articles made using polymers produced herein may include, for example, molded articles (such as containers and bottles, e.g., household containers, industrial chemical containers, personal care bottles, medical containers, fuel tanks, and storage ware, toys, sheets, pipes, tubing) films, non-wovens, and the like. It should be appreciated that the

list of applications above is merely exemplary, and is not intended to be limiting.

## EXPERIMENTAL

$[H_2CO]_x$ is paraformaldehyde.

Example 1

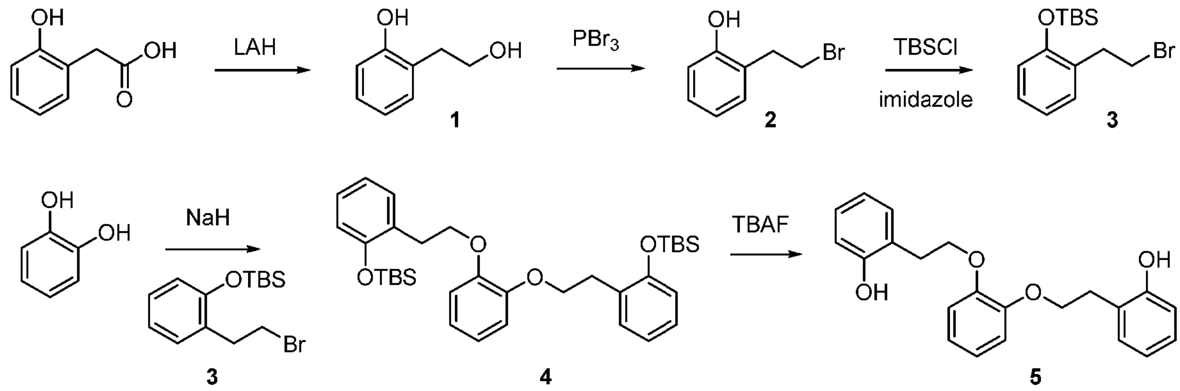


1 2 3 3 4 5

**2-(2-hydroxyethyl)phenol (1):** 2-(2-hydroxyphenyl)acetic acid (5.0 g, 32 mmol) was dissolved in 160 mL of THF and cooled to -35°C. Borane (49 mL of 1.0 M in THF) was slowly added and the reaction warmed to ambient temperature. After 4 h, the mixture was cooled to 0°C and quenched with concentrated HCl. Once warmed to ambient, saturated ammonium chloride was added and THF removed by distillation under reduced pressure. The aqueous portion was extracted with ethyl acetate, which was then dried over $MgSO_4$, filtered, and concentrated to give the reduced alcohol in quantitative yield. $^1H$ NMR (400 MHz, $CDCl_3$, $\delta$): 2.19 (t, J = 8.0 Hz, 2 H), 4.01 (t, J = 8.0 Hz, 2 H), 6.92 (m, 4 H).

**2-(2-bromoethyl)phenol (2):** Alcohol **1** (2.0 g, 14.4 mmol) was dissolved in 30 mL of dichloromethane and cooled to -35°C. Phosphorous tribromide (2.0 mL, 21.7 mmol) was slowly added and the reaction warmed to ambient temperature over several hours. The reaction was poured onto ice and extracted with methylene chloride. The combined organic layers were washed with saturated sodium bicarbonate, dried over $MgSO_4$,filtered, and concentrated to give the product as a pale yellow oil in 62% yield. $^1H$ NMR (400 MHz, $CDCl_3$, $\delta$).

**(2-(2-bromoethyl)phenoxy)(tert-butyl)dimethylsilane (3):** Bromide **2** (1.8 g, 8.9 mmol), imidazole (2.2 g, 23.9 mmol), and tert-butyldimethylsilyl chloride (1.4 g, 9.79 mmol) were dissolved in 30 mL of acetonitrile and stirred at ambient temperature for over 24 h. Water was added and the mixture extracted with 2 portions of ethyl acetate. Combined organic layers were washed with brine, dried over $MgSO_4$, filtered through a silica gel plug, and then concentrated to give a protected phenol. $^1H$ NMR (400 MHz, $CDCl_3$, $\delta$): 0.26 (s, 6 H), 1.03 (s, 9 H), 3.17 (t, *J* = 8.0 Hz, 2 H), 3.54 (t, *J* = 8.0 Hz, 2 H), 6.87 (m, 2 H), 7.11 (m, 2 H).

**1,2-bis(2-((tert-butyldimethylsilyl)oxy)phenethoxy)benzene (4):** Catechol (104 mg, 0.95 mmol) was dissolved in 15 mL of THF and cooled to 0°C. Sodium hydride (101 mg, 3.99 mmol) was added and the mixture allowed to warm to ambient temperature and stirred for 30 min. The catecholate salt was added to a solution of bromide **3** (0.6 g, 1.9 mmol) in 5 mL of THF and stirred for 5 h. The reaction was quenched with ice water and extracted with ether. Combined organic layers were washed with brine, dried over $MgSO_4$, filtered, and concentrated under reduced pressure to give the product as a pale yellow oil.

Example A

## Synthesis of 1-Zr and 1-Hf

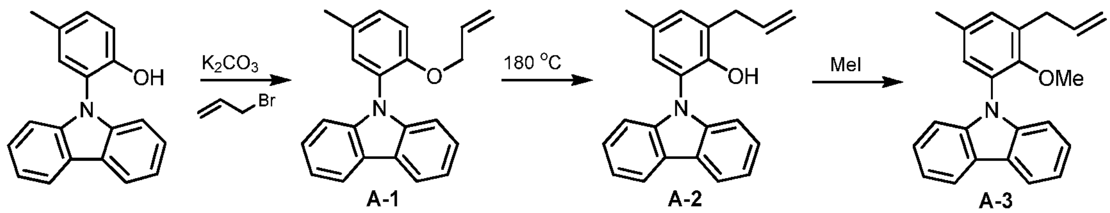


A-1 A-2 A-3

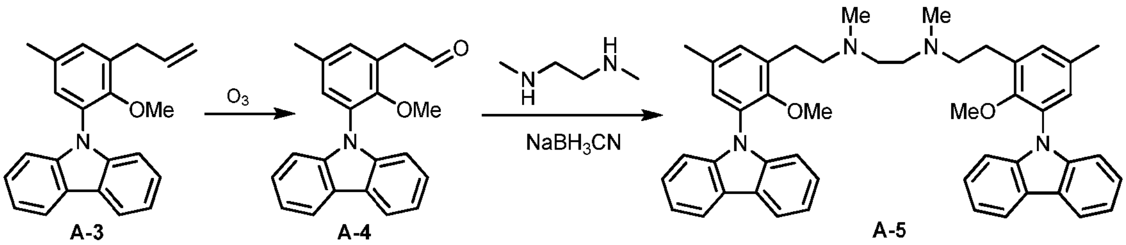


A-3 A-4 A-5

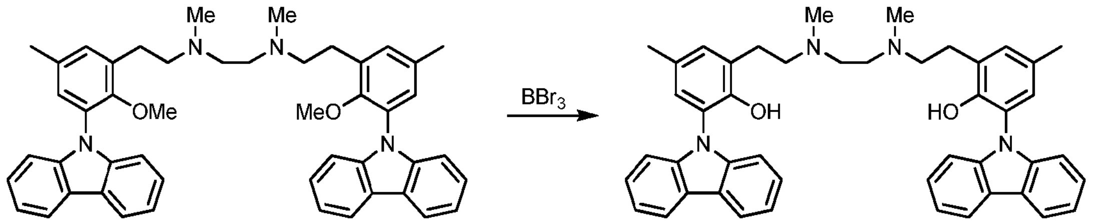


A-5 A-6

**9-(2-(Allyloxy)-5-methylphenyl)-9*H*-carbazole (A-1).** Allyl bromide (19 mL, 220 mmol, 1.2 equiv) was added to a solution of compound 2-(9*H*-carbazol-9-yl)-4-methylphenol (50.0 g, 183 mmol, 1 equiv) and anhydrous potassium carbonate (50.6 g, 366 mmol, 2 equiv) in acetone (1 L). After refluxing overnight, the reaction was cooled to room temperature, and filtered, washing the filter cake with acetone (500 mL). The filtrate was concentrated under reduced pressure to give compound A-1 (53.6 g, 94% yield) as an off white solid which was used subsequently.

**2-Allyl-6-(9*H*-carbazol-9-yl)-4-methylphenol (A-2).** Compound A-1 (53.6 g, 171 mmol, 1 equiv) was dissolved in 1,2-dichlorobenzene (186 mL) in a 500 mL sealed glass pressure vessel and heated to 180°C for 6 days. The solution was purified over silica gel (1 kg) eluting with a gradient of 0 to 50% toluene in heptanes to give compound A-2 (47.4 g, 88% yield) as a tan oil that slowly solidified.

**9-(3-Allyl-2-methoxy-5-methylphenyl)-9*H*-carbazole (A-3).** Iodomethane (3.0 mL, 48.8 mmol, 1.5 equiv) was added to a solution of compound A-2 (10.2 g, 32.5 mmol, 1 equiv) and anhydrous potassium carbonate (6.7 g, 48.8 mmol, 1.5 equiv) in acetone (200 mL). After refluxing overnight, the reaction was cooled to room temperature and filtered. The filter cake was washed with acetone (50 mL). The filtrate was concentrated under reduced pressure and the residue was partially purified on an AnaLogix column (120 g), eluting with a gradient of 0 to 40% toluene in heptanes. The mixed fractions were purified on an AnaLogix column (25 g) eluting with a gradient of 0 to 40% toluene in heptanes. The clean material from each column was combined to compound A-3 (7.7 g, 72% yield) as a colorless oil that slowly solidified.

**2-(3-(9*H*-Carbazol-9-yl)-2-methoxy-5-methylphenyl)acetaldehyde (A-4).** Compound A-3, 5.9 g, 18 mmol, 1 equiv) was dissolved in dichloromethane (120 mL) and methanol (140 mL) and cooled to -78°C. Ozone was bubbled through the solution for 50 minutes until a yellow color persisted. The solution was sparged with air for an extra 10 minutes. The solution was quenched with dimethyl sulfide (2.5 mL) and stirred while warming to room temperature for 5 hours. The solution was concentrated under reduced pressure and the residue was partially purified on an AnaLogix column (80 g), eluting with a gradient of 0 to 50% ethyl acetate in heptanes. The mixed fractions were purified on an AnaLogix column (25 g), eluting with a gradient of 0 to 50% ethyl acetate in heptanes. The clean material from each column was combined to give compound A-4 (4.2 g, 71% yield) as a white solid.

***N,N'*-Bis(3-(9*H*-carbazol-9-yl)-2-methoxy-5-methylphenethyl)-*N,N'*-dimethylethane-1,2-diamine (A-5).** *N,N'*-Dimethylethane-1,2-diamine (0.53 mL, 4.9 mmol, 1 equiv) was added to a solution compound A-4 (3.9 g, 12 mmol, 2.4 equiv) and sodium cyanoborohydride (1.23 g, 19.6 mmol, 4 equiv) in methanol (156 mL). Acetic acid (45 drops) was added and the solution was stirred at room temperature overnight. The solution was concentrated under reduced pressure. The residue was purified on an AnaLogix column (40 g), eluting with a gradient of 0 to 10% methanol in dichloromethane, to give compound A-5 (3.7 g, > theoretical yield) as a colorless oil that slowly solidified.

**6,6'-((Ethane-1,2-diylbis(methylazanediyl))bis(ethane-2,1-diyl))bis(2-(9*H*-carbazol-9-yl)-4-methylphenol) (A-6).** Boron tribromide (3.0 mL, 31 mmol, 6.4 equiv) as added dropwise to solution of compound A-5 (3.5 g, 4.9 mmol, 1 equiv) in anhydrous dichloromethane (100 mL) at -78°C. The solution was stirred at -78°C for 4 hours and warmed to room temperature overnight. The solution was carefully diluted with ice water (100 mL). The layers were separated and the aqueous layer was extracted with dichloromethane (100 mL). The combined organic layers were dried over sodium sulfate and concentrated under reduced pressure. The crude reside was dry loaded onto silica (8 g) and partially purified on an AnaLogix column (40 g), eluting with a gradient of 0 to 100% ethyl acetate in heptanes. The mixed fractions from the first column were partially purified on an AnaLogix column (40 g), eluting with a gradient of 0 to 100% methyl tert-butyl ether in heptanes. The mixed fractions from the second column were partially purified in batches, two runs on an AnaLogix Reverse Phase column (100 g) and seven runs on an AnaLogix Reverse Phase column (50 g), eluting each batch with a gradient of 0 to 100% tetrahydrofuran in deionized water. The mixed fractions from the reverse phase columns were partially purified on an AnaLogix column (12 g) eluting isocratically with a solution of 98:2:1 dichloromethane:methyl tert-butyl ether:ammonia. The mixed fractions were partially purified on an AnaLogix column (25 g), eluting isocratically with a solution of 98:2:1 dichloromethane:methyl tert-butyl ether:ammonia. The mixed fractions were purified one last time on an AnaLogix column (12 g), eluting isocratically with a solution of 98:2:1 dichloromethane: methyl tert-butylether:ammonia. All of the clean material was combined to give compound A-6 (1.42 g, 42% yield) as a white solid.

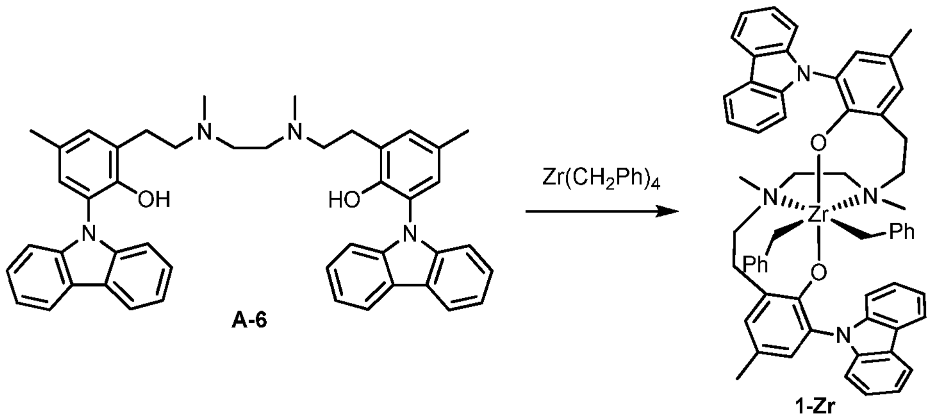


A-6 1-Zr

**6,6'-((Ethane-1,2-diylbis(methylazanediyl))bis(ethane-2,1-diyl))bis(2-(9*H*-carbazol-9-yl)-4-methylphenoxide) zirconium dibenzyl (1-Zr).** In the drybox, $Zr(CH_2Ph)_4$ (0.0996 g, 0.219 mmol, 1 equiv) was dissolved in toluene (3 mL), and the resulting orange solution was added to a slurry of compound A-6 (0.1500 g, 0.2184 mmol, 1 equiv) in toluene (2 mL). The mixture was stirred for 2.5 hours at room temperature then concentrated under a stream of nitrogen. The resulting yellow-orange residue was washed with hexane (5 mL) and dried under vacuum to give 1-Zr (0.1836 g, ~88% yield) as a yellow powder.

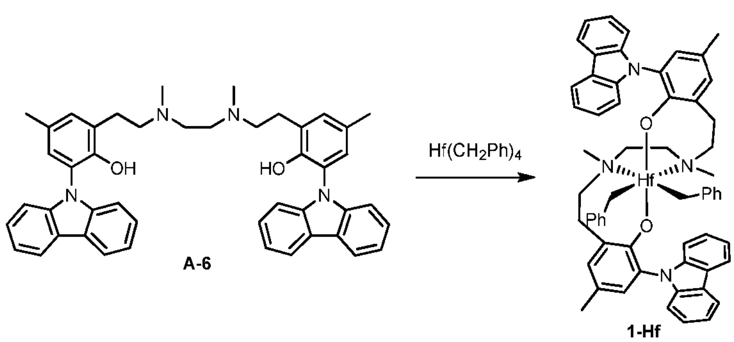


A-6 1-Hf

**6,6'-((Ethane-1,2-diylbis(methylazanediyl))bis(ethane-2,1-diyl))bis(2-(9*H*-carbazol-9-yl)-4-methylphenoxide) hafnium dibenzyl (1-Hf).** In the drybox, $Hf(CH_2Ph)_4$ (0.0786 g, 0.145 mmol, 1 equiv) was dissolved in toluene (3 mL), and the resulting pale yellow solution was added to a slurry of compound A-6 (0.0998 g, 0.145 mmol, 1 equiv) in toluene (2 mL). The mixture was stirred for 2.5 hours at room temperature then concentrated under a stream of nitrogen. The resulting pale yellow residue was washed with hexane (5 mL) and dried under vacuum to give 1-Hf (0.1262 g, ~83% yield) as a cream powder.

## Synthesis of 2-Zr and 2-Hf

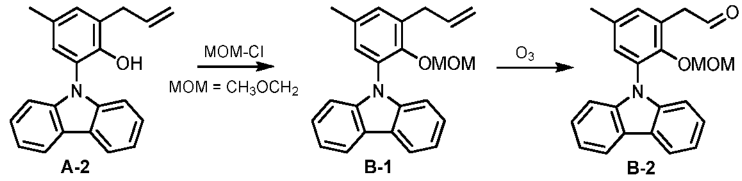


A-2 B-1 B-2

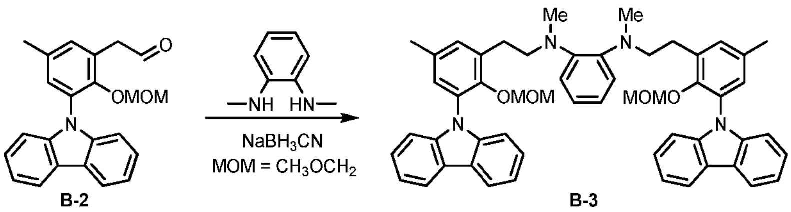


B-2 B-3

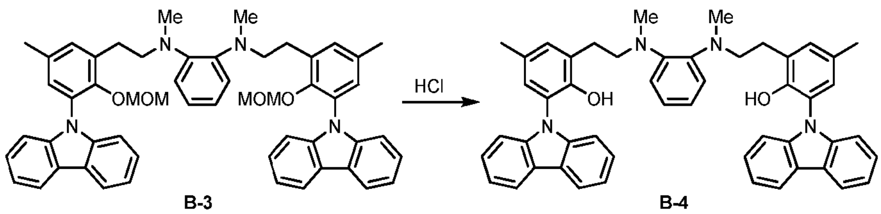


B-3 B-4

**9-(3-Allyl-2-(methoxymethoxy)-5-methylphenyl)-9*H*-carbazole (B-1).** Compound A-2 (45.4 g, 145 mmol, 1 equiv), chloromethyl methyl ether (MOM-Cl, 22 mL, 290 mmol, 2 equiv) and N,N-diisopropylethylamine (50.6 mL, 290 mmol, 2 equiv) were dissolved in dichloromethane (450 mL) and refluxed for 6.5 hours. The solution was diluted with water (400 mL) and the layers were separated. The aqueous layer was extracted with dichloromethane (100 mL). The combined organic layers were concentrated under reduced pressure. The residue was partially purified over silica gel (1 kg), eluting with a gradient of 10 to 50% toluene in heptanes. The mixed fractions from the first column were purified on an AnaLogix column (330 g), eluting with a gradient of 10 to 50% toluene in heptanes. The clean material from each column was combined to give compound B-1 (41.7 g, 73% yield) as a yellow oil.

**2-(3-(9*H*-Carbazol-9-yl)-2-(methoxymethoxy)-5-methylphenyl)acetaldehyde (B-2).** Compound B-1 (30.7 g, 85.9 mmol, 1 equiv) was dissolved in dichloro-methane (600 mL) and methanol (700 mL) and cooled to -78°C. Ozone was bubbled through the solution for 2.5 hours until a yellow color persisted. The solution was sparged with air for an additional 10 minutes. The solution was quenched with dimethyl sulfide (12 mL) and stirred while warming to room temperature for 5 hours. The solution was concentrated under reduced pressure. The residue was purified over silica gel (300 g), eluting with a gradient of 0 to 60% ethyl acetate in heptanes, to give compound B-2 (20.5 g, 66% yield) as a yellow oil.

***N,N'*-Bis(3-(9*H*-carbazol-9-yl)-2-(methoxymethoxy)-5-methylphenethyl)-*N,N'*-dimethylbenzene-1,2-diamine (B-3):** Sodium cyanoborohydride (1.8 g, 29.2 mmol, 4 equiv) and acetic acid (3.6 mL, 57 mmol, 7.8 equiv) were added to a solution of compound B-2 (6.3 g, 17.6 mmol, 2.4 equiv) and *N,N'*-dimethylbenzene-1,2-diamine (1.0 g, 7.3 mmol, 1 equiv) in methanol (250 mL). The solution was stirred at room temperature overnight. The solution was filtered and the filtrate was partially concentrated under reduced pressure and filtered again. The solids from each filtration were combined, dissolved in dichloromethane and dry loaded onto Celite (10 g). The dry loaded solid was then partially purified on an AnaLogix column (120 g), eluting with a gradient of 0 to 25% ethyl acetate in heptanes. The mixed fractions from the first column were combined and dry loaded onto Celite (5 g) and partially purified on an AnaLogix column (80 g), eluting with a gradient of 0 to 25% ethyl acetate in heptanes. The mixed fractions from the second column were combined and dry loaded onto Celite (5 g) and partially purified on an AnaLogix column (80 g), eluting with a gradient of 0 to 25% ethyl acetate in heptanes. The clean material from each column was combined to give compound B-3 (2.5 g, 42% yield) as a white solid.

**6,6'-((1,2-Phenylenebis(methylazanediyl))bis(ethane-2,1-diyl))bis(2-(9*H*-carbazol-9-yl)-4-methylphenol) (B-4).** Compound B-4 (2.4 g, 3.3 mmol, 1 equiv) was dissolved in a solution of 2.5% v/v concentrated hydrochloric acid in methanol (102.5 mL) and stirred at 45°C for 5 hours. Solid sodium bicarbonate (6.5 g) was added to neutralize the solution to pH 8. The solution was concentrated under reduced pressure. The residue was partitioned between ethyl

acetate (200 mL) and water (200 mL). The organic layer was dried over sodium sulfate and concentrated under reduced pressure. The residue was dry loaded onto Celite (5 g) and purified on an AnaLogix column (25 g), eluting with a gradient of 0 to 25% ethyl acetate in heptanes, to give compound B-4 (1.5 g, 71% yield) as a white solid.

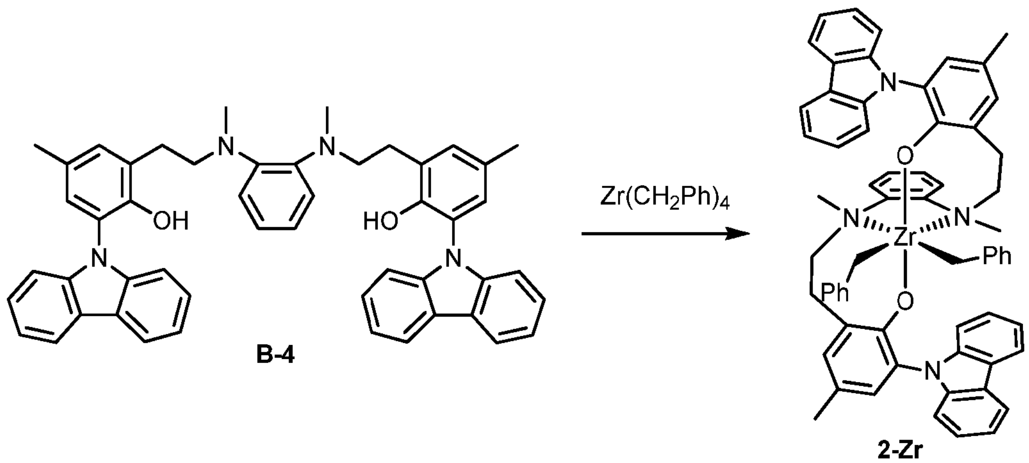

**B-4**

**2-Zr**

**6,6'-((1,2-phenylenebis(methylazanediyl))bis(ethane-2,1-diyl))bis(2-(9*H*-carbazol-9-yl)-4-methylphenoxide) zirconium dibenzyl (2-Zr).** In the drybox, $Zr(CH_2Ph)_4$ (0.0606 g, 0.133 mmol, 1 equiv) was dissolved in toluene (3 mL), and the resulting orange solution was added to a solution of compound B-4 (0.0998 g, 0.136 mmol, 1 equiv) in toluene (2 mL). The mixture was stirred for 2.5 hours at room temperature then concentrated under a stream of nitrogen. The resulting yellow-orange residue was stirred in hexane (5 mL), collected in a funnel with a glass frit and dried under vacuum to give 2-Zr (0.0983 g, ~73% yield) as a yellow powder.

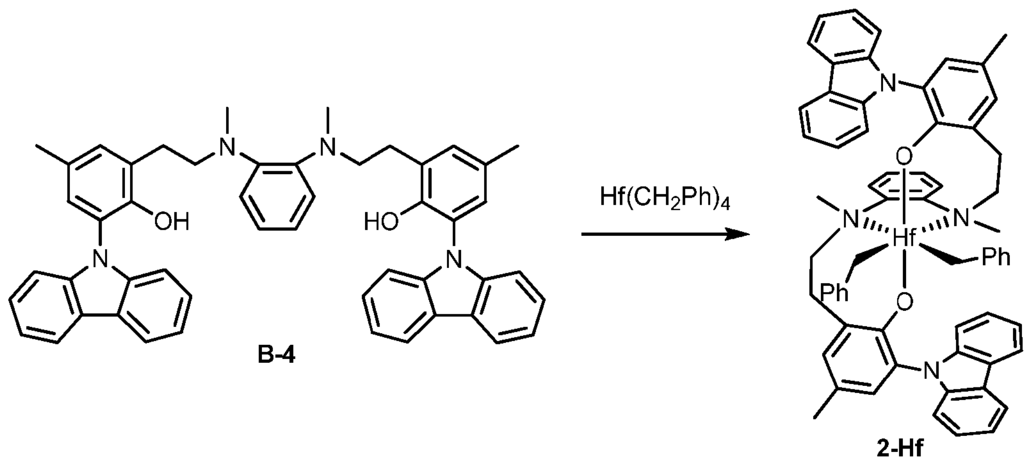

**B-4**

**2-Hf**

**6,6'-((1,2-phenylenebis(methylazanediyl))bis(ethane-2,1-diyl))bis(2-(9*H*-carbazol-9-yl)-4-methylphenoxide) hafnium dibenzyl (2-Hf).** In the drybox, $Hf(CH_2Ph)_4$ (0.0726 g, 0.134 mmol, 1 equiv) was dissolved in toluene (3 mL), and the resulting pale yellow solution was added to a solution of compound B-4 (0.0992 g, 0.135 mmol, 1 equiv) in toluene (2 mL). The mixture was stirred for 2.5 hours at room temperature then concentrated under a stream of nitrogen. The resulting pale yellow residue was washed with hexane (5 mL) and dried under vacuum to give 2-Hf (0.1180 g, ~81% yield) as a cream powder.

<u>**Synthesis of 3-Zr and 3-Hf**</u>

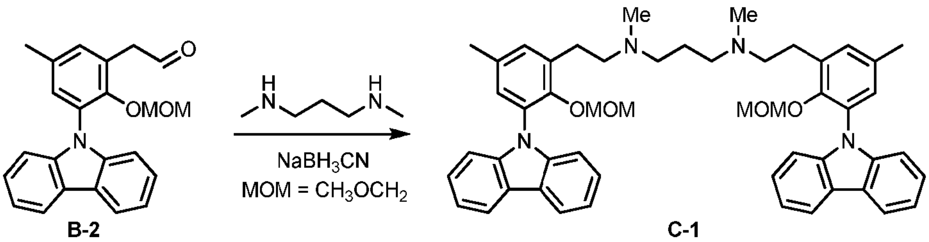


B-2 C-1

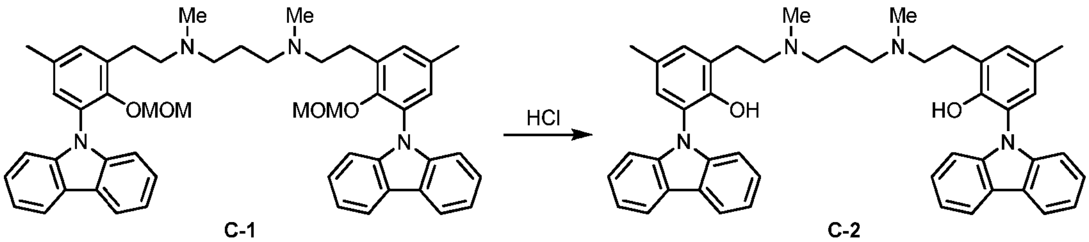


C-1 C-2

***N,N'*-bis(3-(9*H*-carbazol-9-yl)-2-(methoxymethoxy)-5-methylphenethyl)-*N,N'*-dimethylpropane-1,3-diamine (C-1).** Sodium cyanoborohydride (0.556 g, 8.85 mmol, 4 equiv) and acetic acid (10 drops) were added to a solution of compound B-2 (1.904 g, 5.30 mmol, 2.4 equiv) and *N,N'*-dimethylpropane-1,3-diamine (0.226 g, 2.21 mmol, 1 equiv) in methanol (25 mL). The solution was stirred at room temperature overnight then filtered and concentrated under reduced pressure. The resulting residue was purified on a Biotage SNAP Ultra column (50 g), eluting with a gradient of 0 to 20% ethyl acetate in hexane, to give compound C-1 as an off-white solid. The compound was carried over to the next step as isolated.

**6,6'-(2,2'-(propane-1,3-diylbis(methylazanediyl))bis(ethane-2,1-diyl))bis(2-(9*H*-carbazol-9-yl)-4-methylphenol) (C-2).** Compound C-1 (from previous reaction) was dissolved in 30 mL of methanol and a solution of 2.5% v/v concentrated hydrochloric acid in methanol (10.3 mL) and stirred at 45 °C for 5 hours. Solid sodium bicarbonate was added to neutralize the solution to pH 8. The solution was concentrated under reduced pressure. The residue was partitioned between ethyl acetate (50 mL) and water (50 mL). Organic layer washed with water (2 x 50 mL) and brine (1 x 50 mL). The organic layer was separated, dried over sodium sulfate, and concentrated under reduced pressure. The resulting residue was purified on a Biotage SNAP Ultra column (50 g), eluting with a gradient of 0 to 20% ethyl acetate in hexane, to give compound C-2 (0.954 g, 62 % overall yield) as a white solid.

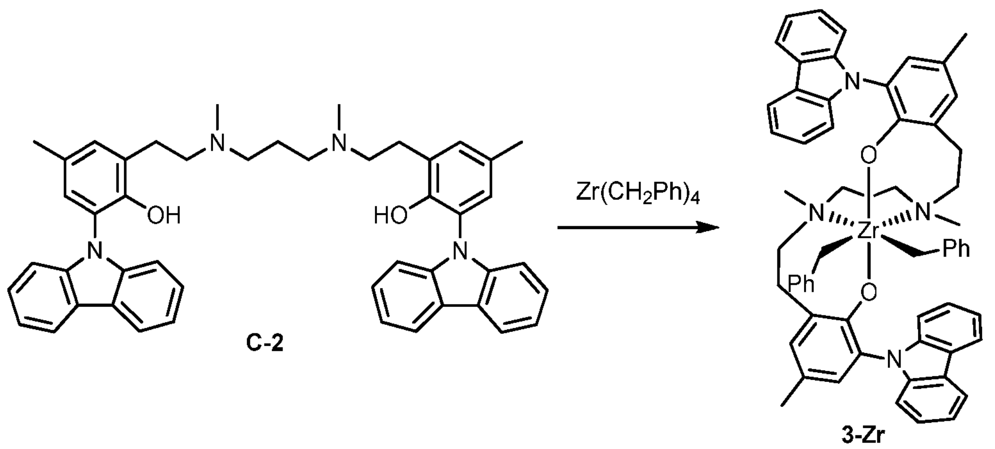


C-2 3-Zr

**6,6'-(2,2'-(propane-1,3-diylbis(methylazanediyl))bis(ethane-2,1-diyl))bis(2-(9*H*-carbazol-9-yl)-4-methylphenoxide) zirconium dibenzyl (3-Zr).** In the drybox, Zr(CH$_2$Ph)$_4$ (0.099 g, 0.217 mmol, 1 equiv) was dissolved in toluene (3 mL), and the resulting orange solution was added to a solution of compound C-2 (0.152 g, 0.217 mmol, 1 equiv) in toluene (2 mL). The mixture was stirred for 1 hour at room temperature then concentrated under a stream of nitrogen. The resulting yellow-orange residue was stirred in hexane (10 mL), collected in a funnel with a glass frit and dried under vacuum to give 3-Zr (0.169 g, 80 % yield) as a yellow powder.

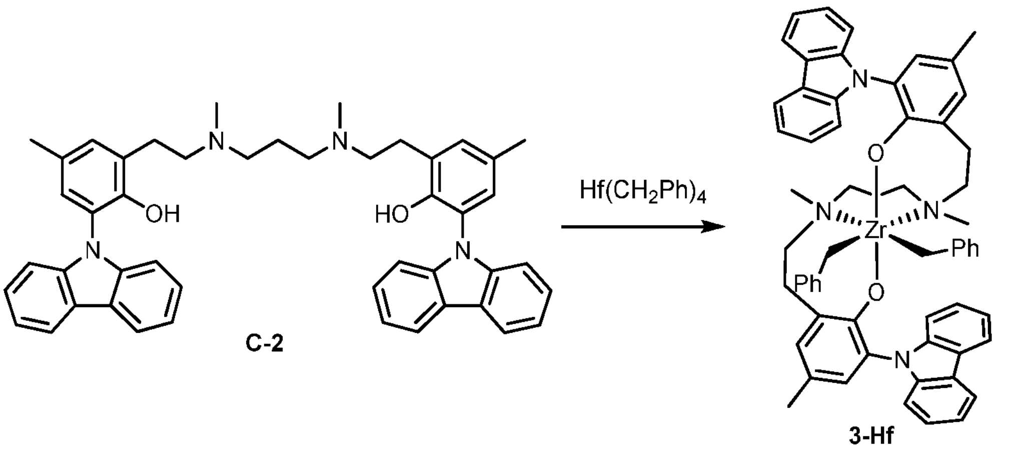


C-2

3-Hf

**6,6'-(2,2'-(propane-1,3-diylbis(methylazanediyl))bis(ethane-2,1-diyl))bis(2-(9*H*-carbazol-9-yl)-4-methylphenoxide) hafnium dibenzyl (3-Hf).** In the drybox, $Hf(CH_2Ph)_4$ (0.136 g, 0.250 mmol, 1 equiv) was dissolved in toluene (3 mL), and the resulting pale yellow solution was added to a solution of compound C-2 (0.175 g, 0.250 mmol, 1 equiv) in toluene (2 mL). The mixture was stirred for 1 hour at room temperature then concentrated under a stream of nitrogen. The resulting off-white residue was stirred in hexane (10 mL), collected in a funnel with a glass frit, washed with hexane, and dried under vacuum to give 3-Hf (0.213 g, 80 % yield) as a white powder.

Likewise, the following can also be made:

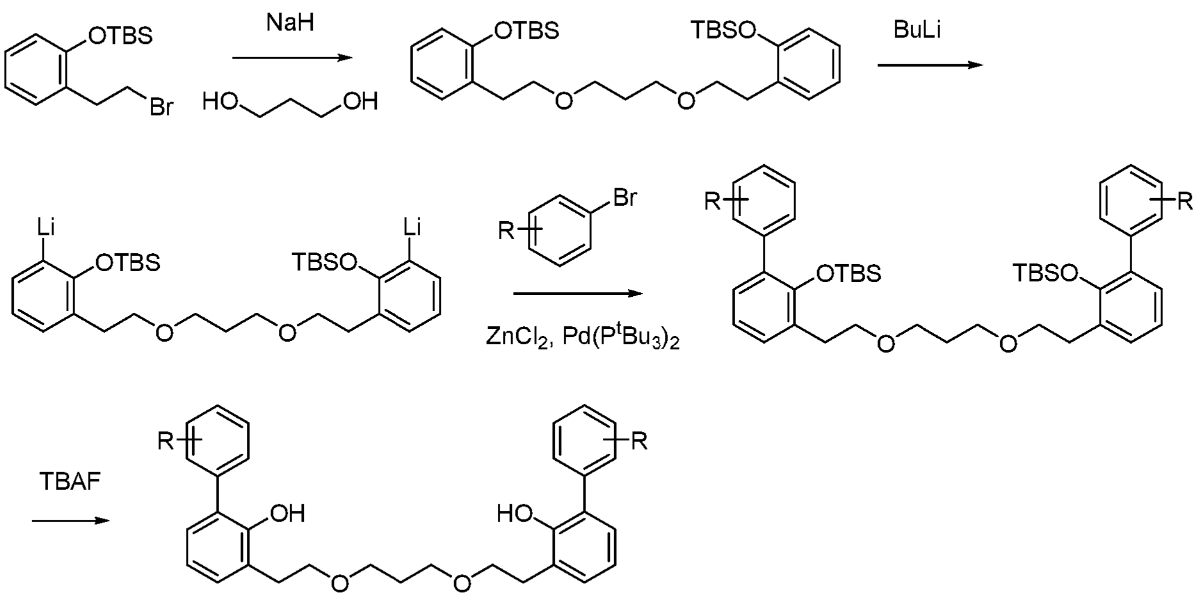


## Preparation of Supported Catalysts

**Preparation of Silica Support (sMAO).** MAO (71.4 g, 30 wt% toluene solution, 351.1 mmol of Al) was slowly added to a slurry of silica (D948, 40.7 g, calcined at 600°C) in toluene (200 mL). The slurry was heated to 80°C, stirred for 1 hour then filtered and washed with toluene (3 x 70 mL) and pentane (70 mL). The silica support was dried under vacuum overnight to give a free flowing white solid (60.7 g).

**Preparation of Supported Catalyst.** In the drybox, a slurry of sMAO (0.500 g) in toluene (30 mL) was prepared in a Cel-Stir reaction vessel. The slurry was charged with a toluene (3 mL) solution of the metal complex (0.020 mmol), and the reaction was stirred at room temperature for 1 hour. The slurry was filtered through a funnel with a glass frit (10 micron), washed with toluene (5 mL) and pentane (10 mL), and dried under vacuum for 1 hour to afford a free flowing yellow or white solid (~ 0.5 g; catalyst loading = 0.040 mmol metal complex / g sMAO). In the following polymerization examples, supported catalysts are indicated by "_s" following the catalyst name. For example, catalyst 1-Zr supported on silica is labeled as 1-Zr_s.

## Polymerization Examples

**General polymerization procedures for parallel pressure reactor.** Solvents, polymerization-grade toluene, and isohexane were supplied by ExxonMobil Chemical Company and purified by passing through a series of columns: two 500 cc Oxyclear cylinders in series from Labclear (Oakland, Calif.), followed by two 500 cc columns in series packed with dried 3 Å mole sieves (8-12 mesh; Aldrich Chemical Company), and two 500 cc columns in series packed with dried 5 Å mole sieves (8-12 mesh; Aldrich Chemical Company).

1-octene (C8) and 1-hexene (C6) (98%, Aldrich Chemical Company) were dried by stirring over NaK overnight followed by filtration through basic alumina (Aldrich Chemical Company, Brockman Basic 1).

Polymerization-grade ethylene (C2) was used and further purified by passing the gas through a series of columns: 500 cc Oxyclear cylinder from Labclear (Oakland, CA) followed yb a 500 cc column packed with dried 3Å mole sieves (8-12 mesh; Aldrich Chemical Company) and a 500 cc column packed with dried 5Å mole sieves (8-12 mesh; Aldrich Chemical Company).

Polymerization grade propylene (C3) was used and further purified by passing it through a series of columns: 2250 cc Oxiclear cylinder from Labclear followed by a 2250 cc column packed with 3 Å mole sieves (8-12 mesh; Aldrich Chemical Company), then two 500 cc columns in series packed with 5 Å mole sieves (8-12 mesh; Aldrich Chemical Company), then a 500 cc column packed with Selexsorb CD (BASF), and finally a 500 cc column packed with Selexsorb COS (BASF).

Solutions of the metal complexes and activators were prepared in a drybox using toluene (ExxonMobil Chemical Company; anhydrous, stored under nitrogen; 98%). Concentrations were typically 0.2 mmol/L for the metal complexes and N,N-dimethyl anilinium tetrakis-pentafluorophenyl borate (Activator-1) and 0.5 % w/w for methyl alumoxane (MAO).

Slurries of supported catalysts in toluene were prepared in the drybox using 45 mg of the supported catalyst and 15 mL of toluene. The resulting mixture was vortexed for uniform distribution of particles prior to injection.

For polymerization experiments with supported catalysts or Activator-1 as activator, tri-n-octylaluminum (TNOAL, neat, AkzoNobel) was used as a scavenger. Concentration of the TNOAL solution in toluene ranged from 0.5 to 2.0 mmol/L.

Polymerizations were carried out in a parallel, pressure reactor, as generally described in US 6,306,658; US 6,455,316; US 6,489,168; WO 00/09255; and Murphy et al., J. Am. Chem. Soc., 2003, 125, pp. 4306-4317. The experiments were conducted in an inert atmosphere ($N_2$) drybox using autoclaves equipped with an external heater for temperature control, glass inserts (internal volume of reactor = 23.5 mL for C2 and C2/C8; 22.5 mL for C3 runs), septum inlets, regulated supply of nitrogen, ethylene and propylene, and equipped with disposable PEEK mechanical stirrers (800 RPM). The autoclaves were prepared by purging with dry nitrogen at 110°C or 115°C for 5 hours and then at 25°C for 5 hours. Although the specific quantities, temperatures, solvents, reactants, reactant ratios, pressures, and other variables are frequently changed from one polymerization run to the next, the following describes a typical polymerization performed in a parallel, pressure reactor.

Catalyst systems dissolved in solution were used in the polymerization examples below, unless specified otherwise.

**Ethylene Homopolymerization (HDPE) and Ethylene-Octene Copolymerization (EO).** A pre-weighed glass vial insert and disposable stirring paddle were fitted to each reaction vessel of the reactor, which contains 48 individual reaction vessels. The reactor was then closed and purged with ethylene. Each vessel was charged with enough solvent (typically isohexane) to bring the total reaction volume, including the subsequent additions, to the desired volume, typically 5 mL. 1-octene, if required, was injected into the reaction vessel and the reactor was heated to the set temperature and pressurized to the predetermined pressure of ethylene, while stirring at 800 rpm. The aluminum and/or zinc compound in toluene was then injected as scavenger and/or chain transfer agent followed by addition of the activator solution (typically 1.0-1.2 molar equivalents of *N,N*-dimethyl anilinium tetrakis-pentafluorophenyl borate - Activator-1).

The catalyst solution (typically 0.020-0.080 umol of metal complex) was injected into the reaction vessel and the polymerization was allowed to proceed until a pre-determined amount of ethylene (quench value typically 1.4 bar (20 psi)) had been used up by the reaction. Alternatively, the reaction may be allowed to proceed for a set amount of time (maximum reaction time typically 30 minutes). Ethylene was added continuously (through the use of computer controlled solenoid valves) to the autoclaves during polymerization to maintain reactor gauge pressure (+/-0.14 bar (2 psig)) and the reactor temperature was monitored and typically maintained within +/-1°C. The reaction was quenched by pressurizing the vessel with compressed air. After the reactor was vented and cooled, the glass vial insert containing the polymer product and solvent was removed from the pressure cell and the inert atmosphere glove box, and the volatile components were removed using a Genevac HT-12 centrifuge and Genevac VC3000D vacuum evaporator operating at elevated temperature and reduced pressure. The vial was then weighed to determine the yield of the polymer product. The resultant polymer was analyzed by Rapid GPC (see below) to determine the molecular weight, by FT-IR (see below) to determine percent octene incorporation, and by DSC (see below) to determine melting point (Tm).

For polymerizations using MAO as activator (typically 100 to 1000 molar equivalents), the MAO solution was

injected into the reaction vessel after the addition of 1-octene and prior to heating the vessel to the set temperature and pressurizing with ethylene. No additional aluminum reagent was used as scavenger during these runs.

Equivalence is determined based on the mole equivalents relative to the moles of the transition metal in the catalyst complex.

**Ethylene-Propylene Copolymerization (EP).** The reactor was prepared as described above and purged with propylene. Isohexane was then injected into each vessel at room temperature followed by a predetermined amount of propylene gas. The reactor was heated to the set temperature and pressurized with the required amount of ethylene while stirring at 800 rpm. The scavenger, activator (typically Activator-1) and catalyst solutions were injected sequentially to each vessel and the polymerization was allowed to proceed as described previously.

**Propylene Homopolymerization (PP).** The reactor was prepared as described above and purged with propylene. Isohexane was then injected into each vessel at room temperature followed by a predetermined amount of propylene gas. The reactor was heated to the set temperature while stirring at 800 rpm, and the scavenger, activator (typically Activator-1) and catalyst solutions were injected sequentially to each vessel. The polymerization was allowed to proceed as described previously.

For propylene homopolymerization in the presence of hydrogen (PP/$H_2$), the reactor was prepared as described above and purged with 25% v/v $H_2/N_2$ gas. With an atmosphere of $H_2/N_2$ gas in the reaction vessel, isohexane, the scavenger solution and a predetermined amount of propylene gas were injected sequentially at room temperature. The reactor was then heated to the set temperature followed by sequential injection of the activator and catalyst solutions. The polymerization was allowed to proceed as described previously.

For polymerizations using MAO as activator, the MAO solution was injected into the vessel after the addition of isohexane. No additional aluminum reagent was used as scavenger during these runs.

**Polymerizations using Supported Catalysts.** For ethylene-hexene copolymerization (EH), the reactor was prepared as described above and purged with ethylene. Isohexane, 1-hexene, and the scavenger solution were added sequentially to the reaction vessel via syringe at room temperature and atmospheric pressure. The reactor was then heated to the process temperature (85°C) and charged with ethylene to the pressure setpoint (130 psig = 896 kPa) while stirring at 800 rpm. The supported catalyst slurry was injected into the vessel and the polymerization was allowed to proceed as described in the previous section. To test for catalyst response to hydrogen, the EH experiments were also carried out using 300 ppm $H_2$/ethylene mixed gas.

For EP, the reactor was prepared as described above and purged with propylene. Isohexane, the scavenger solution and a predetermined amount of propylene gas were added sequentially to the reaction vessels at room temperature. The reactor was then heated to the set temperature and pressurized with the required amount of ethylene while stirring at 800 rpm, followed by injection of the catalyst slurry. The polymerization was allowed to proceed as described previously.

Propylene homopolymerizations were set up similar to EP copolymerizations less the addition of ethylene.

For propylene homopolymerization in the presence of hydrogen (PP/$H_2$), the reactor was prepared as described above and purged with 25% v/v $H_2/N_2$ gas. With an atmosphere of $H_2/N_2$ gas in the reaction vessel, isohexane, the scavenger solution and a predetermined amount of propylene gas were injected sequentially at room temperature. The reactor was then heated to the set temperature followed by injection of the catalyst slurry. The polymerization was allowed to proceed as described previously.

**Polymer Characterization.** Polymer sample solutions were prepared by dissolving polymer in 1,2,4-trichlorobenzene (TCB, 99+% purity from Sigma-Aldrich) containing 2,6-di-tert-butyl-4-methylphenol (BHT, 99% from Aldrich) at 165°C in a shaker oven for approximately 3 hours. The typical concentration of polymer in solution was between 0.1 to 0.9 mg/mL with a BHT concentration of 1.25 mg BHT/mL of TCB.

To determine various molecular weight related values by GPC, high temperature size exclusion chromatography was performed using an automated "Rapid GPC" system as generally described in US 6,491,816; US 6,491,823; US 6,475,391; US 6,461,515; US 6,436,292; US 6,406,632; US 6,175,409; US 6,454,947; US 6,260,407; and US 6,294,388. This apparatus has a series of three 30 cm x 7.5 mm linear columns, each containing PLgel 10 μm, Mix B. The GPC system was calibrated using polystyrene standards ranging from 580 - 3,390,000 g/mol. The system was operated at an eluent flow rate of 2.0 mL/minutes and an oven temperature of 165°C. 1,2,4-trichlorobenzene was used as the eluent. The polymer samples were dissolved in 1,2,4-trichlorobenzene at a concentration of 0.28 mg/mL and 400 uL of a polymer solution was injected into the system. The concentration of the polymer in the eluent was monitored using an evaporative light scattering detector. The molecular weights presented are relative to linear polystyrene standards and are uncorrected, unless indicated otherwise.

Differential Scanning Calorimetry (DSC) measurements were performed on a TA-Q100 instrument to determine the melting point (Tm) of the polymers. Samples were pre-annealed at 220°C for 15 minutes and then allowed to cool to room temperature overnight. The samples were then heated to 220°C at a rate of 100°C/min and then cooled at a rate of 50°C/min. Melting points were collected during the heating period.

The weight percent of ethylene incorporated in polymers was determined by rapid FT-IR spectroscopy on a

Bruker Equinox 55+ IR in reflection mode. Samples were prepared in a thin film format by evaporative deposition techniques. FT-IR methods were calibrated using a set of samples with a range of known wt% ethylene content. For ethylene-1-octene copolymers, the wt% octene in the copolymer was determined via measurement of the methyl deformation band at ~1375 $cm^{-1}$. The peak height of this band was normalized by the combination and overtone band at ~4321 $cm^{-1}$, which corrects for path length differences.

For ethylene-propylene copolymers, the wt% ethylene is determined via measurement of the methylene rocking band (~770 $cm^{-1}$ to 700$cm^{-1}$). The peak area of this band is normalized by sum of the band areas of the combination and overtone bands in the 4500 $cm^{-1}$ to 4000 $cm^{-1}$ range. For samples with composition outside the calibration range, the wt% ethylene was determined by $^1H$ NMR spectroscopy or estimated from the polymer Tm.

$^1H$ NMR data were collected at 120°C in a 5 mm probe using a spectrometer with a $^1H$ frequency of 500 MHz. Data was recorded using a maximum pulse width of 45°, 5 seconds between pulses and signal averaging 120 transients. Spectral signals were integrated. Samples were dissolved in deuterated 1,1,2,2,-tetrachloroethane at concentrations of 1-2 wt% prior to being inserted into the spectrometer magnet. Prior to data analysis, spectra were referenced by setting the residual hydrogen-containing solvent resonance to 5.98 ppm. Vinylenes were measured as the number of vinylenes per 1000 carbon atoms using the resonances between 5.5-5.31 ppm. Trisubstituted end-groups ("trisubs") were measured as the number of trisubstituted groups per 1000 carbon atoms using the resonances between 5.3-4.85 ppm, by difference from vinyls. Vinyl end-groups were measured as the number of vinyls per 1000 carbon atoms using the resonances between 5.9-5.65 and between 5.3-4.85 ppm. Vinylidene end-groups were measured as the number of vinylidenes per 1000 carbon atoms using the resonances between 4.85-4.65 ppm.

**Table 1.** Reaction conditions for ethylene homopolymerization (HDPE) and ethylene-octene copolymerization (EO) using Activator-1 or MAO.

| | |
|---|---|
| Catalyst loading | 0.020-0.080 μmol |
| Activator- 1 | 1.1 equiv |
| MAO | 500 equiv |
| Temperature | 100°C |
| Pressure Setpoint | 9.31 bar (135 psi) |
| 1-Octene | 100 μL |
| Total Volume | 5 mL |
| Solvent | Isohexane |
| Aluminum compound (scavenger) | 0.5 or 1 μmol tri-*n*-octyl aluminum (TNOAL) |
| Quench Value | 1.4 bar (20 psi) |
| Maximum Reaction Time | 30 in |

**Table 2.** Catalyst activity and polymer properties for ethylene homopolymerization (HDPE) and ethylene-octene copolymerization (EO) using Activator-1 or MAO.

| Entry | Run | Catalyst, umol | | Activator | TNOAL umol | Polymer | time (s) | yield (g) | activity (g/ mmol-hr) | Mw (kg/mol) | Mn (kg/mol) | Mw/Mn | wt% octene | Tm (°C) |
|---|---|---|---|---|---|---|---|---|---|---|---|---|---|---|
| 1 | 1 | 1-Zr | 0.02 | MAO | - | EO | 29.1 | 0.079 | 488,660 | 265 | 162 | 1.64 | 6.7 | 116.2 |
| 2 | 2 | 1-Zr | 0.02 | MAO | - | EO | 26.5 | 0.075 | 509,434 | 268 | 155 | 1.73 | 6.2 | 117.4 |
| 3 | 1 | 1-Hf | 0.02 | MAO | - | EO | 125.5 | 0.068 | 97,530 | 789 | 471 | 1.68 | 7.4 | 113.6 |
| 4 | 2 | 1-Hf | 0.02 | MAO | - | EO | 113.8 | 0.064 | 101,230 | 1,101 | 642 | 1.72 | 6.3 | 113.7 |
| 5 | 1 | 2-Zr | 0.02 | MAO | - | EO | 24.7 | 0.078 | 568,421 | 65 | 41 | 1.60 | 3.5 | 129.4 |
| 6 | 2 | 2-Zr | 0.02 | MAO | - | EO | 24.0 | 0.068 | 510,000 | 63 | 39 | 1.60 | 3.4 | 128.5 |
| 7 | 1 | 2-Hf | 0.02 | MAO | - | EO | 227.8 | 0.039 | 30,817 | 259 | 142 | 1.83 | 4.8 | 127.5 |
| 8 | 2 | 2-Hf | 0.02 | MAO | - | EO | 247.1 | 0.034 | 24,767 | 307 | 195 | 1.57 | 2.7 | 128.1 |
| 9 | 1 | 3-Zr | 0.02 | MAO | - | EO | 1800.7 | 0.008 | 800 | - | - | - | - | - |
| 10 | 2 | 3-Zr | 0.08 | MAO | - | EO | 1800.9 | 0.030 | 750 | 211 | 100 | 2.11 | 9.1 | 112.5 |
| 11 | 1 | 3-Hf | 0.02 | MAO | - | EO | 1801.1 | 0.005 | 500 | - | - | - | - | - |
| 12 | 2 | 3-Hf | 0.08 | MAO | - | EO | 1800.4 | 0.004 | 100 | - | - | - | - | - |
| 13 | 1 | 1-Zr | 0.02 | Activator-1 | 1 | EO | 30.1 | 0.058 | 346,844 | 241 | 148 | 1.62 | 4.1 | 119.7 |
| 14 | 2 | 1-Zr | 0.02 | Activator-1 | 1 | EO | 30.9 | 0.068 | 396,117 | 235 | 130 | 1.81 | 5.0 | 119.7 |
| 15 | 1 | 1-Hf | 0.02 | Activator-1 | 1 | EO | 388.3 | 0.033 | 15,297 | 855 | 479 | 1.79 | 4.9 | 117.4 |
| 16 | 2 | 1-Hf | 0.02 | Activator-1 | 1 | EO | 236.8 | 0.041 | 31,166 | 890 | 572 | 1.55 | 3.9 | 120.5 |
| 17 | 1 | 2-Zr | 0.02 | Activator-1 | 0.5 | EO | 34.1 | 0.060 | 316,716 | 85 | 53 | 1.61 | 3.2 | 129.4 |
| 18 | 2 | 2-Zr | 0.02 | Activator-1 | 0.5 | EO | 31.7 | 0.060 | 340,694 | 82 | 47 | 1.74 | 3.4 | 129.7 |
| 19 | 1 | 2-Hf | 0.02 | Activator-1 | 0.5 | EO | 591.8 | 0.030 | 9,125 | 442 | 270 | 1.64 | 2.6 | 127.9 |
| 20 | 2 | 2-Hf | 0.02 | Activator-1 | 0.5 | EO | 688.7 | 0.030 | 7,841 | 452 | 292 | 1.55 | 3.0 | 129.3 |
| 21 | 1 | 3-Zr | 0.02 | Activator-1 | 0.5 | EO | 1800.7 | 0.001 | 100 | - | - | - | - | - |
| 22 | 2 | 3-Zr | 0.08 | Activator-1 | 0.5 | EO | 1800.9 | 0.006 | 150 | - | - | - | - | - |
| 23 | 1 | 3-Hf | 0.02 | Activator-1 | 0.5 | EO | 1800.2 | 0.000 | 0 | - | - | - | - | - |
| 24 | 2 | 3-Hf | 0.08 | Activator-1 | 0.5 | EO | 1086.1 | 0.044 | 1,823 | 328 | 204 | 1.61 | 5.7 | 114.9 |
| 25 | 1 | 1-Zr | 0.02 | Activator-1 | 1 | HDPE | 31.4 | 0.056 | 321,019 | 243 | 151 | 1.61 | NA | 135.7 |
| 26 | 2 | 1-Zr | 0.02 | Activator-1 | 1 | HDPE | 53.6 | 0.054 | 181,343 | 253 | 155 | 1.64 | NA | 135.8 |

(continued)

| Entry | Run | Catalyst, umol | | Activator | TNOAL umol | Polymer | time (s) | yield (g) | activity (g/ mmol-hr) | Mw (kg/mol) | Mn (kg/mol) | Mw/Mn | wt% octene | Tm (°C) |
|---|---|---|---|---|---|---|---|---|---|---|---|---|---|---|
| 27 | 1 | 1-Hf | 0.02 | Activator-1 | 1 | HDPE | 459.9 | 0.037 | 14,481 | 965 | 575 | 1.68 | NA | 135.7 |
| 28 | 1 | 2-Zr | 0.02 | Activator-1 | 0.5 | HDPE | 1801.0 | 0.000 | 0 | - | - | - | NA | - |
| 29 | 2 | 2-Zr | 0.08 | Activator-1 | 0.5 | HDPE | 1801.0 | 0.003 | 75 | - | - | - | NA | - |
| 30 | 1 | 2-Hf | 0.02 | Activator-1 | 0.5 | HDPE | 1800.2 | 0.000 | 0 | - | - | - | NA | - |
| 31 | 2 | 2-Hf | 0.08 | Activator-1 | 0.5 | HDPE | 1801.1 | 0.010 | 250 | - | - | - | NA | - |
| 32 | 1 | 3-Zr | 0.02 | Activator-1 | 0.5 | HDPE | 35.3 | 0.065 | 331,445 | 91 | 55 | 1.66 | NA | 135.5 |
| 33 | 2 | 3-Zr | 0.02 | Activator-1 | 0.5 | HDPE | 31.3 | 0.058 | 333,546 | 92 | 58 | 1.59 | NA | 135.5 |
| 34 | 1 | 3-Hf | 0.02 | Activator-1 | 0.5 | HDPE | 483.3 | 0.035 | 13,035 | 535 | 335 | 1.60 | NA | 137.8 |
| 35 | 2 | 3-Hf | 0.02 | Activator-1 | 0.5 | HDPE | 571.7 | 0.032 | 10,075 | 510 | 285 | 1.79 | NA | 137.4 |

**Table 3.** Reaction conditions for propylene homopolymerization (PP) using Activator-1 or MAO.

| | |
|---|---|
| Catalyst loading | 0.040 μmol |
| Activator- 1 | 1.1 equiv |
| MAO | 500 equiv |
| Temperature | 70°C |
| Propylene Pressure | 8.27 bar (120 psi) |
| Total Volume | 4 mL |
| Solvent | Isohexane |
| Aluminum compound (scavenger) | 0.3 μmol tri-*n*-octyl aluminum (TNOAL) |
| Quench Value | 0.21 bar (3 psi) |
| Maximum Reaction Time | 20 in |

**Table 4.** Catalyst activity and polymer properties for propylene homopolymerization (PP) using Activator-1 or MAO.

| Entry | Run | Catalyst | Activator | TNOAL umol | time (s) | yield (g) | activity (g/mmol -hr) | Mw (kg/mol) | Mn (kg/mol) | Mw/Mn | Tm (°C) |
|---|---|---|---|---|---|---|---|---|---|---|---|
| 1 | 1 | 1-Zr | Activator-1 | 0.3 | 101.9 | 0.053 | 46,811 | 259 | 153 | 1.69 | 160.0 |
| 2 | 2 | 1-Zr | Activator-1 | 0.3 | 97.3 | 0.050 | 46,249 | 253 | 156 | 1.62 | 158.8 |
| 3 | 1 | 1-Hf | Activator-1 | 0.3 | 1072.7 | 0.010 | 839 | 97 | 61 | 1.59 | 161.4 |
| 4 | 2 | 1-Hf | Activator-1 | 0.3 | 1020.6 | 0.011 | 970 | 103 | 65 | 1.58 | 160.5 |
| 5 | 1 | 2-Zr | Activator-1 | 0.3 | 1200.4 | 0.000 | 0 | - | - | - | - |
| 6 | 2 | 2-Zr | Activator-1 | 0.3 | 1200.6 | 0.000 | 0 | - | - | - | - |
| 7 | 1 | 2-Hf | Activator-1 | 0.3 | 1200.3 | 0.004 | 300 | - | - | - | - |
| 8 | 2 | 2-Hf | Activator-1 | 0.3 | 1201.0 | 0.004 | 300 | - | - | - | - |
| 9 | 1 | 1-Zr | MAO | - | 49.2 | 0.096 | 175,610 | 367 | 222 | 1.65 | 157.4 |
| 10 | 2 | 1-Zr | MAO | - | 50.4 | 0.096 | 171,429 | 371 | 232 | 1.60 | 157.9 |
| 11 | 1 | 1-Hf | MAO | - | 595.9 | 0.036 | 5,437 | 120 | 68 | 1.76 | 154.1 |
| 12 | 2 | 1-Hf | MAO | - | 506.4 | 0.037 | 6,576 | 161 | 91 | 1.77 | 155.1 |
| 13 | 1 | 2-Zr | MAO | - | 1200.7 | 0.002 | 150 | - | - | - | - |
| 14 | 2 | 2-Zr | MAO | - | 1200.2 | 0.003 | 225 | - | - | - | - |
| 15 | 1 | 2-Hf | MAO | - | 1200.3 | 0.009 | 675 | - | - | - | - |
| 16 | 2 | 2-Hf | MAO | - | 1201.2 | 0.010 | 749 | 19 | 12 | 1.60 | 101.7 |

**Table 5.** Reaction conditions for ethylene-propylene copolymerization (EP) using Activator-1.

| | |
|---|---|
| Catalyst loading | 0.015 μmol |
| Activator- 1 | 1.1 equiv |
| Temperature | 110°C |
| Ethylene Pressure | 8.62 bar (125 psi) |
| Propylene Pressure | 8.62 or 12.8 bar (125 or 185 psi) |
| Total Volume | 5.1 mL |
| Solvent | Isohexane |
| Aluminum compound (scavenger) | 0.5 μmol tri-*n*-octyl aluminum (TNOAL) |
| Quench Value | 0.28 bar (4 psi) |
| Maximum Reaction Time | 30 in |

**Table 6.** Catalyst activity and polymer properties for ethylene-propylene copolymerization (EP) using Activator-1.

| Entry | Run | Catalyst | TNOAL umol | C3 Pressure | C2 Pressure | time (s) | yield (g) | activity (g/ mmol-hr) | Mw (kg/mol) | Mn (kg/mol) | Mw/Mn | wt% propylene (est from Tm) | Tm (°C) |
|---|---|---|---|---|---|---|---|---|---|---|---|---|---|
| 1 | 1 | 1-Zr | 0.5 | 8.62 bar (125 psi) | 8.62 bar (125 psi) | 15.4 | 0.043 | 670,565 | 242 | 154 | 1.57 | 11 | 105.9 |
| 2 | 2 | 1-Zr | 0.5 | 8.62 bar (125 psi) | 8.62 bar (125 psi) | 18.0 | 0.037 | 493,608 | 283 | 167 | 1.70 | 11 | 105.4 |
| 3 | 3 | 1-Zr | 0.5 | 8.62 bar (125 psi) | 8.62 bar (125 | 14.2 | 0.042 | 709,859 | 243 | 146 | 1.67 | 11 | 107.2 |
| 4 | 1 | 1-Zr | 0.5 | 12.8 bar (185 psi) | 8.62 bar (125 psi) | 25.4 | 0.041 | 386,792 | 272 | 160 | 1.70 | 14 | 94.7 |
| 5 | 2 | 1-Zr | 0.5 | 12.8 bar (185 psi) | 8.62 bar (125 psi) | 37.7 | 0.025 | 159,025 | 271 | 161 | 1.68 | 15 | 93.6 |
| 6 | 3 | 1-Zr | 0.5 | 12.8 bar (185 psi) | 8.62 bar (125 psi) | 26.7 | 0.039 | 351,088 | 262 | 167 | 1.57 | 14 | 95.1 |
| 7 | 1 | 1-Hf | 0.5 | 8.62 bar (125 psi) | 8.62 bar (125 psi) | 112.4 | 0.016 | 34,158 | 585 | 353 | 1.66 | 12 | 101.7 |
| 8 | 2 | 1-Hf | 0.5 | 8.62 bar (125 psi) | 8.62 bar (125 psi) | 102.8 | 0.017 | 39,704 | 891 | 479 | 1.86 | 12 | 101.6 |
| 9 | 3 | 1-Hf | 0.5 | 8.62 bar (125 psi) | 8.62 bar (125 psi) | 280.6 | 0.013 | 11,120 | 710 | 447 | 1.59 | 16 | 88.1 |
| 10 | 1 | 1-Hf | 0.5 | 12.8 bar (185 psi) | 8.62 bar (125 psi) | 184.6 | 0.017 | 22,102 | 583 | 322 | 1.81 | 16 | 90.0 |
| 11 | 2 | 1-Hf | 0.5 | 12.8 bar (185 psi) | 8.62 bar (125 psi) | 148.9 | 0.018 | 29,015 | 498 | 282 | 1.77 | 15 | 91.4 |

**Table 7.** Reaction conditions for ethylene-hexene copolymerization (EH) using supported catalysts.

| | |
|---|---|
| Catalyst loading | 0.012 μmol of metal complex |
| Temperature | 85°C |
| Pressure Setpoint | 8.96 bar (130 psi) |
| 1-hexene | 30, 180 or 300 uL |
| Total Volume | 5 mL |
| Solvent | Isohexane |
| Aluminum compound (scavenger) | 4 μmol tri-*n*-octyl aluminum (TNOAL) |
| Quench Value | 3.8 bar (55 psi) |
| Maximum Reaction Time | 45 min |

**Table 8.** Catalyst activity and polymer properties for ethylene-hexene copolymerization (EH) using supported catalysts.

| Entry | Run | Catalyst | hexene uL | time (s) | yield (g) | activity (g/mmol-hr) | Mw (kg/mol) | Mn (kg/mol) | Mw/Mn | wt% C6 | Tm (°C) |
|---|---|---|---|---|---|---|---|---|---|---|---|
| 1 | 1 | 1-Zr_s | 30 | 2701 | 0.020 | 2222 | 451 | 121 | 3.7 | 1.6 | 130 |
| 2 | 2 | 1-Zr_s | 30 | 2700 | 0.025 | 2777 | 538 | 110 | 4.9 | 0.7 | 130 |
| 3 | 1 | 1-Hf_s | 30 | 2300 | 0.072 | 9392 | 2372 | 1220 | 1.9 | 1.8 | 127 |
| 4 | 2 | 1-Hf_s | 30 | 2701 | 0.080 | 8887 | 2303 | 960 | 2.4 | 1.2 | 126 |
| 5 | 1 | 1-Zr_s | 180 | 2352 | 0.059 | 7526 | 224 | 101 | 2.2 | 6.2 | 120 |
| 6 | 2 | 1-Zr_s | 180 | 2700 | 0.070 | 7777 | 465 | 126 | 3.7 | 6.4 | 119 |
| 7 | 1 | 1-Hf_s | 180 | 1086 | 0.097 | 26801 | 2176 | 1196 | 1.8 | 8.3 | 112 |
| 8 | 2 | 1-Hf_s | 180 | 1212 | 0.092 | 22782 | 2111 | 1116 | 1.9 | 7.7 | 113 |
| 9 | 1 | 1-Zr_s | 300 | 844 | 0.085 | 30228 | 237 | 120 | 2.0 | 8.3 | 112 |
| 10 | 2 | 1-Zr_s | 300 | 1798 | 0.092 | 15347 | 250 | 110 | 2.3 | 9.0 | 112 |
| 11 | 1 | 1-Hf_s | 300 | 791 | 0.097 | 36770 | 1788 | 995 | 1.8 | 11.5 | 101 |
| 12 | 2 | 1-Hf_s | 300 | 830 | 0.094 | 33980 | 1836 | 1045 | 1.8 | 11.8 | 101 |

**Table 9.** Reaction conditions for propylene homopolymerization (PP) using supported catalysts.

| | |
|---|---|
| Catalyst loading | 0.015 μmol of metal complex |
| Temperature | 70°C |
| Propylene Pressure | 7.93 bar (115 psi) |
| Total Volume | 5 mL |
| Solvent | Isohexane |
| Aluminum compound (scavenger) | 4 μmol tri-*n*-octyl aluminum (TNOAL) |
| Quench Value | 1.0 bar (15 psi) |
| Maximum Reaction Time | 45 in |

**Table 10.** Catalyst activity and polymer properties for propylene homopolymerization (PP) using supported catalysts.

| Entry | Run | Catalyst | Experiment | time (s) | yield (g) | activity-(g/mmol- hr) | Mw (kg/mol) | Mn (kg/mol) | Mw/Mn | Tm (°C) |
|---|---|---|---|---|---|---|---|---|---|---|
| 1 | 1 | 1-Zr_s | PP | 2700.4 | 0.008 | 711 | - | - | - | - |
| 2 | 2 | 1-Zr_s | PP | 2700.5 | 0.011 | 978 | 72 | 39 | 1.845 | 158.8 |
| 3 | 1 | 1-Hf_s | PP | 2700.7 | 0.012 | 1,066 | 142 | 80 | 1.773 | 161.5 |
| 4 | 2 | 1-Hf_s | PP | 2700.6 | 0.014 | 1,244 | 149 | 76 | 1.959 | 162.8 |
| 5 | 1 | 1-Zr_s | PP/H2 | 2700.8 | 0.012 | 1,066 | 34 | 15 | 2.281 | 155.4 |
| 6 | 2 | 1-Zr_s | PP/H2 | 2700.2 | 0.015 | 1,333 | 37 | 19 | 1.951 | 156.0 |
| 7 | 1 | 1-Hf_s | PP/H2 | 2700.8 | 0.012 | 1,066 | 85 | 46 | 1.83 | 160.6 |
| 8 | 2 | 1-Hf_s | PP/H2 | 2700.2 | 0.014 | 1,244 | 78 | 43 | 1.835 | 161.4 |

**Table 11.** Reaction conditions for ethylene-propylene copolymerization (EP) using supported catalysts.

| | |
|---|---|
| Catalyst loading | 0.015 μmol of metal complex |
| Temperature | 70°C |
| Propylene Pressure | 7.93 bar (115 psi) |
| Ethylene Pressure | 8.62 bar (125 psi) |
| Total Volume | 5 mL |
| Solvent | Isohexane |
| Aluminum compound (scavenger) | 4 μmol tri-*n*-octyl aluminum (TNOAL) |
| Quench Value | 1.0 bar (15 psi) |
| Maximum Reaction Time | 45 in |

**Table 12.** Catalyst activity and polymer properties for ethylene-propylene copolymerization (EP) using supported catalysts.

| Entry | Run | Catalyst | time (s) | yield (g) | activity (g/mmol-hr) | Mw (kg/mol) | Mn (kg/mol) | Mw/Mn | wt% propylene ($^1$H NMR) | Tm (°C) |
|---|---|---|---|---|---|---|---|---|---|---|
| 1 | 1 | 1-Zr_s | 190.2 | 0.072 | 90,852 | 194 | 122 | 1.588 | - | 45.4 |
| 2 | 2 | 1-Zr_s | 149.9 | 0.083 | 132,889 | 248 | 150 | 1.652 | 25.8 | - |
| 3 | 1 | 1-Hf_s | 356.7 | 0.058 | 39,024 | 1,344 | 801 | 1.678 | - | 59.1 |
| 4 | 2 | 1-Hf_s | 294.7 | 0.065 | 52,935 | 2,299 | 1,525 | 1.508 | 23.0 | 61.3 |

**Table 13.** Reaction conditions for ethylene-octene copolymerization (EO) using various chain transfer agents (CTA).

| | |
|---|---|
| Catalyst loading | 0.020 μmol |
| Temperature | 100°C |
| Pressure Setpoint | 9.31 bar (135 psi) |
| 1-Octene | 100 μL |
| Total Volume | 5 mL |
| Solvent | Isohexane |
| Chain Transfer Agent | variable |
| Quench Value | 1.4 bar (20 psi) |
| Maximum Reaction Time | 30 in |

**Table 14.** Catalyst activity and polymer properties for ethylene-octene copolymerization (EO) using various CTAs.

| Entry | Run | Catalyst | CTA | nmol Al or Zn | time (s) | yield (g) | activity (g/mmol-hr) | Mw corr (kg/mol) | Mn corr (kg/mol) | Mw/Mn | wt% octene | Tm (°C) |
|---|---|---|---|---|---|---|---|---|---|---|---|---|
| 1 | 1 | 1-Zr | diethylzinc | 400 | 30 | 0.088 | 526246 | 97 | 56 | 1.7 | 6.6 | 118 |
| 2 | 2 | 1-Zr | diethylzinc | 400 | 25 | 0.085 | 621951 | 100 | 60 | 1.7 | 4.6 | 119 |
| 3 | 1 | 1-Zr | diethylzinc | 1600 | 23 | 0.076 | 602643 | 51 | 30 | 1.7 | 4.5 | 122 |
| 4 | 2 | 1-Zr | diethylzinc | 1600 | 24 | 0.071 | 532500 | 51 | 32 | 1.6 | 2.8 | 124 |
| 5 | 1 | 1-Zr | diethylzinc | 2800 | 25 | 0.067 | 474803 | 37 | 22 | 1.7 | 4.3 | 126 |
| 6 | 2 | 1-Zr | diethylzinc | 2800 | 26 | 0.070 | 492188 | 35 | 21 | 1.7 | 3.3 | 127 |
| 7 | 1 | 1-Zr | diethylzinc | 4000 | 23 | 0.069 | 533047 | 28 | 15 | 2.0 | 3.6 | 129 |
| 8 | 2 | 1-Zr | diethylzinc | 4000 | 22 | 0.065 | 531818 | 29 | 16 | 1.8 | 2.7 | 132 |
| 9 | 1 | 1-Zr | TNOAL | 400 | 31 | 0.092 | 539414 | 142 | 86 | 1.7 | 3.7 | 118 |
| 10 | 2 | 1-Zr | TNOAL | 400 | 29 | 0.092 | 567123 | 137 | 75 | 1.8 | 4.7 | 118 |
| 11 | 1 | 1-Zr | TNOAL | 1600 | 23 | 0.082 | 650220 | 112 | 61 | 1.8 | 6.8 | 119 |
| 12 | 2 | 1-Zr | TNOAL | 1600 | 23 | 0.075 | 576923 | 113 | 66 | 1.7 | 4.7 | 119 |
| 13 | 1 | 1-Zr | TNOAL | 2800 | 23 | 0.078 | 618502 | 100 | 50 | 2.0 | 4.5 | 120 |
| 14 | 2 | 1-Zr | TNOAL | 2800 | 21 | 0.079 | 667606 | 94 | 45 | 2.1 | 4.2 | 120 |
| 15 | 1 | 1-Zr | TNOAL | 4000 | 24 | 0.073 | 549791 | 86 | 45 | 1.9 | 3.9 | 122 |
| 16 | 2 | 1-Zr | TNOAL | 4000 | 21 | 0.075 | 630841 | 83 | 47 | 1.8 | 3.6 | 124 |
| 17 | 1 | 1-Zr | DIBALO | 400 | 30 | 0.087 | 516832 | 148 | 87 | 1.7 | 5.8 | 118 |
| 18 | 2 | 1-Zr | DIBALO | 400 | 32 | 0.086 | 486792 | 146 | 84 | 1.7 | 5.2 | 118 |
| 19 | 1 | 1-Zr | DIBALO | 1600 | 25 | 0.082 | 583399 | 147 | 85 | 1.7 | 4.9 | 118 |
| 20 | 2 | 1-Zr | DIBALO | 1600 | 26 | 0.083 | 568061 | 137 | 73 | 1.9 | 4.4 | 119 |
| 21 | 1 | 1-Zr | DIBALO | 2800 | 26 | 0.081 | 556489 | 138 | 79 | 1.8 | 5.3 | 119 |
| 22 | 2 | 1-Zr | DIBALO | 2800 | 26 | 0.083 | 574615 | 137 | 71 | 1.9 | 4.5 | 120 |
| 23 | 1 | 1-Zr | DIBALO | 4000 | 25 | 0.086 | 619200 | 141 | 79 | 1.8 | 4.7 | 122 |
| 24 | 2 | 1-Zr | DIBALO | 4000 | 24 | 0.084 | 643404 | 131 | 72 | 1.8 | 3.4 | 124 |
| 25 | 1 | 1-Hf | diethylzinc | 400 | 179 | 0.048 | 48376 | 110 | 69 | 1.6 | 4.0 | 121 |
| 26 | 2 | 1-Hf | diethylzinc | 400 | 164 | 0.046 | 50396 | 100 | 60 | 1.7 | 3.5 | 121 |
| 27 | 1 | 1-Hf | diethylzinc | 1600 | 224 | 0.044 | 35373 | 44 | 28 | 1.6 | 3.4 | 124 |
| 28 | 2 | 1-Hf | diethylzinc | 1600 | 369 | 0.040 | 19528 | 36 | 20 | 1.8 | 5.1 | 124 |

(continued)

| Entry | Run | Catalyst | CTA | nmol Al or Zn | time (s) | yield (g) | activity (g/mmol-hr) | Mw corr (kg/mol) | Mn corr (kg/mol) | Mw/Mn | wt% octene | Tm (°C) |
|---|---|---|---|---|---|---|---|---|---|---|---|---|
| 29 | 1 | 1-Hf | diethylzinc | 2800 | 427 | 0.037 | 15590 | 25 | 14 | 1.7 | 4.0 | 128 |
| 30 | 2 | 1-Hf | diethylzinc | 2800 | 277 | 0.041 | 26681 | 25 | 13 | 1.9 | 3.8 | 128 |
| 31 | 1 | 1-Hf | diethylzinc | 4000 | 310 | 0.034 | 19729 | 18 | 8 | 2.2 | 3.8 | 130 |
| 32 | 2 | 1-Hf | diethylzinc | 4000 | 304 | 0.038 | 22500 | 18 | 10 | 1.8 | 5.0 | 128 |
| 33 | 1 | 1-Hf | TNOAL | 400 | 378 | 0.086 | 40942 | 606 | 351 | 1.7 | 6.6 | 114 |
| 34 | 2 | 1-Hf | TNOAL | 400 | 299 | 0.077 | 46386 | 684 | 428 | 1.6 | 5.1 | 115 |
| 35 | 1 | 1-Hf | TNOAL | 1600 | 154 | 0.050 | 58480 | 427 | 247 | 1.7 | 4.9 | 117 |
| 36 | 2 | 1-Hf | TNOAL | 1600 | 157 | 0.049 | 56036 | 412 | 249 | 1.7 | 4.5 | 117 |
| 37 | 1 | 1-Hf | TNOAL | 2800 | 174 | 0.047 | 48593 | 326 | 188 | 1.7 | 4.6 | 119 |
| 38 | 2 | 1-Hf | TNOAL | 2800 | 182 | 0.045 | 44579 | 296 | 163 | 1.8 | 4.0 | 120 |
| 39 | 1 | 1-Hf | TNOAL | 4000 | 173 | 0.041 | 42733 | 289 | 149 | 1.9 | 3.7 | 122 |
| 40 | 2 | 1-Hf | TNOAL | 4000 | 201 | 0.042 | 37556 | 236 | 130 | 1.8 | 3.7 | 122 |
| 41 | 1 | 1-Hf | DIBALO | 400 | 567 | 0.092 | 29201 | 688 | 379 | 1.8 | 6.5 | 113 |
| 42 | 2 | 1-Hf | DIBALO | 400 | 189 | 0.059 | 56101 | 787 | 474 | 1.7 | 4.6 | 115 |
| 43 | 1 | 1-Hf | DIBALO | 1600 | 402 | 0.086 | 38527 | 753 | 452 | 1.7 | 4.9 | 115 |
| 44 | 2 | 1-Hf | DIBALO | 1600 | 168 | 0.053 | 56752 | 763 | 437 | 1.7 | 5.6 | 117 |
| 45 | 1 | 1-Hf | DIBALO | 2800 | 217 | 0.066 | 54822 | 744 | 377 | 2.0 | 3.5 | 119 |
| 46 | 2 | 1-Hf | DIBALO | 2800 | 147 | 0.048 | 58856 | 774 | 386 | 2.0 | 3.4 | 120 |
| 47 | 1 | 1-Hf | DIBALO | 4000 | 183 | 0.053 | 52131 | 789 | 391 | 2.0 | 2.7 | 122 |
| 1-48 | 2 | 1-Hf | DIBALO | 4000 | 180 | 0.057 | 57095 | 824 | 495 | 1.7 | 3.1 | 122 |

DIBALO is bis(diisobutylaluminum)oxide. Entries 1-8 and 25-32 had 300 nmol of TNOAL in addition to the diethylzinc reagent. Mw corr and Mn corr correspond to the GPC values (based on polystyrene standards) for Mw and Mn, respectively, divided by 2 to correct for EO.

The procedure of Tables 3 and 4 was repeated except that the temperature was 85 or 100°C and the setpoint pressure is 8.96 or 9.65 bar (130 or 140 psi), as indicated in Table 15. The data are presented in Table 15.

**Table 15.** Propylene Polymerization Using Activator-1.

| Catalyst | T (°C) | P set point bar (psi) | Uptake bar (psig) | time (s) | Yield (g) | activity (kg/mmol-hr) | Mw (g/mol) | Mn (g/mol) | Mw/Mn | Tm **(°C)** | Hf (J/g) |
|---|---|---|---|---|---|---|---|---|---|---|---|
| 1-Zr | | | | | | | | | | | |
| | | | | | | | | | | | |
| | 85 | 8.96 (130) | 0.32 (4.7) | 1097.3 | 0.045 | 4 | 95 | 59 | 1.6 | 158.7 | 158 |
| | | | 0.32 (4.7) | 1096.1 | 0.045 | 4 | 91 | 54 | 1.7 | 158.0 | 153 |
| | 100 | 9.65 (140) | 0.06 (0.9) | 1054.7 | 0.012 | 1 | 36 | 23 | 1.6 | 153.1 | 91 |
| | | | 0.04 (0.6) | 957.7 | 0.012 | 1 | 35 | 20 | 1.7 | 154.1 | 154 |
| 1-Hf | | | | | | | | | | | |
| | | | | | | | | | | | |
| | 85 | 8.96 (130) | 0.10 (1.5) | 1018.1 | 0.008 | 1 | - | - | - | - | - |
| | | | 0.075 (11) | 949.2 | 0.008 | 1 | - | - | - | - | - |
| | 100 | 9.65 (140) | 0.06 (0.8) | 907.8 | 0.004 | 0 | - | - | - | - | - |
| | | | 0.06 (0.9) | 973.5 | 0.004 | 0 | - | - | - | - | - |

Figure 2 shows chain transfer efficiency of 1-Zr (data from Table 14, Entries 1-24). The equation and coefficient of determination of the linear fits (least squared fit, Microsoft™ Excel 2010) are included in the figure. The slope of the linear fit corresponds to the number of chains transferred to the CTA metal (per metal). The figure shows that diethylzinc is an effective CTA with 1-Zr.

Figure 3 shows the chain transfer efficiency of 1-Hf (data from Table 14, Entries 1-24). The equation and coefficient of determination of the linear fits (least squared fit, Microsoft™ Excel 2010) are included in the figure. The slope of the linear fit corresponds to the number of chains transferred to the CTA metal (per metal). The figure shows that diethylzinc is an effective CTA with 1-Hf.

**Additional GPC Analysis for Selected Polymer Samples.** Mw, Mn, and Mw/Mn are determined by using a High Temperature Size Exclusion Chromatograph (Polymer Laboratories), equipped with three in-line detectors, a differential refractive index detector (DRI), a light scattering (LS) detector, and a viscometer. Experimental details, including detector calibration, are described in: T. Sun, P. Brant, R. R. Chance, and W. W. Graessley, Macromolecules, Volume 34, Number 19, pp. 6812-6820, (2001), and references therein. Three Polymer Laboratories PLgel 10μm Mixed-B LS columns are used. The nominal flow rate is 0.5 mL/min, and the nominal injection volume is 300 μL. The various transfer lines, columns, viscometer and differential refractometer (the DRI detector) are contained in an oven maintained at 145°C. Solvent for the experiment is prepared by dissolving 6 grams of butylated hydroxytoluene as an antioxidant in 4 liters of Aldrich reagent grade 1, 2, 4 trichlorobenzene (TCB). The TCB mixture is then filtered through a 0.1 μm Teflon filter. The TCB is then degassed with an online degasser before entering the Size Exclusion Chromatograph. Polymer solutions are prepared by placing dry polymer in a glass container, adding the desired amount of TCB, then heating the mixture at 160°C with continuous shaking for about 2 hours. All quantities are measured gravimetrically. The TCB densities used to express the polymer concentration in mass/volume units are 1.463 g/ml at room temperature and 1.284 g/ml at 145°C. The injection concentration is from 0.5 to 2.0 mg/ml, with lower concentrations being used for higher molecular weight samples. Prior to running each sample the DRI detector and the injector are purged. Flow rate in the apparatus is then increased to 0.5 ml/minute, and the DRI is allowed to stabilize for 8 to 9 hours before injecting the first sample. The LS laser is turned on at least 1 to 1.5 hours before running the samples. The concentration, c, at each point in the chromatogram is calculated from the baseline-subtracted DRI signal, IDRI, using the following equation:

$$c = K_{DRI} I_{DRI} /(dn/dc)$$

where KDRI is a constant determined by calibrating the DRI, and (dn/dc) is the refractive index increment for the system. The refractive index, n = 1.500 for TCB at 145°C and λ = 660 nm. Units on parameters throughout this description of the GPC-SEC method are such that concentration is expressed in g/cm3, molecular weight is expressed in g/mole, and intrinsic viscosity is expressed in dL/g.

The LS detector is a Wyatt Technology High Temperature DAWN HELEOS. The molecular weight, M, at each point in the chromatogram is determined by analyzing the LS output using the Zimm model for static light scattering (M.B. Huglin, LIGHT SCATTERING FROM POLYMER SOLUTIONS, Academic Press, 1971):

$$\frac{K_o c}{\Delta R(\theta)} = \frac{1}{MP(\theta)} + 2A_2 c\ .$$

Here, ΔR(θ) is the measured excess Rayleigh scattering intensity at scattering angle θ, c is the polymer concentration determined from the DRI analysis, $A_2$ is the second virial coefficient, for purposes of this invention $A_2$ = 0.0006, (dn/dc) is the refractive index increment for the system. P(θ) is the form factor for a monodisperse random coil, and $K_o$ is the optical constant for the system:

$$K_o = \frac{4\pi^2 n^2 (dn/dc)^2}{\lambda^4 N_A}$$

where NA is Avogadro's number, and (dn/dc) is the refractive index increment for the system. The refractive index, n = 1.500 for TCB at 145°C and λ = 660 nm.

A high temperature Viscotek Corporation viscometer, which has four capillaries arranged in a Wheatstone bridge configuration with two pressure transducers, is used to determine specific viscosity. One transducer measures the total pressure drop across the detector, and the other, positioned between the two sides of the bridge, measures a differential pressure. The specific viscosity, $\eta_s$, for the solution flowing through the viscometer is calculated from their

outputs. The intrinsic viscosity, [η], at each point in the chromatogram is calculated from the following equation:

$$\eta_s = c[\eta] + 0.3(c[\eta])^2$$

where c is concentration and was determined from the DRI output.

The branching index ($g'_{vis}$) is calculated using the output of the SEC-DRI-LS-VIS method as follows. The average intrinsic viscosity, $[\eta]_{avg}$, of the sample is calculated by:

$$[\eta]_{avg} = \frac{\sum c_i [\eta]_i}{\sum c_i}$$

where the summations are over the chromatographic slices, i, between the integration limits.

The branching index g'vis is defined as:

$$g'vis = \frac{[\eta]_{avg}}{kM_v^{\alpha}}$$

where, for purpose of this invention and claims thereto, $\alpha$ = 0.695 and k = 0.000579 for linear ethylene polymers, $\alpha$ = 0.705 k = 0.000262 for linear propylene polymers, and $\alpha$ = 0.695 and k = 0.000181 for linear butene polymers. $M_v$ is the viscosity-average molecular weight based on molecular weights determined by LS analysis. See Macromolecules, 2001, 34, 6812-6820 and Macromolecules, 2005, 38, 7181-7183, for guidance on selecting a linear standard having similar molecular weight and comonomer content, and determining k coefficients and $\alpha$ exponents.

As is apparent from the foregoing general description and the specific embodiments, while forms of the invention have been illustrated and described, various modifications can be made. Accordingly, it is not intended that the invention be limited thereby. The term "comprising" is considered synonymous with the term "including." Likewise, whenever a composition, an element, or a group of elements is preceded with the transitional phrase "comprising," it is understood that we also contemplate the same composition or group of elements with transitional phrases "consisting essentially of," "consisting of," "selected from the group of consisting of," or "is" preceding the recitation of the composition, element, or elements and vice versa.

## Claims

**1.** A transition metal complex represented by the formula (I):

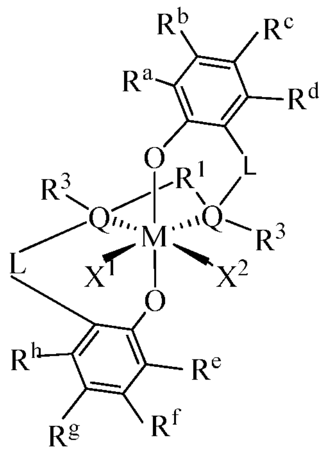

(I)

wherein M is Hf or Zr;

each Q is neutral donor group comprising at least one atom from O, N, S or P, and $R^3$ is not present when Q

is O or S;
each L is independently

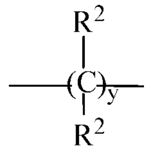

and is not part of an aromatic ring;
y is 2, 3, 4, 5, or 6;
$X^1$ and $X^2$ are, independently, a univalent $C_1$ to $C_{20}$ hydrocarbyl radical, a $C_1$ to $C_{20}$ substituted hydrocarbyl radical, a heteroatom or a heteroatom-containing group, or $X^1$ and $X^2$ join together to form a $C_4$ to $C_{62}$ cyclic, polycyclic or heterocyclic structure;
$R^1$ is a divalent $C_1$-$C_{40}$ hydrocarbyl radical or divalent substituted hydrocarbyl radical comprising a portion that comprises a linker backbone comprising from 1 to 18 carbon atoms linking or bridging between the two Q groups;
each $R^a$, $R^b$, $R^c$, $R^d$, $R^e$, $R^f$, $R^g$, and $R^h$ is, independently, a hydrogen, a $C_1$-$C_{40}$ hydrocarbyl radical, a $C_1$-$C_{40}$ substituted hydrocarbyl radical, a heteroatom or a heteroatom-containing group, or two or more of $R^a$ to $R^h$ may independently join together to form a $C_4$ to $C_{62}$ cyclic, polycyclic or heterocyclic structure, or a combination thereof;
each $R^2$ is, independently, a hydrogen, a $C_1$-$C_{40}$ hydrocarbyl radical, a $C_1$-$C_{40}$ substituted hydrocarbyl radical, a heteroatom or a heteroatom-containing group, or two or more adjacent $R^2$ groups may independently join together to form a $C_4$ to $C_{62}$ cyclic or polycyclic ring structure, or a combination thereof, provided that such cyclic or polycyclic ring structure is not aromatic; and each $R^3$ is, independently, a hydrogen, a $C_1$-$C_{40}$ hydrocarbyl radical, a $C_1$-$C_{40}$ substituted hydrocarbyl radical, a heteroatom or a heteroatom-containing group,
wherein a substituted hydrocarbyl radical is a radical made of carbon and hydrogen where at least one hydrogen is replaced by a heteroatom or heteroatom-containing group.

2. The transition metal complex of claim 1 wherein the complex is represented by the formula (II)

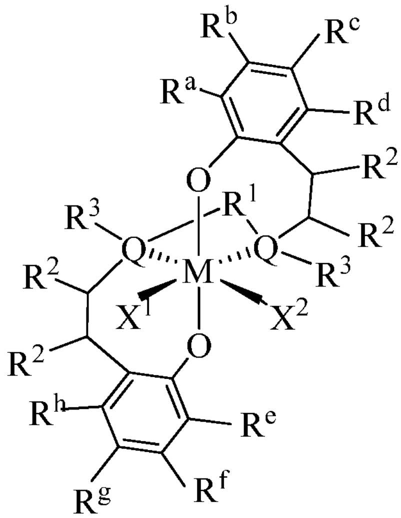

(II)

wherein M, Q, $X^1$ , $X^2$, $R^1$, $R^2$, $R^3$ $R^a$, $R^b$, $R^c$, $R^d$, $R^e$, $R^f$, $R^g$, and $R^h$ have the same meaning as in claim 1.

3. The transition metal complex of claim 1 or 2, wherein $R^a$ and $R^e$ are carbazolyl, substituted carbazolyl, indolyl, substituted indolyl, indolinyl, substituted indolinyl, imidazolyl, substituted imidazolyl, indenyl, substituted indenyl, indanyl, substituted indanyl, fluorenyl, or substituted fluorenyl, preferably $R^a$ and $R^e$ are the same.

4. The transition metal complex of any of claims 1 to 3, wherein Q is a neutral donor group comprising at least one atom from O, N, S or P and the -(-Q-$R^1$-Q-)- fragment can form a substituted or unsubstituted heterocycle which may or may not be aromatic and may have multiple fused rings, preferably wherein Q is $NR'_2$, OR', SR', $PR'_2$, where

R’ is as defined for $R^a$.

5. The transition metal complex of any of claims 1 to 4, wherein $R^a$ and $R^e$ are carbazolyl or substituted carbazolyl.

6. The transition metal complex of any of claims 1 to 5, wherein $R^1$ is a divalent $C_1$-$C_{20}$ hydrocarbyl radical or divalent substituted hydrocarbyl radical comprising a portion that comprises a linker backbone comprising from 1 to 18 carbon atoms linking or bridging between the two Q groups.

7. A catalyst system comprising activator, preferably an alumoxane and/or a non-coordinating anion and the complex of any of claims 1 to 6.

8. The catalyst system of claim 7, wherein the catalyst system further comprises chain transfer agent, preferably represented by the formula $R_3Al$ or $R_2Zn$, where R is a $C_1$ to $C_{20}$ alkyl group.

9. The catalyst system of claim 7 or 8, wherein the activator is selected from the group consisting of: methylalumoxane, ethylalumoxane, isobutyl alumoxane, N,N-dimethylanilinium tetra(perfluorophenyl)borate, N,N-dimethylanilinium tetrakis(perfluoronaphthyl)borate, N,N-dimethylanilinium tetrakis(perfluorobiphenyl)borate, N,N-dimethylanilinium tetrakis(3,5-bis(trifluoromethyl)phenyl)borate, triphenylcarbenium tetrakis(perfluoronaphthyl)borate, triphenylcarbenium tetrakis(perfluorobiphenyl)borate, triphenylcarbenium tetrakis(3,5-bis(trifluoromethyl)phenyl)borate, triphenylcarbenium tetra(perfluorophenyl)borate, triphenylcarbenium tetrakis(perfluorophenyl)borate, 1-(4-(tris(pentafluorophenyl)borate)-2,3,5,6-tetrafluorophenyl)pyrrolidinium, tetrakis(pentafluorophenyl)borate, and 4-(tris(pentafluorophenyl)borate)-2,3,5,6-tetrafluoropyridine.

10. A polymerization process to produce polyolefin comprising:

a) contacting one or more olefin monomers, preferably comprising ethylene and/or propylene, with the catalyst system of any of claims 7 to 9, and
b) recovering olefin polymer.

11. The process of claim 10, wherein the transition metal complex is supported.

12. The process of any of claims 10 or 11, wherein chain transfer agent represented by the formula $R_3Al$ or $R_2Zn$ is present at a molar ratio of transition metal to Al and Zn of at least 10:1, where R is a $C_1$ to $C_{40}$ alkyl group.

13. The process of any of claims 10 to 12, wherein the process is a continuous process.

14. The process of any of claims 10 to 13, wherein step a) occurs at a temperature of at least 70°C, preferably wherein hydrogen is present in step a), more preferably wherein hydrogen and chain transfer agent are present in step a).

15. The transition metal complex of claim 1 represented by the formula:

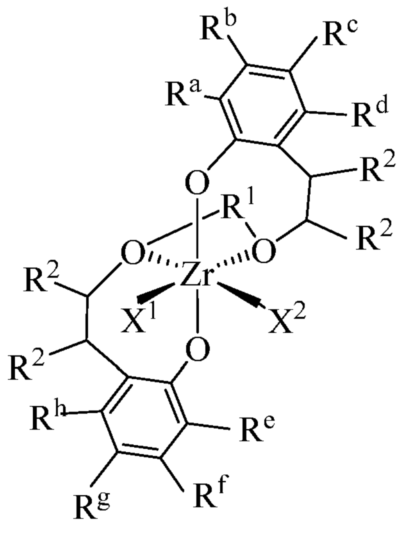

wherein $X^1$ and $X^2$, $R^1$, $R^b$, $R^c$, $R^d$, $R^f$, $R^g$, $R^h$ and $R^2$ have the same meaning as in any of the preceding claims; and each $R^a$ and $R^e$ is, independently, phenyl or substituted phenyl.

**16.** The transition metal complex of claim 1 represented by the formula:

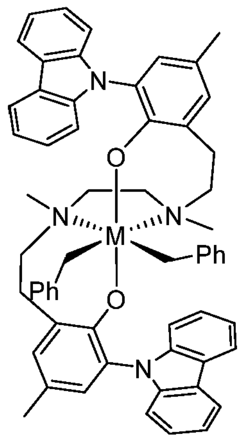
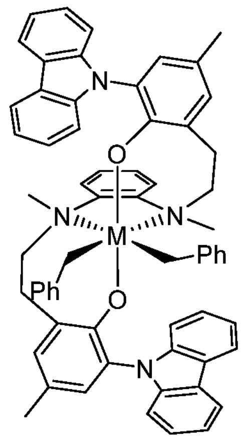
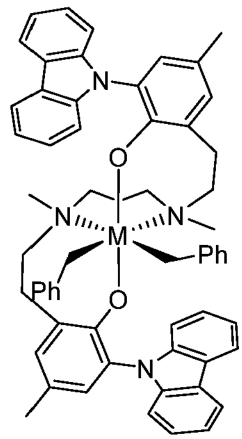
,

wherein M is Hf or Zr.

**Patentansprüche**

**1.** Übergangsmetallkomplex, der durch die Formel (I) wiedergegeben wird:

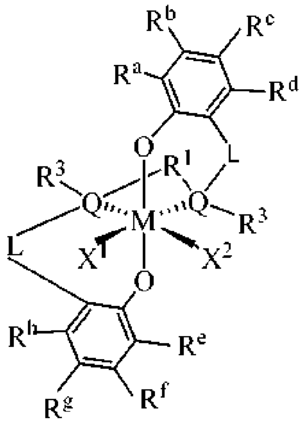
(I)

wobei M Hf oder Zr ist,
jedes Q eine neutrale Donorgruppe ist, die mindestens ein Atom aus O, N, S oder P umfasst, und $R^3$ nicht vorhanden ist, wenn Q O oder S ist,
jedes L unabhängig

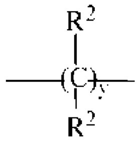

ist und kein Teil eines aromatischen Rings ist,
y 2, 3, 4, 5 oder 6 ist,
$X^1$ und $X^2$ unabhängig ein einwertiger $C_1$- bis $C_{20}$-Kohlenwasserstoffrest, ein substituierter $C_1$- bis $C_{20}$-Kohlen-

wasserstoffrest, ein Heteroatom oder eine heteroatomhaltige Gruppe sind, oder $X^1$ und $X^2$ unter Bildung einer cyclischen, polycyclischen oder heterocyclischen $C_4$- bis $C_{62}$-Ringstruktur miteinander verbunden sind,
$R^1$ ein zweiwertiger $C_1$- bis $C_{40}$-Kohlenwasserstoffrest oder zweiwertiger substituierter Kohlenwasserstoffrest ist, der einen Anteil umfasst, der ein Linkergrundgerüst umfasst, welches 1 bis 18 Kohlenstoffatome umfasst, welche die beiden Q-Gruppen verlinken oder verbrücken,
jedes $R^a$, $R^b$, $R^c$, $R^d$, $R^e$, $R^f$, $R^g$ und $R^h$ unabhängig Wasserstoff, ein $C_1$- bis $C_{40}$-Kohlenwasserstoffrest, ein substituierter $C_1$-bis $C_{40}$-Kohlenwasserstoffrest, ein Heteroatom oder eine heteroatomhaltige Gruppe ist, oder zwei oder mehr von $R^a$ bis $R^h$ unabhängig miteinander verbunden sein können, um eine cyclische, polycyclische oder heterocyclische $C_4$- bis $C_{62}$-Ringstruktur zu bilden, oder eine Kombination davon,
jedes $R^2$ unabhängig Wasserstoff, ein $C_1$- bis $C_{40}$-Kohlenwasserstoffrest, ein substituierter $C_1$- bis $C_{40}$-Kohlenwasserstoffrest, ein Heteroatom oder eine heteroatomhaltige Gruppe ist, oder zwei oder mehr benachbarte Gruppen $R^2$ unabhängig miteinander verbunden sein können, um eine cyclische oder polycyclische $C_4$- bis $C_{62}$-Ringstruktur zu bilden, oder eine Kombination davon, mit der Maßgabe, dass diese cyclische oder polycyclische Ringstruktur nicht aromatisch ist, und
jedes $R^3$ unabhängig Wasserstoff, ein $C_1$- bis $C_{40}$-Kohlenwasserstoffrest, ein substituierter $C_1$- bis $C_{40}$-Kohlenwasserstoffrest, ein Heteroatom oder eine heteroatomhaltige Gruppe ist, wobei ein substituierter Kohlenwasserstoffrest ein Rest aus Kohlenstoff und Wasserstoff ist, wobei mindestens ein Wasserstoff durch ein Heteroatom oder eine heteroatomhaltige Gruppe ersetzt wird.

**2.** Übergangsmetallkomplex nach Anspruch 1, bei dem der Komplex durch Formel (II) wiedergegeben wird:

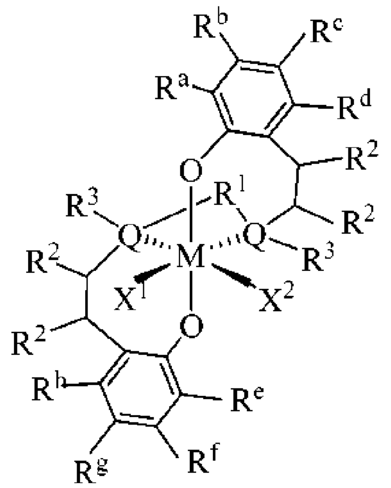

**(II)**

wobei M, Q, $X^1$ $X^2$, $R^1$, $R^2$, $R^3$, $R^a$, $R^b$, $R^c$, $R^d$, $R^e$, $R^f$, $R^g$ und $R^h$ die gleiche Bedeutung wie in Anspruch 1 haben.

**3.** Übergangsmetallkomplex nach Anspruch 1 oder 2, bei dem $R^a$ und $R^e$ Carbazolyl, substituiertes Carbazolyl, Indolyl, substituiertes Indolyl, Indolinyl, substituiertes Indolinyl, Imidazolyl, substituiertes Imidazolyl, Indenyl, substituiertes Indenyl, Indanyl, substituiertes Indanyl, Fluorenyl oder substituiertes Fluorenyl sind, wobei vorzugsweise $R^a$ und $R^e$ gleich sind.

**4.** Übergangsmetallkomplex nach einem der Ansprüche 1 bis 3, bei dem Q eine neutrale Donorgruppe ist, die mindestens ein Atom aus O, N, S oder P umfasst, und wobei das Fragment -(-Q-$R^1$-Q-)-einen substituierten oder unsubstituierten Heterocyclus bilden kann, der aromatisch sein kann oder nicht und mehrere kondensierte Ringe aufweisen kann, wobei Q vorzugsweise $NR'_2$, OR', SR', $PR'_2$ ist, wobei R' wie für $R^a$ definiert ist.

**5.** Übergangsmetallkomplex nach einem der Ansprüche 1 bis 4, bei dem $R^a$ und $R^e$ Carbazolyl oder substituiertes Carbazolyl sind.

**6.** Übergangsmetallkomplex nach einem der Ansprüche 1 bis 5, bei dem $R^1$ ein zweiwertiger $C_1$- bis $C_{20}$-Kohlenwasserstoffrest oder zweiwertiger substituierter Kohlenwasserstoffrest ist, der einen Anteil umfasst, der ein Linkergrundgerüst umfasst, welches 1 bis 18 Kohlenstoffatome umfasst, welche die beiden Q-Gruppen verlinken oder verbrücken,

**7.** Katalysatorsystem, das Aktivator, vorzugsweise Alumoxan und/oder nicht-koordinierendes Anion, und den Komplex nach einem der Ansprüche 1 bis 6 umfasst.

**8.** Katalysatorsystem nach Anspruch 7, das des Weiteren Kettenübertragungsmittel umfasst, das vorzugsweise durch die Formel $R_3Al$ oder $R_2Zn$ wiedergegeben wird, wobei R eine $C_1$- bis $C_{20}$-Alkylgruppe ist.

**9.** Katalysatorsystem Anspruch 7 oder 8, bei dem der Aktivator ausgewählt ist aus der Gruppe bestehend aus: Methylalumoxan, Ethylalumoxan, Isobutylalumoxan, N,N-Dimethylaniliniumtetra(perfluorphenyl)borat, N,N-Dimethylaniliniumtetrakis-(perfluornaphthyl)borat, N,N-Dimethylaniliniumtetrakis(perfluorbiphenyl)borat, N,N-Dimethylaniliniumtetrakis(3,5-bis-(trifluormethyl)phenyl)borat, Triphenylcarbeniumtetrakis-(perfluornaphthyl)borat, Triphenylcarbeniumtetrakis(perfluorbiphenyl)borat, Triphenylcarbeniumtetrakis(3,5-bis-(trifluormethyl)phenyl)borat, Triphenylcarbeniumtetra(perfluorphenyl)borat, Triphenylcarbeniumtetrakis(perfluorphenyl)borat, 1-(4-(Tris(pentafluorphenyl)borat)-2,3,5,6-tetrafluorphenyl)pyrrolidinium, Tetrakis(pentafluorphenyl)-borat und 4-(Tris(pentafluorphenyl)borat)-2,3,5,6-tetrafluorpyridin.

**10.** Polymerisationsverfahren zur Produktion von Polyolefin, bei dem

a) ein oder mehr Olefinmonomere, die vorzugsweise Ethylen und/oder Propylen umfassen, mit dem Katalysatorsystem gemäß einem der Ansprüche 7 bis 9 in Kontakt gebracht werden, und
b) Olefinpolymer gewonnen wird.

**11.** Verfahren nach Anspruch 10, bei dem der Übergangsmetallkomplex trägergestützt ist.

**12.** Verfahren nach Anspruch 10 oder 11, bei dem Kettenübertragungsmittel, das durch die Formel $R_3Al$ oder $R_2Zn$ wiedergegeben wird, in einem Molverhältnis von Übergangsmetall zu Al und Zn von mindestens 10:1 vorhanden sind, wobei R eine $C_1$- bis $C_{40}$-Alkylgruppe ist.

**13.** Verfahren nach einem der Ansprüche 10 bis 12, das ein kontinuierliches Verfahren ist.

**14.** Verfahren nach einem der Ansprüche 10 bis 13, bei dem Schritt a) bei einer Temperatur von mindestens 70°C stattfindet, wobei vorzugsweise in Schritt a) Wasserstoff vorhanden ist, wobei insbesondere in Schritt a) Wasserstoff und Kettenübertragungsmittel vorhanden sind.

**15.** Übergangsmetallkomplex nach Anspruch 1, der durch die Formel wiedergegeben wird:

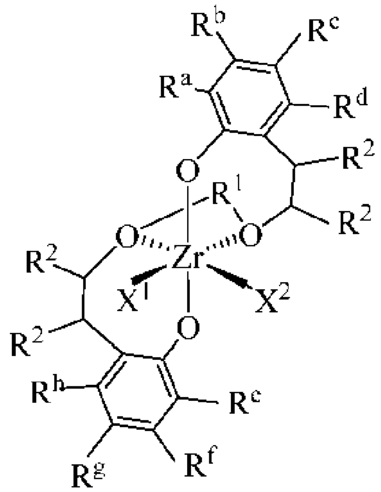

wobei $X^1$ und $X^2$, $R^1$, $R^b$, $R^c$, $R^d$, $R^f$, $R^g$, $R^h$ und $R^2$ die gleiche Bedeutung wie in einem der vorhergehenden Ansprüche haben, und jedes $R^a$ und $R^e$ unabhängig Phenyl oder substituiertes Phenyl ist.

**16.** Übergangsmetallkomplex nach Anspruch 1, der durch die folgende Formel wiedergegeben wird:

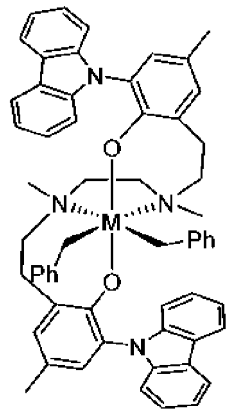
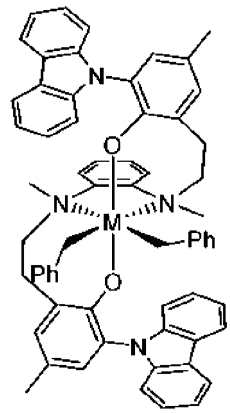
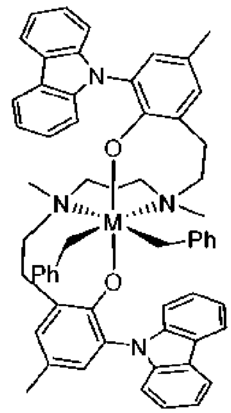
,

wobei M Hf oder Zr ist.

## Revendications

**1.** Complexe de métal de transition représenté par la formule (I) :

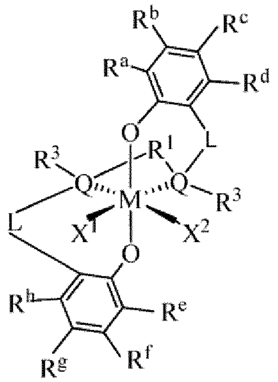
(I)

dans lequel M est Hf ou Zr ;

chaque Q est un groupe donneur neutre comprenant au moins un atome parmi O, N, S ou P, et $R^3$ n'est pas présent lorsque Q est O ou S ;

chaque L est indépendamment

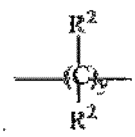

et ne fait pas partie d'un cycle aromatique ;

y est 2, 3, 4, 5 ou 6 ;

$X^1$ et $X^2$ sont, indépendamment, un radical hydrocarbyle en $C_1$ à $C_{20}$ monovalent, un radical hydrocarbyle substitué en $C_1$ à $C_{20}$, un hétéroatome ou un groupe contenant un hétéroatome, ou $X^1$ et $X^2$ sont assemblés conjointement pour former une structure cyclique, polycyclique ou hétérocyclique en $C_4$ à $C_{62}$ ;

$R^1$ est un radical hydrocarbyle en $C_1$-$C_{40}$ divalent ou un radical hydrocarbyle substitué divalent comprenant une partie qui comprend une chaîne principale de lieur comprenant de 1 à 18 atomes de carbone reliant ou pontant entre les deux groupes Q ;

chaque $R^a$, $R^b$, $R^c$, $R^d$, $R^e$, $R^f$, $R^g$ et $R^h$ est, indépendamment, un hydrogène, un radical hydrocarbyle en $C_1$-$C_{40}$, un radical hydrocarbyle en $C_1$-$C_{40}$ substitué, un hétéroatome ou un groupe contenant un hétéroatome, ou deux ou plus de deux parmi $R^a$ à $R^h$ peuvent indépendamment être assemblés conjointement pour former une

structure cyclique, polycyclique ou hétérocyclique en $C_4$ à $C_{62}$, ou une combinaison de celles-ci ; chaque $R^2$ est, indépendamment, un hydrogène, un radical hydrocarbyle en $C_1$-$C_{40}$, un radical hydrocarbyle en $C_1$-$C_{40}$ substitué, un hétéroatome ou un groupe contenant un hétéroatome, ou deux ou plus de deux groupes $R^2$ adjacents peuvent indépendamment être assemblés conjointement pour former une structure d'anneau cyclique ou polycyclique en $C_4$ à $C_{62}$, ou une combinaison de celles-ci, à condition qu'une telle structure d'anneau cyclique ou polycyclique ne soit pas aromatique ; et chaque $R^3$ est, indépendamment, un hydrogène, un radical hydrocarbyle en $C_1$-$C_{40}$, un radical hydrocarbyle en $C_1$-$C_{40}$ substitué, un hétéroatome ou un groupe contenant un hétéroatome,

dans lequel un radical hydrocarbyle substitué est un radical constitué de carbone et d'hydrogène où au moins un hydrogène est remplacé par un hétéroatome ou un groupe contenant un hétéroatome.

**2.** Complexe de métal de transition selon la revendication 1 dans lequel le complexe est représenté par la formule (II)

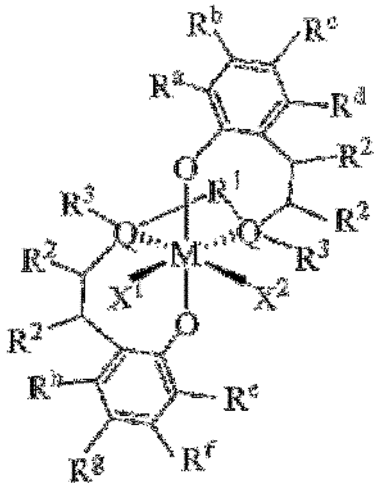

(II)

dans lequel M, Q, $X^1$, $X^2$, $R^1$, $R^2$, $R^3$, $R^a$, $R^b$, $R^c$, $R^d$, $R^e$, $R^f$, $R^g$ et $R^h$ ont la même signification que dans la revendication 1.

**3.** Complexe de métal de transition selon la revendication 1 ou 2, dans lequel $R^a$ et $R^e$ sont carbazolyle, carbazolyle substitué, indolyle, indolyle substitué, indolinyle, indolinyle substitué, imidazolyle, imidazolyle substitué, indényle, indényle substitué, indanyle, indanyle substitué, fluorényle ou fluorényle substitué, de préférence $R^a$ et $R^e$ sont identiques.

**4.** Complexe de métal de transition selon l'une quelconque des revendications 1 à 3, dans lequel Q est un groupe donneur neutre comprenant au moins un atome parmi O, N, S ou P et le fragment - (-Q-$R^1$-Q-) - peut former un hétérocycle substitué ou non substitué qui peut être aromatique ou non et peut comporter des cycles condensés multiples, de préférence dans lequel Q est $NR'_2$, OR', SR', $PR'_2$, où R' est tel que défini pour $R^a$.

**5.** Complexe de métal de transition selon l'une quelconque des revendications 1 à 4, dans lequel $R^a$ et $R^e$ sont carbazolyle ou carbazolyle substitué.

**6.** Complexe de métal de transition selon l'une quelconque des revendications 1 à 5, dans lequel $R^1$ est un radical hydrocarbyle en $C_1$-$C_{20}$ divalent ou un radical hydrocarbyle divalent substitué comprenant une partie qui comprend une chaîne principale de lieur comprenant de 1 à 18 atomes de carbone reliant ou pontant entre les deux groupes Q.

**7.** Système de catalyseur comprenant un activateur, de préférence un alumoxane et/ou un anion non coordinateur et le complexe selon l'une quelconque des revendications 1 à 6.

**8.** Système de catalyseur selon la revendication 7, où le système de catalyseur comprend en outre un agent de transfert de chaîne, de préférence représenté par la formule $R_3Al$ ou $R_2Zn$, où R est un groupe alkyle en $C_1$ à $C_{20}$.

**9.** Système de catalyseur selon la revendication 7 ou 8, dans lequel l'activateur est choisi dans le groupe constitué de : méthylalumoxane, éthylalumoxane, isobutylalumoxane, tétra(perfluorophényl)borate de N,N-diméthylanilinium,

tétrakis(perfluoronaphtyl)borate de N,N-diméthylanilinium, tétrakis(perfluorobiphényl)borate de N,N-diméthylanilinium, tétrakis(3,5-bis(trifluorométhyl)phényl)borate de N,N-diméthylanilinium, tétrakis(perfluoronaphtyl)borate de triphénylcarbénium, tétrakis(perfluorobiphényl)borate de triphénylcarbénium, tétrakis(3,5-bis(trifluorométhyl)phényl)borate de triphénylcarbénium, tétra(perfluorophényl)borate de triphénylcarbénium, tétrakis(perfluorophényl)borate de triphénylcarbénium, 1-(4-(tris(pentafluorophényl)borate)-2,3,5,6-tétrafluorophényl)pyrrolidinium, tétrakis(pentafluorophényl)borate et 4-(tris(pentafluorophényl)borate)-2,3,5, 6-tétrafluoropyridine.

**10.** Procédé de polymérisation pour produire une polyoléfine comprenant :

a) la mise en contact d'un ou plusieurs monomères oléfiniques, de préférence comprenant de l'éthylène et/ou du propylène, avec le système de catalyseur selon l'une quelconque des revendications 7 à 9, et
b) la récupération du polymère d'oléfine.

**11.** Procédé selon la revendication 10, dans lequel le complexe de métal de transition est immobilisé sur un support.

**12.** Procédé selon l'une quelconque des revendications 10 ou 11, dans lequel l'agent de transfert de chaîne représenté par la formule $R_3Al$ ou $R_2Zn$ est présent à un rapport molaire du métal de transition à Al et Zn d'au moins 10:1, où R est un groupe alkyle en $C_1$ à $C_{40}$.

**13.** Procédé selon l'une quelconque des revendications 10 à 12, où le procédé est un procédé continu.

**14.** Procédé selon l'une quelconque des revendications 10 à 13, dans lequel l'étape a) est conduite à une température d'au moins 70 °C, de préférence dans lequel de l'hydrogène est présent dans l'étape a), plus préférablement dans lequel de l'hydrogène et l'agent de transfert de chaîne sont présents dans l'étape a).

**15.** Complexe de métal de transition selon la revendication 1 représenté par la formule :

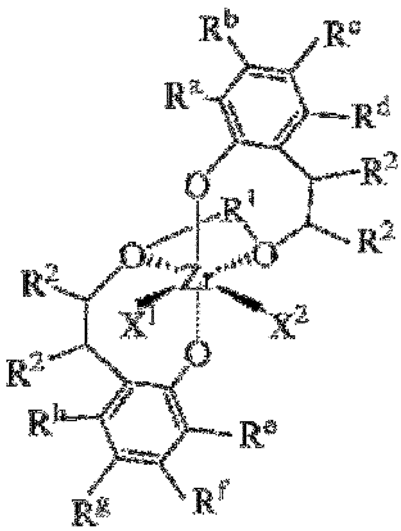

dans lequel $X^1$ et $X^2$, $R^1$, $R^b$, $R^c$, $R^d$, $R^f$, $R^g$, $R^h$ et $R^2$ ont la même signification que dans l'une quelconque des revendications précédentes ; et
chaque $R^a$ et $R^e$ est, indépendamment, phényle ou phényle substitué.

**16.** Complexe de métal de transition selon la revendication 1 représenté par la formule :

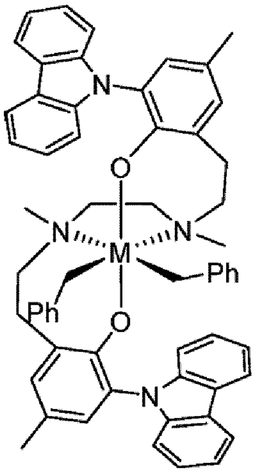 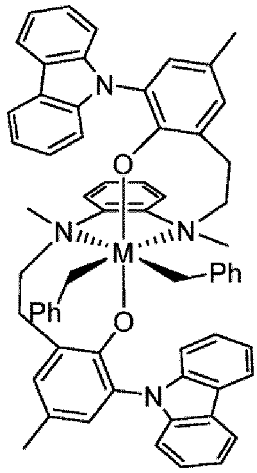 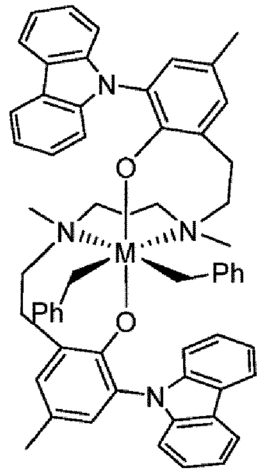 ,

dans lequel M est Hf ou Zr.

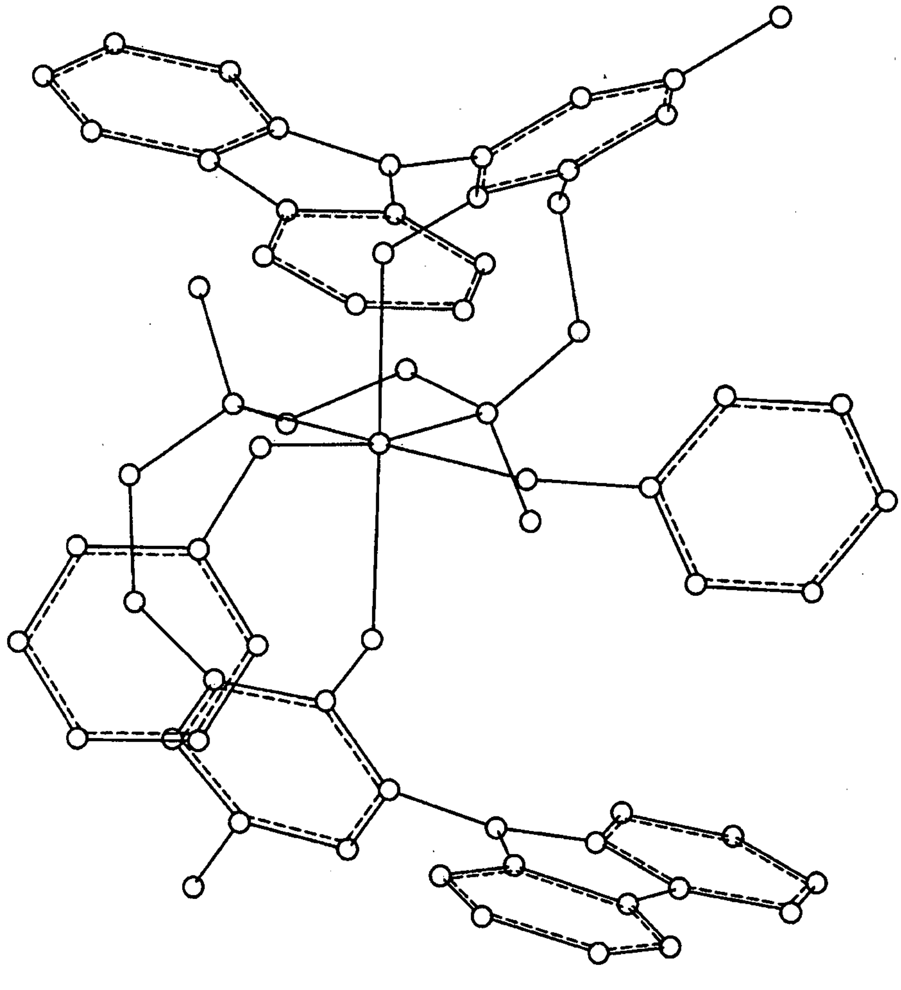

FIG. 1

Ball and stick model of 1-Zr

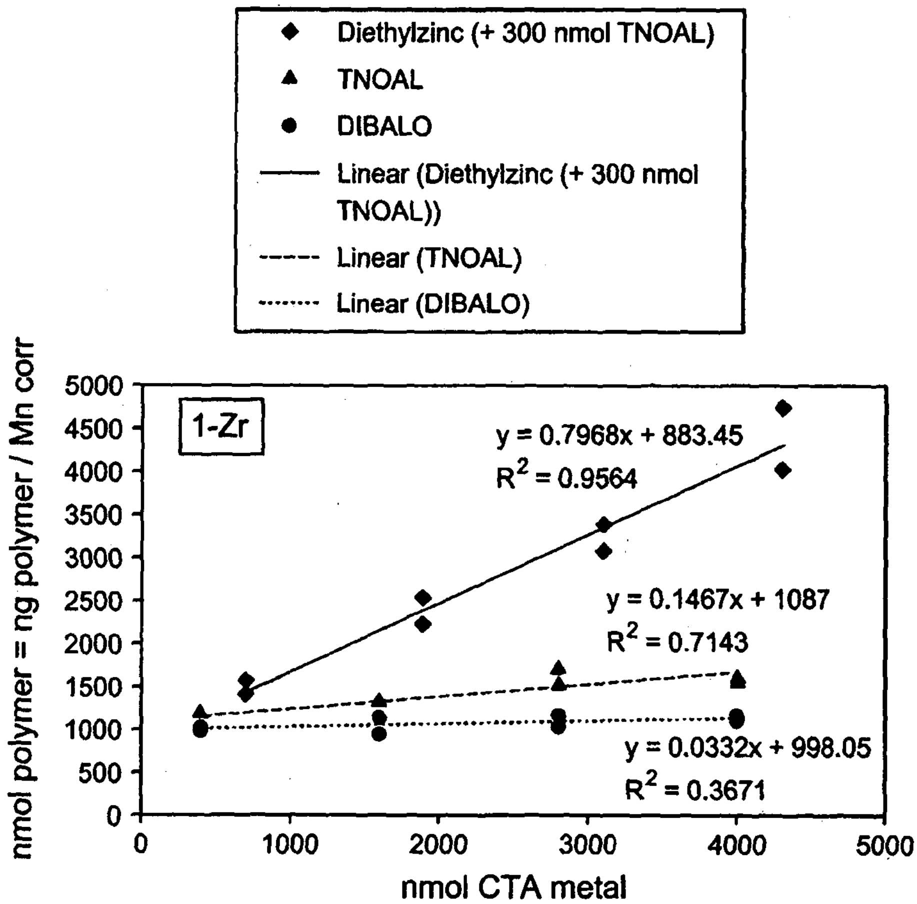
Diethylzinc (+ 300 nmol TNOAL)
TNOAL
DIBALO
Linear (Diethylzinc (+ 300 nmol TNOAL))
Linear (TNOAL)
Linear (DIBALO)
1-Zr
y = 0.7968x + 883.45
R2 = 0.9564
y = 0.1467x + 1087
R2 = 0.7143
y = 0.0332x + 998.05
R2 = 0.3671
nmol polymer = ng polymer / Mn corr
nmol CTA metal
0
500
1000
1500
2000
2500
3000
3500
4000
4500
5000
0
1000
2000
3000
4000
5000

FIG. 2

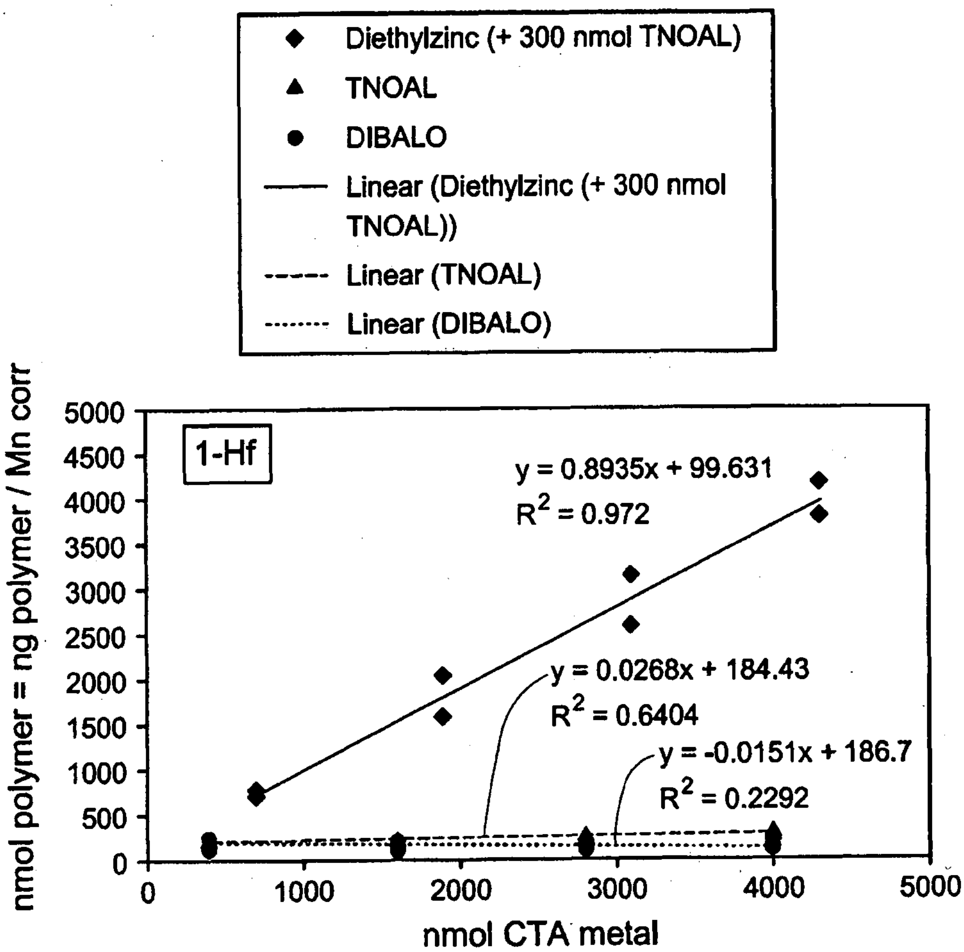
Diethylzinc (+ 300 nmol TNOAL)
TNOAL
DIBALO
Linear (Diethylzinc (+ 300 nmol TNOAL))
Linear (TNOAL)
Linear (DIBALO)
1-Hf
y = 0.8935x + 99.631
R2 = 0.972
y = 0.0268x + 184.43
R2 = 0.6404
y = -0.0151x + 186.7
R2 = 0.2292
nmol polymer = ng polymer / Mn corr
0
500
1000
1500
2000
2500
3000
3500
4000
4500
5000
0
1000
2000
3000
4000
5000
nmol CTA metal

FIG. 3

Compound A-6 in CD2Cl2; 1H NMR spectrum

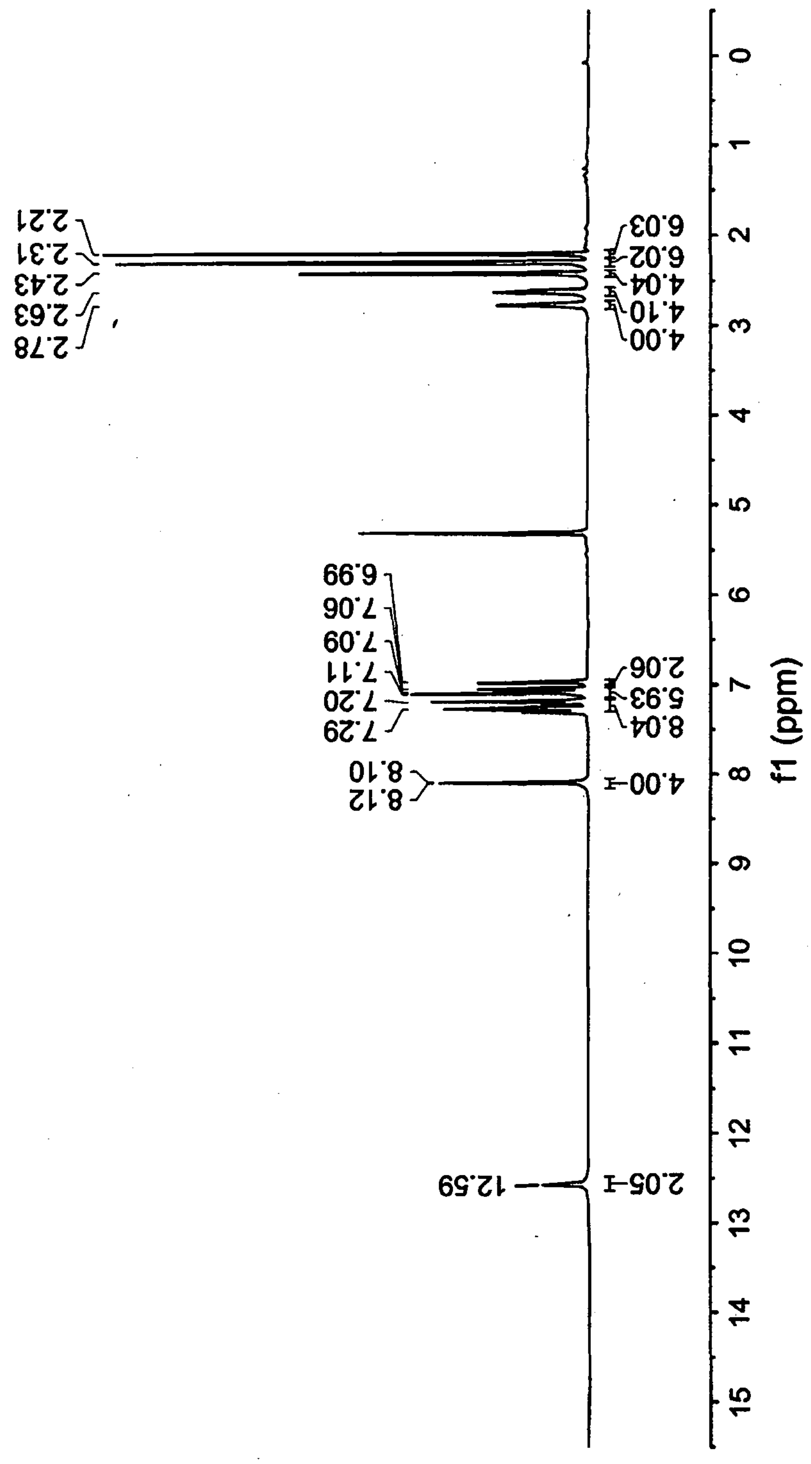


FIG. 4

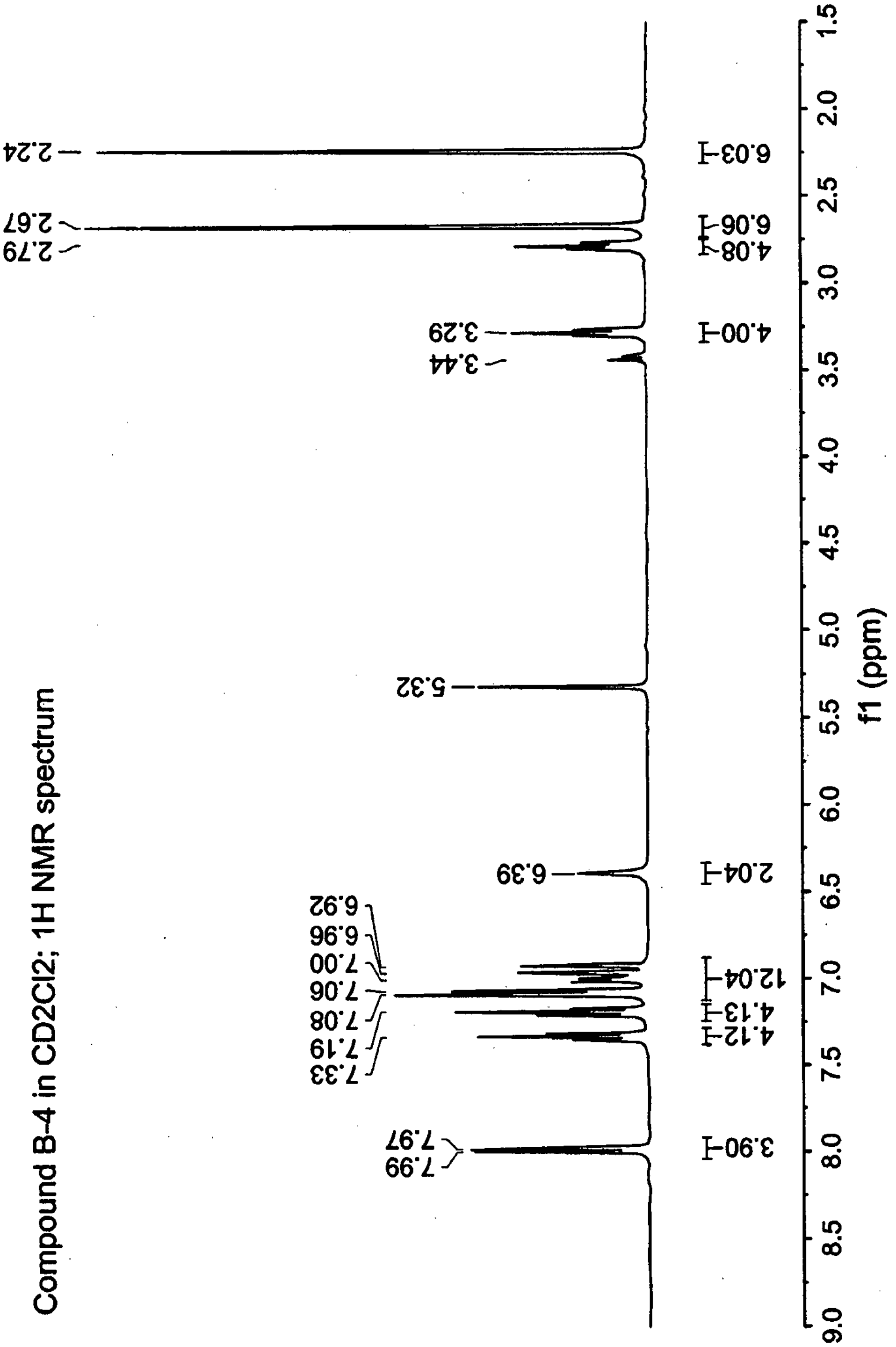
Compound B-4 in CD2Cl2; 1H NMR spectrum
2.24
2.67
2.79
3.29
3.44
5.32
6.39
6.92
6.96
7.00
7.06
7.08
7.19
7.33
7.97
7.99
6.03
6.06
4.08
4.00
2.04
12.04
4.13
4.12
3.90
9.0
8.5
8.0
7.5
7.0
6.5
6.0
5.5
5.0
4.5
4.0
3.5
3.0
2.5
2.0
1.5
f1 (ppm)

FIG. 5

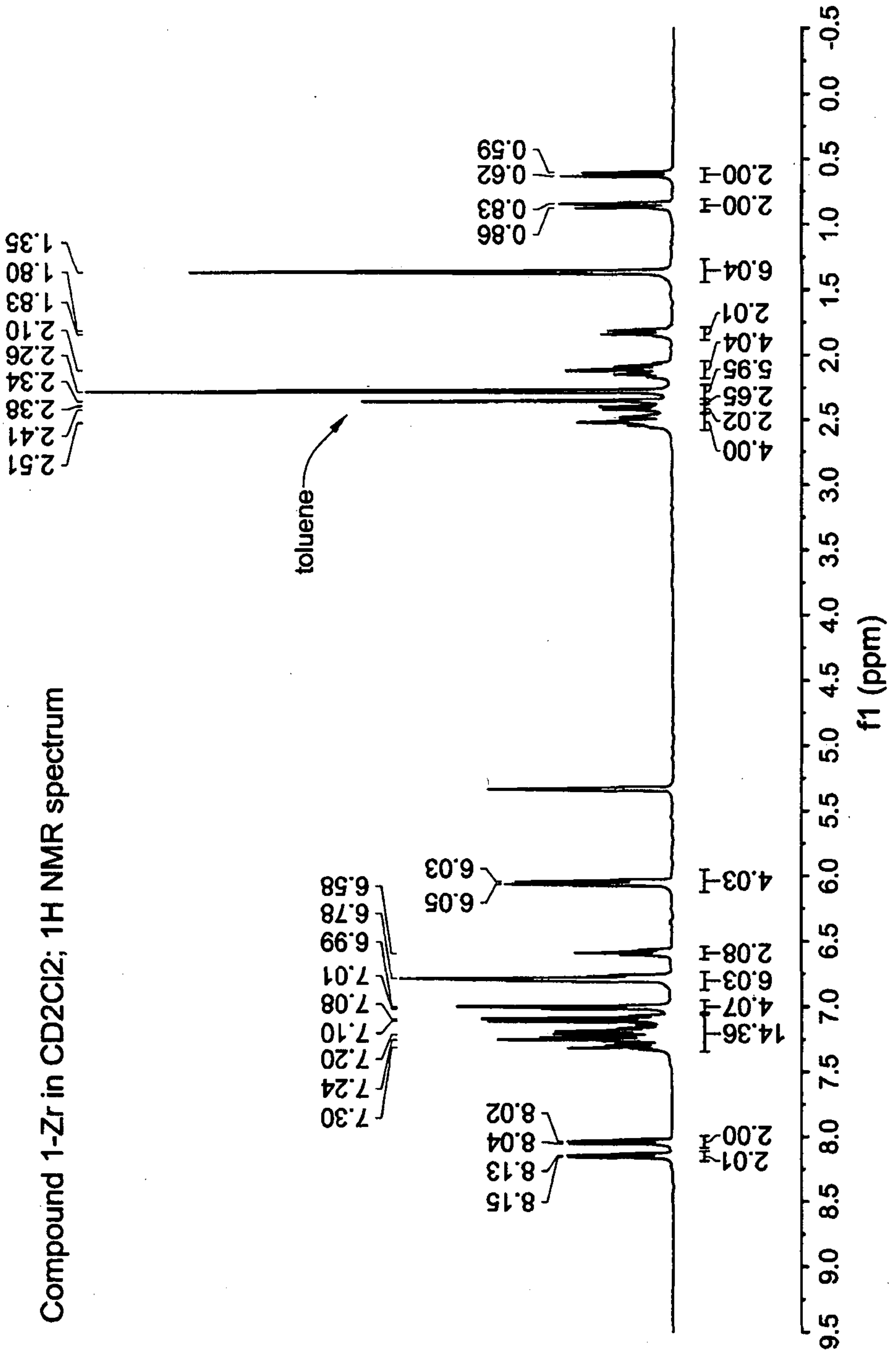
Compound 1-Zr in CD2Cl2; 1H NMR spectrum
toluene
8.15
8.13
8.04
8.02
7.30
7.24
7.20
7.10
7.08
7.01
6.99
6.78
6.58
6.05
6.03
2.51
2.41
2.38
2.34
2.26
2.10
1.83
1.80
1.35
0.86
0.83
0.62
0.59
2.01
2.00
14.36
4.07
6.03
2.08
4.03
4.00
2.02
2.65
5.95
4.04
2.01
6.04
2.00
2.00
9.5
9.0
8.5
8.0
7.5
7.0
6.5
6.0
5.5
5.0
4.5
4.0
3.5
3.0
2.5
2.0
1.5
1.0
0.5
0.0
-0.5
f1 (ppm)

FIG. 6

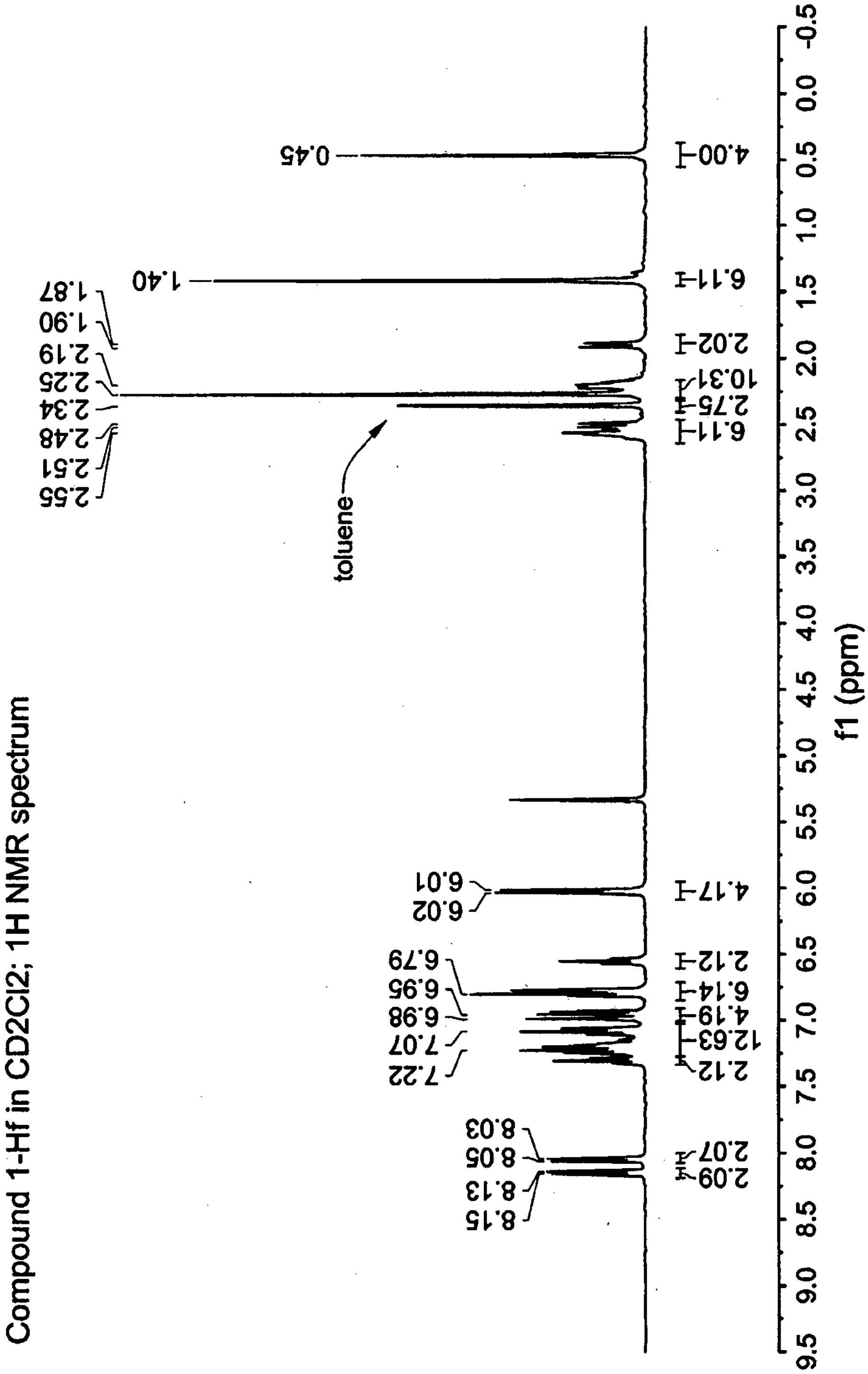
Compound 1-Hf in CD2Cl2; 1H NMR spectrum
toluene
0.45
1.40
1.87
1.90
2.19
2.25
2.34
2.48
2.51
2.55
6.01
6.02
6.79
6.95
6.98
7.07
7.22
8.03
8.05
8.13
8.15
4.00
6.11
2.02
10.31
2.75
6.11
4.17
2.12
6.14
4.19
12.63
2.12
2.07
2.09
f1 (ppm)

FIG. 7

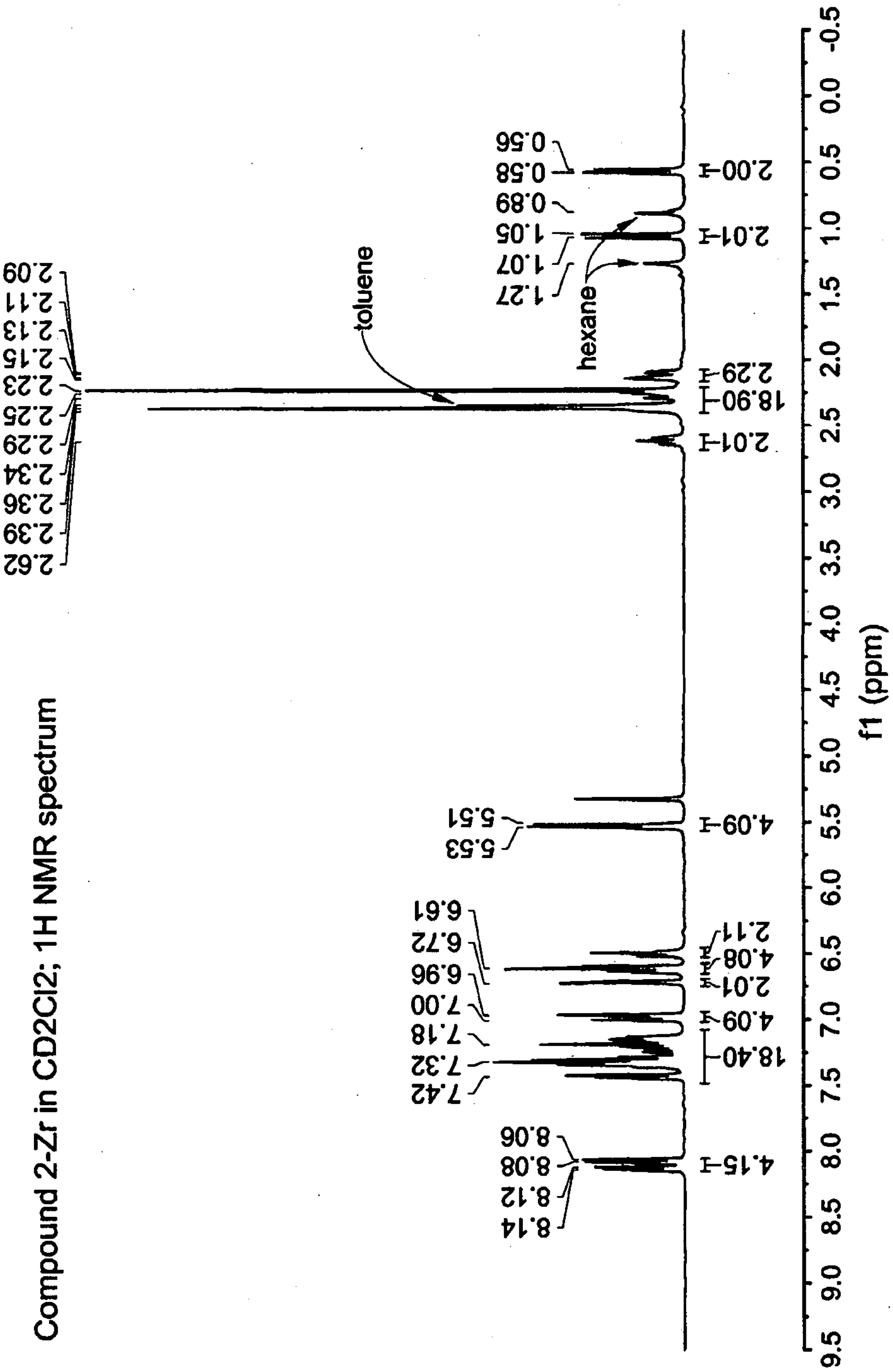
Compound 2-Zr in CD2Cl2; 1H NMR spectrum
toluene
hexane
f1 (ppm)

FIG. 8

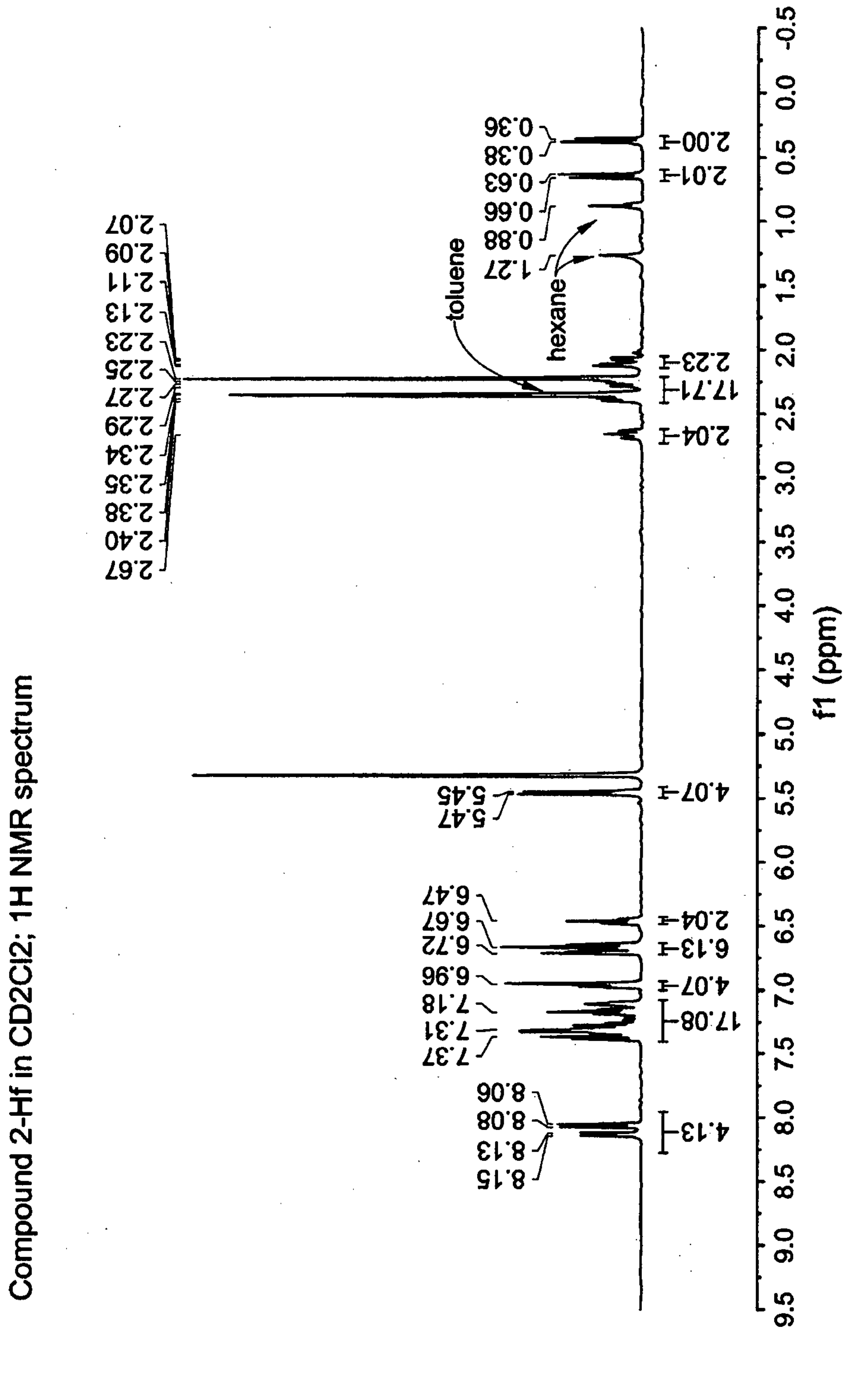
Compound 2-Hf in CD2Cl2; 1H NMR spectrum
2.07
2.09
2.11
2.13
2.23
2.25
2.27
2.29
2.34
2.35
2.38
2.40
2.67
toluene
hexane
0.36
0.38
0.63
0.66
0.88
1.27
5.45
5.47
6.47
6.67
6.72
6.96
7.18
7.31
7.37
8.06
8.08
8.13
8.15
2.00
2.01
2.23
17.71
2.04
4.07
2.04
6.13
4.07
17.08
4.13
9.5 9.0 8.5 8.0 7.5 7.0 6.5 6.0 5.5 5.0 4.5 4.0 3.5 3.0 2.5 2.0 1.5 1.0 0.5 0.0 -0.5
f1 (ppm)

FIG. 9

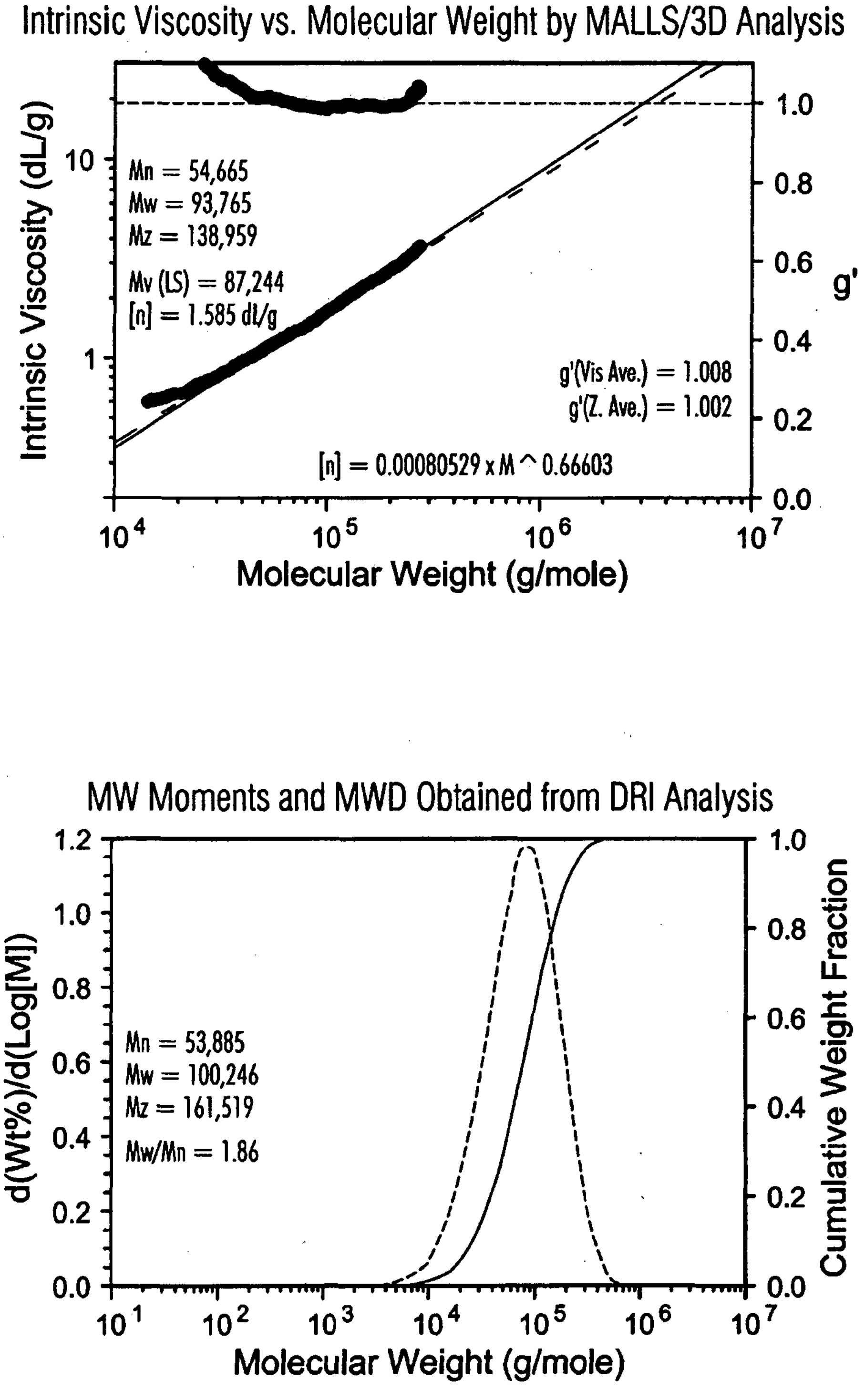
Intrinsic Viscosity vs. Molecular Weight by MALLS/3D Analysis
Mn = 54,665
Mw = 93,765
Mz = 138,959
Mv (LS) = 87,244
[n] = 1.585 dL/g
g'(Vis Ave.) = 1.008
g'(Z. Ave.) = 1.002
[n] = 0.00080529 x M ^ 0.66603
Intrinsic Viscosity (dL/g)
g'
Molecular Weight (g/mole)
MW Moments and MWD Obtained from DRI Analysis
Mn = 53,885
Mw = 100,246
Mz = 161,519
Mw/Mn = 1.86
d(Wt%)/d(Log[M])
Cumulative Weight Fraction
Molecular Weight (g/mole)

FIG. 10A

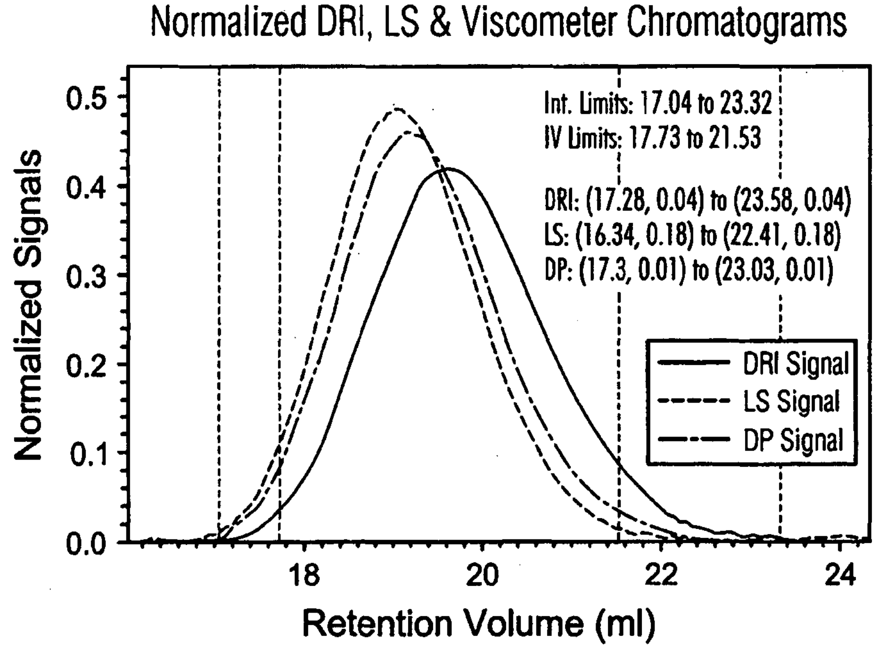


## Run Conditions and Instrument & Polymer Parameters

Analyzed as Polyethylene

Inject Mass (mg) = 0.088
Calc. Mass (mg) = 0.079 (90.1%)
Adjusted Flow Rate (ml/m) = 0.55
Column Cal. C0 = 11.535
Column Cal. C1 = -0.26583
Column Cal. C2 = -0.0023874
Column Cal. C3 = 0
Inject Mark (ml) = 32.404
Vistalon B1 = 1.021

Linear Zimm Analysis
A2 (Input Value) = 0.0015
(dn/dc) = 0.1048
LS to DRI (ml) = 0.26
LS to Vis. (ml) = 0.485
K (sample) = 0.000579
alpha (sample) = 0.695
LS Calib. Const. = 4.6796e-05
DRI Const. = 0.0001239
DP Const. = 0.3299
IP Gain = 27.45 mV/KPa

FIG. 10B

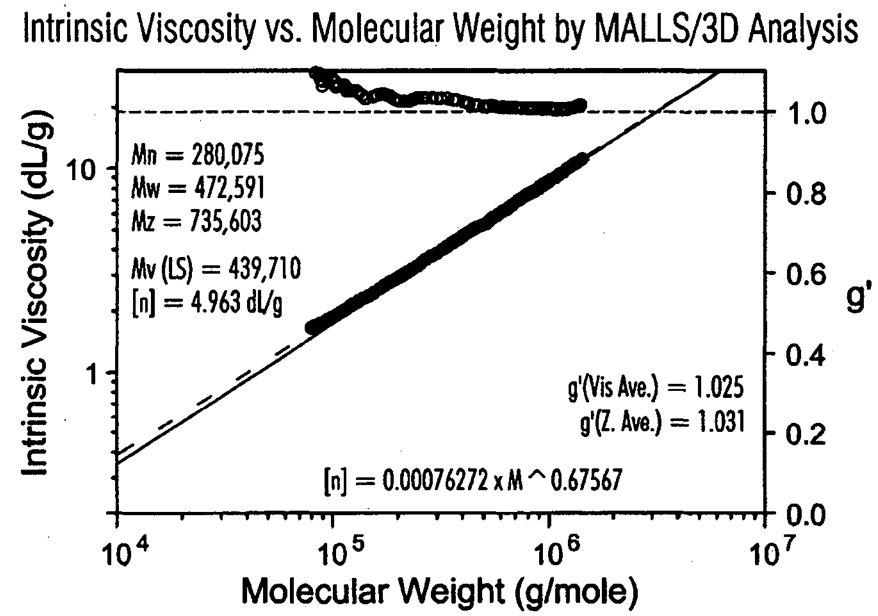
Intrinsic Viscosity vs. Molecular Weight by MALLS/3D Analysis
Intrinsic Viscosity (dL/g)
g'
Mn = 280,075
Mw = 472,591
Mz = 735,603
Mv (LS) = 439,710
[n] = 4.963 dL/g
g'(Vis Ave.) = 1.025
g'(Z. Ave.) = 1.031
[n] = 0.00076272 x M ^ 0.67567
Molecular Weight (g/mole)

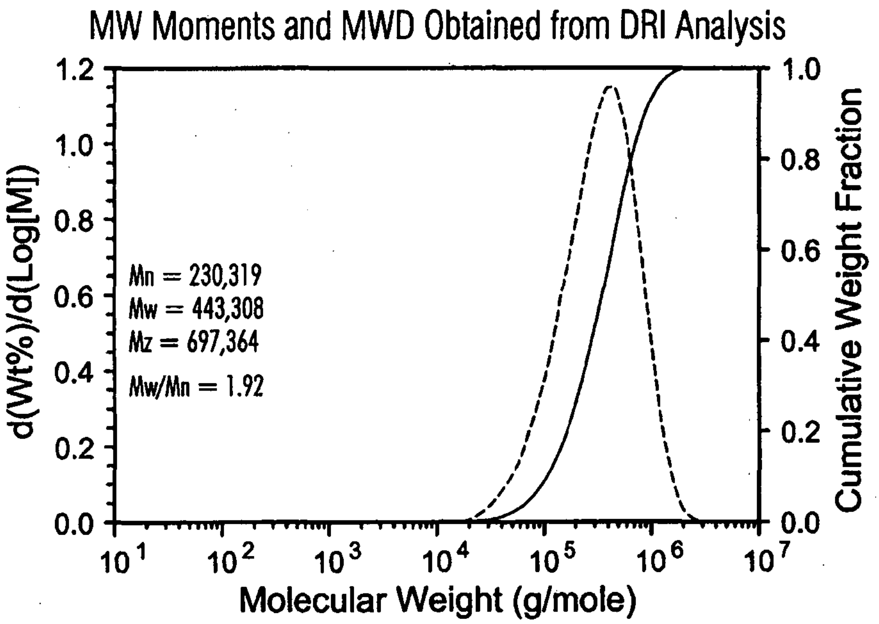
MW Moments and MWD Obtained from DRI Analysis
d(Wt%)/d(Log[M])
Cumulative Weight Fraction
Mn = 230,319
Mw = 443,308
Mz = 697,364
Mw/Mn = 1.92
Molecular Weight (g/mole)

FIG. 11A

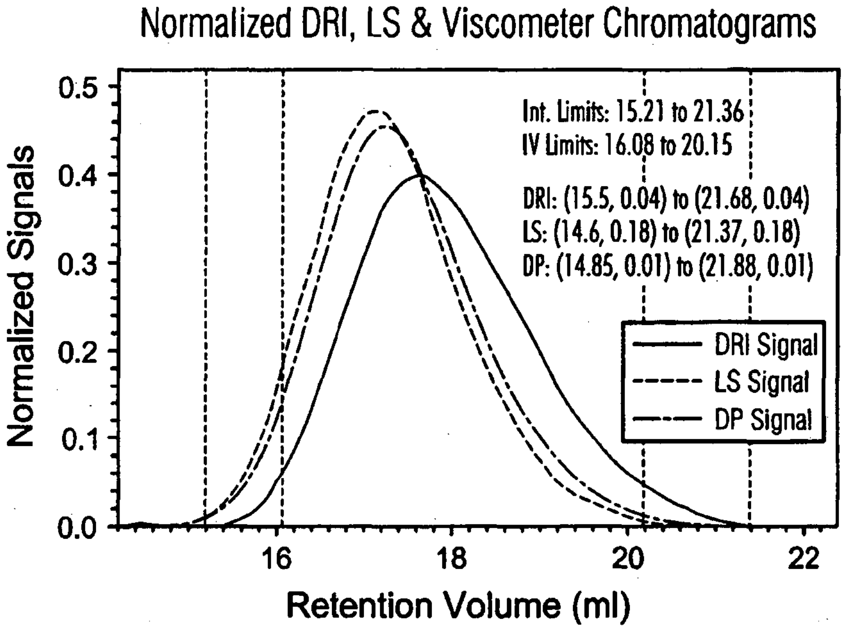


## Run Conditions and Instrument & Polymer Parameters

Analyzed as Polyethylene

| | |
|---|---|
| Inject Mass (mg) = 0.089 | Linear Zimm Analysis |
| Calc. Mass (mg) = 0.085 (95.4%) | A2 (Input Value) = 0.0015 |
| Adjusted Flow Rate (ml/m) = 0.55 | (dn/dc) = 0.1048 |
| Column Cal. C0 = 11.535 | LS to DRI (ml) = 0.26 |
| Column Cal. C1 = -0.26583 | LS to Vis. (ml) = 0.485 |
| Column Cal. C2 = -0.0023874 | K (sample) = 0.000579 |
| Column Cal. C3 = 0 | alpha (sample) = 0.695 |
| Inject Mark (ml) = 32.404 | LS Calib. Const. = 4.6796e-05 |
| Vistalon B1 = 1.047 | DRI Const. = 0.0001239 |
| | DP Const. = 0.3299 |
| | IP Gain = 27.45 mV/KPa |

FIG. 11B

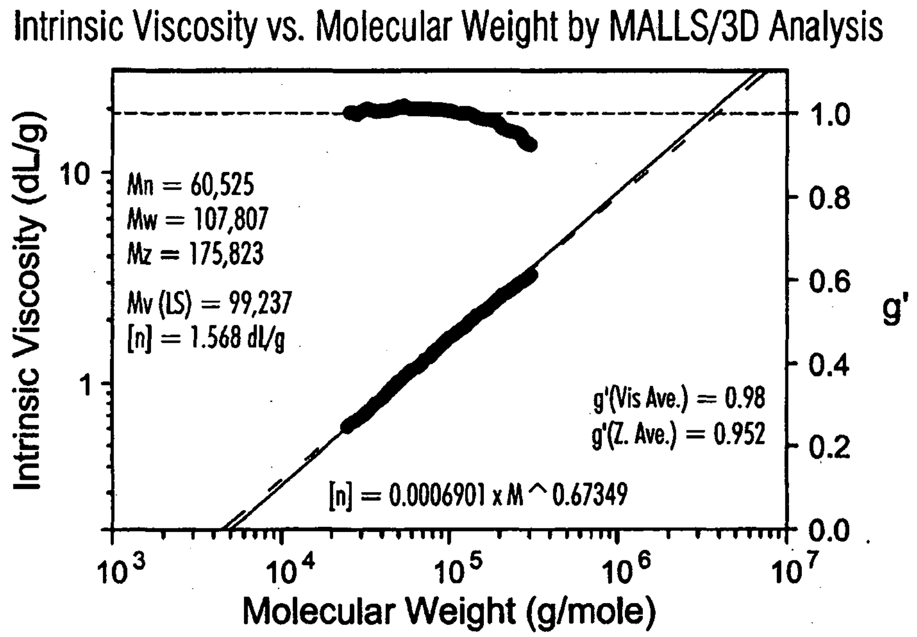
Intrinsic Viscosity vs. Molecular Weight by MALLS/3D Analysis
Intrinsic Viscosity (dL/g)
10
1
Mn = 60,525
Mw = 107,807
Mz = 175,823
Mv (LS) = 99,237
[n] = 1.568 dL/g
g'(Vis Ave.) = 0.98
g'(Z. Ave.) = 0.952
[n] = 0.0006901 x M ^ 0.67349
1.0
0.8
0.6
0.4
0.2
0.0
g'
10^3
10^4
10^5
10^6
10^7
Molecular Weight (g/mole)

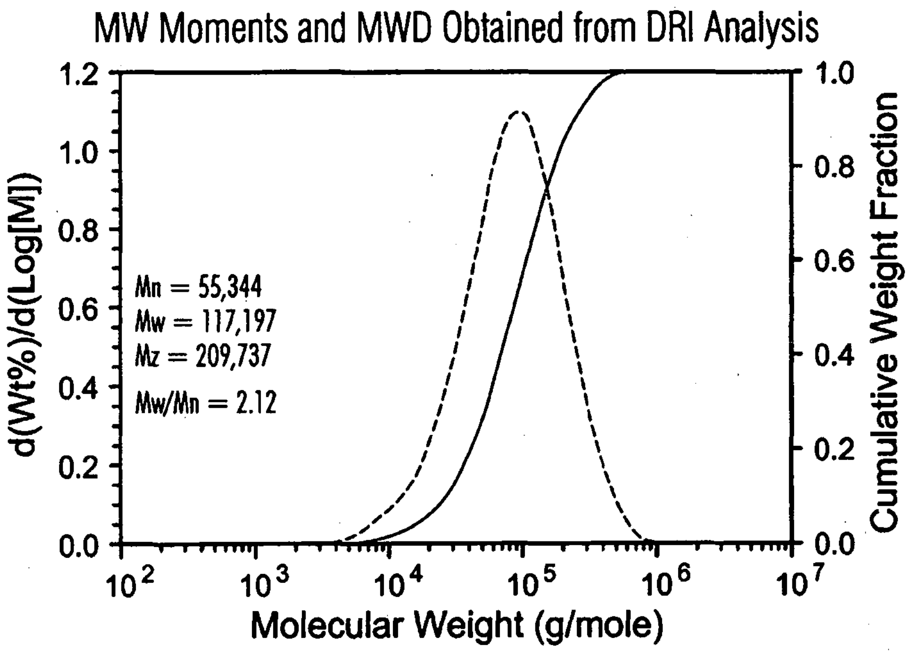
MW Moments and MWD Obtained from DRI Analysis
d(Wt%)/d(Log[M])
1.2
1.0
0.8
0.6
0.4
0.2
0.0
Mn = 55,344
Mw = 117,197
Mz = 209,737
Mw/Mn = 2.12
Cumulative Weight Fraction
1.0
0.8
0.6
0.4
0.2
0.0
10^2
10^3
10^4
10^5
10^6
10^7
Molecular Weight (g/mole)

FIG. 12A

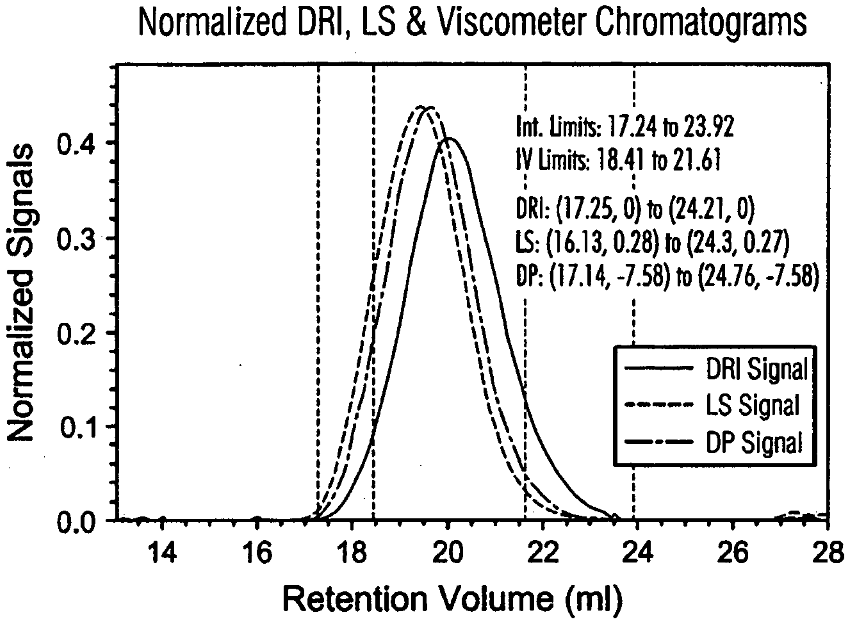


## Run Conditions and Instrument & Polymer Parameters

**Analyzed as ethylene-propylene copolymer with 14 weight percent comonomer**

Inject Mass (mg) = 0.167
Calc. Mass (mg) = 0.159 (95.2%)
Adjusted Flow Rate (ml/m) = 0.559
Column Cal. C0 = 12.284
Column Cal. C1 = -0.31407
Column Cal. C2 = -0.0014314
Column Cal. C3 = 0
Inject Mark (ml) = 33.001
Vistalon B1 = 0.988

Linear Zimm Analysis
A2 (Input Value) = 0.00136
(dn/dc) = 0.1048
LS to DRI (ml) = 0.199
LS to Vis. (ml) = 0.407
K (sample) = 0.00053228
alpha (sample) = 0.696
LS Calib. Const. = 5.0609e-05
DRI Const. = 0.0002665
DP Const. = 0.4178
IP Gain = 23.83 mV/KPa

FIG. 12B

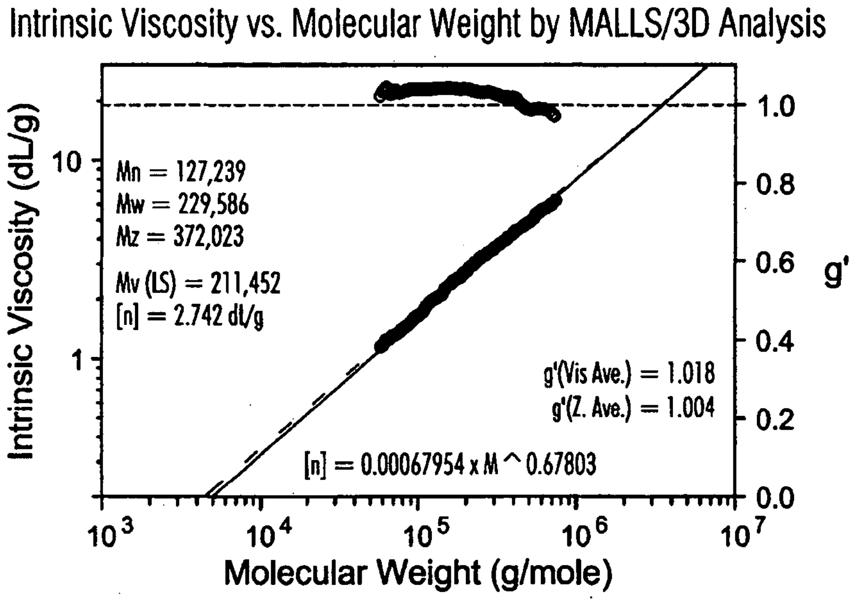
Intrinsic Viscosity vs. Molecular Weight by MALLS/3D Analysis
Intrinsic Viscosity (dL/g)
10
1
Mn = 127,239
Mw = 229,586
Mz = 372,023
Mv (LS) = 211,452
[n] = 2.742 dl/g
g'(Vis Ave.) = 1.018
g'(Z. Ave.) = 1.004
[n] = 0.00067954 x M ^ 0.67803
1.0
0.8
0.6
0.4
0.2
0.0
g'
10^3
10^4
10^5
10^6
10^7
Molecular Weight (g/mole)

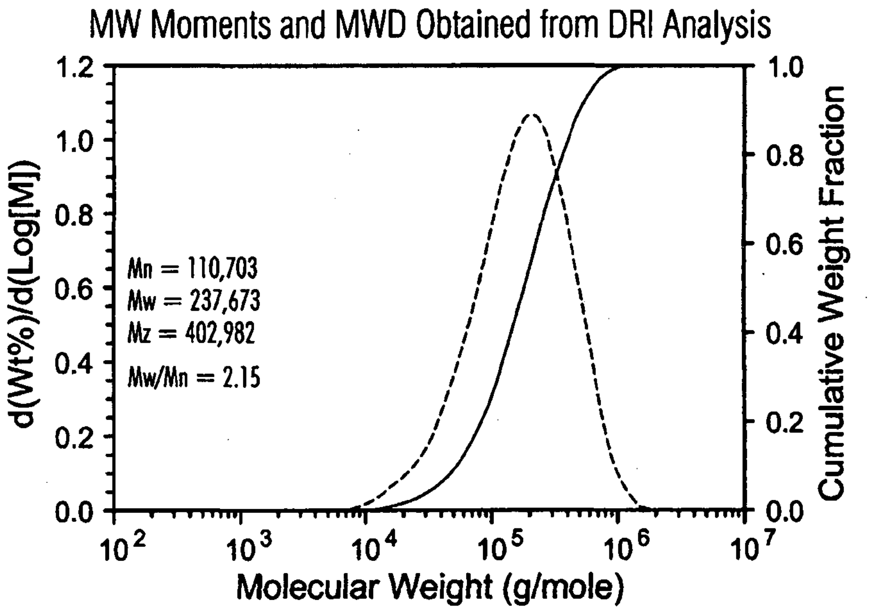
MW Moments and MWD Obtained from DRI Analysis
d(Wt%)/d(Log[M])
1.2
1.0
0.8
0.6
0.4
0.2
0.0
Mn = 110,703
Mw = 237,673
Mz = 402,982
Mw/Mn = 2.15
Cumulative Weight Fraction
1.0
0.8
0.6
0.4
0.2
0.0
10^2
10^3
10^4
10^5
10^6
10^7
Molecular Weight (g/mole)

FIG. 13A

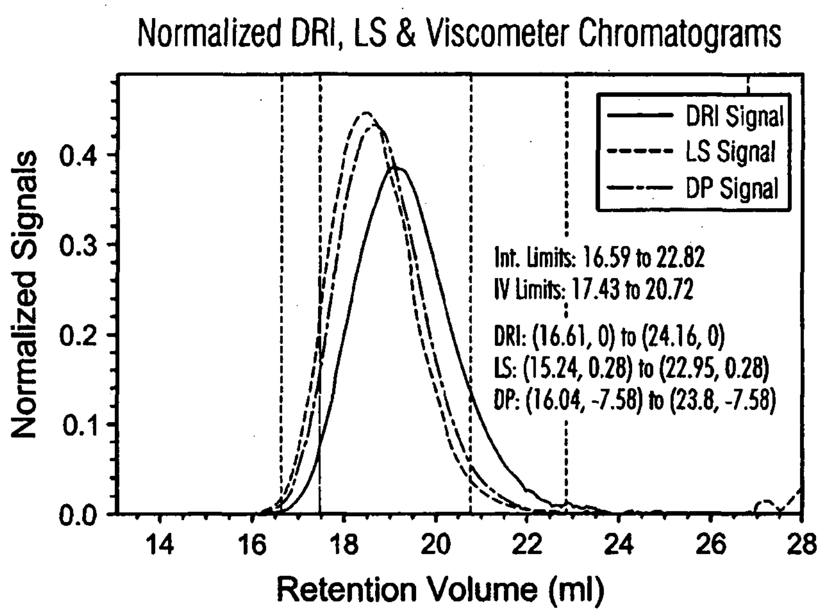


## Run Conditions and Instrument & Polymer Parameters

Analyzed as ethylene-propylene copolymer with 15 weight percent comonomer

Inject Mass (mg) = 0.129
Calc. Mass (mg) = 0.11 (85.1%)
Adjusted Flow Rate (ml/m) = 0.559
Column Cal. C0 = 12.284
Column Cal. C1 = -0.31407
Column Cal. C2 = -0.0014314
Column Cal. C3 = 0
Inject Mark (ml) = 33.001
Vistalon B1 = 1.039

Linear Zimm Analysis
A2 (Input Value) = 0.00135
(dn/dc) = 0.1048
LS to DRI (ml) = 0.199
LS to Vis. (ml) = 0.407
K (sample) = 0.00052939
alpha (sample) = 0.696
LS Calib. Const. = 5.0609e-05
DRI Const. = 0.0002665
DP Const. = 0.4178
IP Gain = 23.83 mV/KPa

FIG. 13B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2002036638 A
- WO 2012098521 A
- WO 2012027448 A
- WO 2003091262 A
- US 8071701 B
- WO 2016089935 A1
- WO 2016003878 A
- WO 2016003879 A
- US 6841502 B
- US 6596827 B
- WO 2014143202 A
- WO 2014022746 A
- WO 2014022010 A
- WO 2014022012 A
- WO 201422008 A
- WO 2014022011 A
- WO 2015088819 A
- US 8791217 B
- US 7812104 B
- US 6232421 B
- US 6333389 B
- US 6333423 B
- US 8907032 B
- WO 2007130306 A
- WO 2014070502 A
- US 20160280722 A
- US 20170096509 A
- WO 2013096573 A
- WO 2016094861 A
- WO 2016089935 A
- WO 2006020624 A
- US 20060025548 A
- US 20060052554 A
- WO 2005108406 A
- US 20060024517 A
- US 8957171 B
- US 8957172 B
- US 8952114 B
- US 9045568 B
- US 9365661 B
- US 9382349 B
- US 8937137 B
- US 9150676 B
- US 9200100 B
- US 9200099 B
- US 9290589 B
- US 9534070 B
- US 20150291713 A
- US 20150337062 A
- US 20150344601 A
- US 9000108 B
- US 9422383 B
- US 8609794 B
- US 9193813 B
- US 5041584 A
- WO 9843983 A
- US 5942459 A
- US 5447895 A
- US 8658556 B
- US 6211105 B
- US 5153157 A
- US 5453410 A
- EP 0573120 A
- WO 9407928 A
- WO 9514044 A
- US 5241025 A
- WO 9109882 A
- WO 9403506 A
- WO 9314132 A
- WO 9507941 A
- WO 9515815 A
- US 5427991 A
- US 6306658 B
- US 6455316 B
- US 6489168 B
- WO 0009255 A
- US 6491816 B
- US 6491823 B
- US 6475391 B
- US 6461515 B
- US 6436292 B
- US 6406632 B
- US 6175409 B
- US 6454947 B
- US 6260407 B
- US 6294388 B

### Non-patent literature cited in the description

- *Macromolecules,* 2005, vol. 38, 2552-2558
- *J. Am. Chem. Soc.,* 2000, vol. 122, 10706
- *Macromolecules,* 2010, vol. 43, 1689

- *Israel Journal of Chemistry,* 2002, vol. 42, 373-381
- *Macromolecules,* 2007, vol. 40, 7061-7064
- *Chem. Comm.,* 2000, 379-380
- *Organometallics,* 2001, vol. 20, 3017-3028
- *Organometallics,* 2005, vol. 24, 200-202
- *Organometallics,* 2009, vol. 28, 1391-1405
- Part A: Polymer Chemistry. *Journal of Polymer Science,* 2013, vol. 51, 593-600
- *Organometallics,* 2002, 662-670
- *Macromolecules,* 2013, vol. 46, 2569-2575
- *J. Am. Chem. Soc.,* 2010, vol. 132, 5566-5567
- *Macromolecules,* 2013, vol. 46, 6758-6764
- *Chemical and Engineering News,* 1985, vol. 63 (5), 27
- **J. VLADIMIR OLIVEIRA ; C. DARIVA ; J. C. PINTO.** *Ind. Eng, Chem. Res.,* 2000, vol. 29, 4627
- A Simple 'Back of the Envelope' Method for Estimating the Densities and Molecular Volumes of Liquids and Solids. *Journal of Chemical Education,* November 1994, vol. 71 (11), 962-964
- **MURPHY et al.** *J. Am. Chem. Soc.,* 2003, vol. 125, 4306-4317
- **T. SUN ; P. BRANT ; R. R. CHANCE ; W. W. GRAESSLEY.** *Macromolecules,* 2001, vol. 34 (19), 6812-6820
- **M.B. HUGLIN.** LIGHT SCATTERING FROM POLYMER SOLUTIONS. Academic Press, 1971
- *Macromolecules,* 2001, vol. 34, 6812-6820
- *Macromolecules,* 2005, vol. 38, 7181-7183